# EUROPEAN PATENT APPLICATION

(11) **EP 3 892 283 A1**
(43) Date of publication of application: **13.10.2021**
(21) Application number: 20315141.0
(22) Date of filing: 09.04.2020
(51) Int. Cl.: A61K 31/7088, A61K 48/00, C12N 15/86, C12N 15/861, A61P 25/28

(54) **NUCLEIC ACIDS ENCODING HUMAN FUS PROTEIN AND USE IN THE TREATMENT OF AMYOTROPHIC LATERAL SCLEROSIS (ALS)**

(71) Applicant: INSERM (Institut National de la Santé et de la Recherche Médicale), 75013 Paris (FR); Université de Strasbourg, 67000 Strasbourg (FR)
(72) Inventor: DUPUIS, Luc, 67000 Strasbourg (FR); Sanjuan Ruiz, Immaculada, 67000 Strasbourg (FR); PICCHIARELLI, Gina, 67100 Strasbourg (FR)
(74) Representative: Novagraaf Technologies

(57) **Abstract**

The invention relates to a nucleic acid encoding human FUS protein comprising a sequence having at least 69% sequence identity with the sequence SEQ ID NO 1 of intron 6 and/or a sequence having at least 60% sequence identity with the sequence SEQ ID NO 2 of intron 7, said sequences being located on either side of exon 7, to a recombinant vector comprising said nucleic acid encoding human FUS protein, and their use as medicament in the treatment and/or prevention of amyotrophic lateral sclerosis (ALS). The invention further relates to a pharmaceutical composition comprising said nucleic acid and/or vector for the prevention and/or treatment of amyotrophic lateral sclerosis (ALS).

The present invention finds application in the therapeutic, veterinary and diagnostic medical technical fields.

SEQUENCE LISTING

<110>  UNIVERSITE DE STRASBOURG
       INSERM - INSTITUT NATIONAL DE LA SANTÉ ET DE LA RECHERCHE
       MÉDICALE

<120>  NUCLEIC ACIDS ENCODING HUMAN FUS PROTEIN AND USE IN THE TREATMENT
        OF AMYOTROPHIC LATERAL SCLEROSIS (ALS)

<130>  BNT226367EP00

<160>  41

<170>  PatentIn version 3.5

<210>  1
<211>  1622
<212>  DNA
<213>  Artificial Sequence

<220>
<223>  intron 7

<400>  1
gtaggtgtct catgagccag ggagtatctt tggtggggag tgtggaggat tgcatgaatc      60

tccctgaagc cagtccctag tgcatggttt agtattcttg ttgtctaggg atctgtgagg     120

gctttgattt gggggcagtg actttctttt tacatcccca ttttattttt gtgagaactt     180

gggagcctga actcccatcc ataccactga atagagattt tgagtaatga tacttgtttc     240

caaaaaaaaa gaaaccatac atagatacgt atggattgga gtcattaata tcctaggcaa     300

gaaacatgga agtgaagact tctttctctg caagggaaac cgatgatccc actcctggga     360

aatagtaggg aaacttggta tgtgtattcc catgtgtcct ctagggagtt ggtaatggtt     420

aacctgactt cagcttccag gaattggcta ctcttcccgt tttctatagt catttgaatc     480

cacgagcttg atttgcacta atttgaccga cattgatttt gtgtgtgact tggtttatgg     540

ggccagctga ctgaagtaag cagacctttt gggcaaaaat atgctttgac agtggtctcc     600

cacctatttg ttccactgtc tgccttcccc tggttactta aaattcatca gcttgtccaa     660

ctggaccttc tttccttcct gctgaagttg atttgaagta aaaccttaga tttgatgtta     720

aaacagttgt caaatctgtt ggtaaataag atttgaagga ccctactctg tctcccttga     780

aaaaggggag gaatgtcagt gttactgttt ttggaaaaag tagattttta aaccgagttt     840

ggaaatggta agtatgcaga ggtgggtggg ggcaatctca aaaacgtgca aaaatgagga     900

aaacaaaaat gaggaaatgt gtgcgtgtgt ttaatgcaaa actttaaaaa gaaaaacaac     960

tgttatgtga ctgttaactt gctctgcatt ttatgtgcca caggtatgaa aggtgacatt    1020

gcaaaatact ccgctcttct cgcagtgtag aaggggtgac cccgggggtt gggggagatc    1080

aaaaacagct cagtagttag gacagagctt agctaagttt gtcttgcttt aaggggaagt    1140

tgcctttggt tttgactttt tatggaatgg ggttgggtct gcttgctgct ttcaaagcaa    1200

aaaccacaaa aatgtgttca aggctacccc agcctggtgt gaaatgtctt ctgggtaaat 1260

tggggtaggg tttttaaacc aactacttgg ttgtcaacca cttgcgacaa gaggaaaaaa 1320

aaacatctgc tccatcggaa gaacgaccaa ggaaaatggg ttattttttt tccagaggaa 1380

atagataacg taacctttta aagcaaaatc tttataaact gtgtctgaga aattgcacac 1440

gtgtgtgtga catgctcaaa ggtcagacaa ggggtggtca ggaagggatg tattttagta 1500

gccacttgta tctttttcca aaaacaccta cccatgtttg gggaatgtta aacaaaatca 1560

aaaaacaacc ttttgtagcc gttggaagct tcatgtcctt tcttctaact tgtcttctcc 1620

ag 1622

<210> 2
<211> 1488
<212> DNA
<213> Artificial Sequence

<220>
<223> intron 8

<400> 2
gtaagtgaac agagtttcca aaattcccaa ctcccagcaa tgctttgtct gattgttcat 60

ttgcagatgt cttagcgtgt taatttaaat gtcaaaggtt ttgaggtgtc cagaaccacc 120

tccagaaagg ggtagggtag aatgccacct gttgcctggt gtgtgctaac ctggagcagg 180

taggggtaag actcaatagt catcttttac caaatgggtt tgccccaggt taataagagg 240

ggtctagtag gccttggact gggccgttgc cacacctggc acttagtgac catcatcatg 300

agaaactgga gagtgcgtgc tggaacacgt ggtgccatct tggctttagg atccttttga 360

tcgttgtgtc caaggcttgt gtgtgtgtga gtgtgtggga gacaactccg aatgtttaat 420

tctggaagag ggatgtaaca ttgccctgag gatggtgaag ttggtataca tttataaagt 480

acggaatggt gtcaatgaat gcaattctat gtatatggac ttaactgaga tgggcaaata 540

gaaactagct ctgggaagga acatgtgcac tacttcaaga aagattggaa gcatgtgtgg 600

ctcatgggaa ataaccaggt cttaaacagc acaaactgaa ttcgtggacc aggaaggtct 660

taaacagcac aaactgaatt catggaaaaa tgacaaattt gagaagtctc ccagtaagct 720

ggaacttttc tggtttggtt aacaaaaggt ttcttgattt gtttcaagat ttaaagccaa 780

aggtgtgggt tcatgactta ggtgtcattg cgtgtgggta caatatttat atatggcgaa 840

ttcagataaa cattggtcaa agatggtctc tggaaaaaca aaatagaggc tgcattacgg 900

aaataagatt tctggtctgt tccctgggac atgcttaaaa aatacaatag ctattatgta 960

tggtttttat tttcatgtgg tttcggggaa acaacacggt tttaaggatg gtttctaaag 1020

atgaaattaa aaattgttcc acaagggtta agtgtctggt ggtaaagttg ggagaaactg 1080

gatggatgca catcgcatgg ctggtggcga gcccatctct cttctctcgg gtgagagaac 1140

cgggccaagc tgagttggtt tgttcacttt aatgggtctc cgtttcccct gccacctgtg 1200

ctgaggacat ttcccagcct gagctggggg aggcagcatt tgctgaagtg tggagttgtc 1260

tctgtggaga ctcaagttac agatcttaag gggcctgcct agaattttct cctctgggca 1320

ggcgacccag gaaagggttt ggagtgaggc tgtgagcact tacttgatat tttacaagtt 1380

tggatttggt gttaattttt ttccttgtcc gttttttcct gttgactaac ggctcatctt 1440

ttccttgttt ttgttttttt tttgttcttt ttttccatgt cactaaag 1488

<210> 3
<211> 1325
<212> DNA
<213> Artificial Sequence

<220>
<223> intron 7 delta 1

<400> 3
gtaggtgtct catgagccag ggagtatctt tggtggggag tgtggaggat tgcatgaatc 60

tccctgaagc cagtccctag tgcatggttt agtattcttg ttgtctaggg atctgtgagg 120

gctttgattt gggggcagtg actttctttt tacatcccca ttttattttt gtgagaactt 180

gggagcctga actcccatcc ataccactga atagagattt tgagtaatga tacttgtttc 240

caaaaaaaaa gaaaccatac atagatacgt atggattgga gtcattaata tcctaggcaa 300

gaaacatgga agtgaagact tctttctctg caagggaaac cgatgatccc actcctggga 360

aatagtaggg aaacttggta tgtgtattcc catgtgtgaa gtaaaacctt agatttgatg 420

ttaaaacagt tgtcaaatct gttggtaaat aagatttgaa ggaccctact ctgtctccct 480

tgaaaaaggg gaggaatgtc agtgttactg tttttggaaa aagtagattt ttaaaccgag 540

tttggaaatg gtaagtatgc agaggtgggt gggggcaatc tcaaaaacgt gcaaaaatga 600

ggaaaacaaa aatgaggaaa tgtgtgcgtg tgtttaatgc aaaactttaa aaagaaaaac 660

aactgttatg tgactgttaa cttgctctgc attttatgtg ccacaggtat gaaaggtgac 720

attgcaaaat actccgctct tctcgcagtg tagaaggggt gaccccgggg gttgggggag 780

atcaaaaaca gctcagtagt taggacagag cttagctaag tttgtcttgc tttaagggga 840

agttgccttt ggttttgact ttttatggaa tggggttggg tctgcttgct gctttcaaag 900

caaaaaccac aaaaatgtgt tcaaggctac cccagcctgg tgtgaaatgt cttctgggta 960

aattggggta gggtttttaa accaactact tggttgtcaa ccacttgcga caagaggaaa 1020

aaaaaacatc tgctccatcg gaagaacgac caaggaaaat gggttatttt ttttccagag 1080

gaaatagata acgtaacctt ttaaagcaaa atctttataa actgtgtctg agaaattgca 1140

cacgtgtgtg tgacatgctc aaaggtcaga caaggggtgg tcaggaaggg atgtatttta 1200

gtagccactt gtatcttttt ccaaaaacac ctacccatgt ttggggaatg ttaaacaaaa 1260

tcaaaaaaca accttttgta gccgttggaa gcttcatgtc ctttcttcta acttgtcttc 1320

tccag 1325

<210> 4
<211> 1424
<212> DNA
<213> Artificial Sequence

<220>
<223> intron 7 delta 2

<400> 4
gtaggtgtct catgagccag ggagtatctt tggtggggag tgtggaggat tgcatgaatc 60

tccctgaagc cagtccctag tgcatggttt agtattcttg ttgtctaggg atctgtgagg 120

gctttgattt gggggcagtg actttctttt tacatcccca ttttattttt gtgagaactt 180

gggagcctga actcccatcc ataccactga atagagattt tgagtaatga tacttgtttc 240

caaaaaaaaa gaaaccatac atagatacgt atggattgga gtcattaata tcctaggcaa 300

gaaacatgga agtgaagact tctttctctg caagggaaac cgatgatccc actcctggga 360

aatagtaggg aaacttggta tgtgtattcc catgtgtcct ctagggagtt ggtaatggtt 420

aacctgactt cagcttccag gaattggcta ctcttcccgt tttctatagt catttgaatc 480

cacgagcttg atttgcacta atttgaccga cattgatttt gtgtgtgact tggtttatgg 540

ggccagctga ctgaagtaag cagacctttt gggcaaaaat atgctttgac agtggtctcc 600

cacctatttg ttccactgtc tgccttcccc tggttactta aaattcatca gcttgtccaa 660

ctggaccttc tttccttcct gctgaagttg atttgaagta aaaccttaga tttgatgtta 720

aaacagttgt caaatctgtt ggtaaataag atttgaagga ccctactctg tctcccttga 780

aaaaggggag gaatgtcagt gttactgttt ttggaaaaag tagattttta aaccgagttt 840

ggaaatggta agtatgcaga ggtgggtggg ggcaatctca aaaacgtgca aaaatgagga 900

aaacaaaaat gaggaaatgt gtgcgtgtgt ttaatgcaaa actttaaaaa gaaaaacaac 960

tgttatgtga ctgttaactt gctctgcatt ctttcaaagc aaaaaccaca aaaatgtgtt 1020

caaggctacc ccagcctggt gtgaaatgtc ttctgggtaa attggggtag ggtttttaaa 1080

ccaactactt ggttgtcaac cacttgcgac aagaggaaaa aaaaacatct gctccatcgg 1140

aagaacgacc aaggaaaatg ggttattttt tttccagagg aaatagataa cgtaaccttt 1200

taaagcaaaa tctttataaa ctgtgtctga gaaattgcac acgtgtgtgt gacatgctca 1260

aaggtcagac aaggggtggt caggaaggga tgtattttag tagccacttg tatctttttc 1320

caaaaacacc tacccatgtt tggggaatgt taaacaaaat caaaaaacaa ccttttgtag 1380

ccgttggaag cttcatgtcc tttcttctaa cttgtcttct ccag 1424

<210> 5
<211> 1290
<212> DNA
<213> Artificial Sequence

<220>
<223> intron 8 delta 3

<400> 5
gtaagtgaac agagtttcca aaattcccaa ctcccagcaa tgctttgtct gattgttcat 60

ttgcagatgt cttagcgtgt taatttaaat gtcaaaggtt ttgaggtgtc cagaaccacc 120

tccagaaagg ggtagggtag aatgccacct gttgcctggt gtgtgctaac ctggagcagg 180

taggggtaag actcaatagt catcttttac caaatgggtt tgcctggaag agggatgtaa 240

cattgccctg aggatggtga agttggtata catttataaa gtacggaatg gtgtcaatga 300

atgcaattct atgtatatgg acttaactga gatgggcaaa tagaaactag ctctgggaag 360

gaacatgtgc actacttcaa gaaagattgg aagcatgtgt ggctcatggg aaataaccag 420

gtcttaaaca gcacaaactg aattcgtgga ccaggaaggt cttaaacagc acaaactgaa 480

ttcatggaaa aatgacaaat ttgagaagtc tcccagtaag ctggaacttt tctggtttgg 540

ttaacaaaag gtttcttgat ttgtttcaag atttaaagcc aaaggtgtgg gttcatgact 600

taggtgtcat tgcgtgtggg tacaatattt atatatggcg aattcagata aacattggtc 660

aaagatggtc tctggaaaaa caaaatagag gctgcattac ggaaataaga tttctggtct 720

gttccctggg acatgcttaa aaaatacaat agctattatg tatggttttt attttcatgt 780

ggtttcgggg aaacaacacg gttttaagga tggtttctaa agatgaaatt aaaaattgtt 840

ccacaagggt taagtgtctg gtggtaaagt tgggagaaac tggatggatg cacatcgcat 900

ggctggtggc gagcccatct ctcttctctc gggtgagaga accgggccaa gctgagttgg 960

tttgttcact ttaatgggtc tccgtttccc ctgccacctg tgctgaggac atttcccagc 1020

ctgagctggg ggaggcagca tttgctgaag tgtggagttg tctctgtgga gactcaagtt 1080

acagatctta aggggcctgc ctagaatttt ctcctctggg caggcgaccc aggaaagggt 1140

ttggagtgag gctgtgagca cttacttgat attttacaag tttggatttg gtgttaattt 1200

ttttccttgt ccgttttttc ctgttgacta acggctcatc ttttccttgt ttttgttttt 1260

tttttgttct ttttttccat gtcactaaag 1290

<210> 6
<211> 1290
<212> DNA
<213> Artificial Sequence

<220>

<223>  intron 8 delta 4

<400>  6
gtaagtgaac agagtttcca aaattcccaa ctcccagcaa tgctttgtct gattgttcat      60

ttgcagatgt cttagcgtgt taatttaaat gtcaaaggtt ttgaggtgtc cagaaccacc     120

tccagaaagg ggtagggtag aatgccacct gttgcctggt gtgtgctaac ctggagcagg     180

taggggtaag actcaatagt catcttttac caaatgggtt tgccccaggt taataagagg     240

ggtctagtag gccttggact gggccgttgc cacacctggc acttagtgac catcatcatg     300

agaaactgga gagtgcgtgc tggaacacgt ggtgccatct tggctttagg atccttttga      360

tcgttgtgtc caaggcttgt gtgtgtgtga gtgtgtggga gacaactccg aatgtttaat     420

tctggaagag ggatgtaaca ttgccctgag gatggtgaag ttggtataca tttataaagt     480

acggaatggt gtcaatgaat gcaattctat gtatatggac ttggaacttt tctggtttgg     540

ttaacaaaag gtttcttgat ttgtttcaag atttaaagcc aaaggtgtgg gttcatgact     600

taggtgtcat tgcgtgtggg tacaatattt atatatggcg aattcagata aacattggtc     660

aaagatggtc tctggaaaaa caaaatagag gctgcattac ggaaataaga tttctggtct     720

gttccctggg acatgcttaa aaaatacaat agctattatg tatggttttt attttcatgt     780

ggtttcgggg aaacaacacg gttttaagga tggtttctaa agatgaaatt aaaaattgtt     840

ccacaagggt taagtgtctg gtggtaaagt tgggagaaac tggatggatg cacatcgcat     900

ggctggtggc gagcccatct ctcttctctc gggtgagaga accgggccaa gctgagttgg     960

tttgttcact ttaatgggtc tccgtttccc ctgccacctg tgctgaggac atttcccagc    1020

ctgagctggg ggaggcagca tttgctgaag tgtggagttg tctctgtgga gactcaagtt    1080

acagatctta aggggcctgc ctagaatttt ctcctctggg caggcgaccc aggaaagggt    1140

ttggagtgag gctgtgagca cttacttgat attttacaag tttggatttg gtgttaattt    1200

ttttccttgt ccgttttttc ctgttgacta acggctcatc ttttccttgt ttttgttttt     1260

tttttgttct ttttttccat gtcactaaag                                     1290


<210>  7
<211>  1290
<212>  DNA
<213>  Artificial Sequence

<220>
<223>  intron 8 delta 5

<400>  7
gtaagtgaac agagtttcca aaattcccaa ctcccagcaa tgctttgtct gattgttcat      60

ttgcagatgt cttagcgtgt taatttaaat gtcaaaggtt ttgaggtgtc cagaaccacc     120

tccagaaagg ggtagggtag aatgccacct gttgcctggt gtgtgctaac ctggagcagg     180

taggggtaag actcaatagt catcttttac caaatgggtt tgccccaggt taataagagg 240

ggtctagtag gccttggact gggccgttgc cacacctggc acttagtgac catcatcatg 300

agaaactgga gagtgcgtgc tggaacacgt ggtgccatct tggctttagg atccttttga 360

tcgttgtgtc caaggcttgt gtgtgtgtga gtgtgtggga gacaactccg aatgtttaat 420

tctggaagag ggatgtaaca ttgccctgag gatggtgaag ttggtataca tttataaagt 480

acggaatggt gtcaatgaat gcaattctat gtatatggac ttaactgaga tgggcaaata 540

gaaactagct ctgggaagga acatgtgcac tacttcaaga aagattggaa gcatgtgtgg 600

ctcatgggaa ataaccaggt cttaaacagc acaaactgaa ttcgtggacc aggaaggtct 660

taaacagcac aaactgaatt catggaaaaa tgacaaattt gagaagtctc ccagtaagct 720

ggaacttttc tggtttggtt aacaaaaggt ttcttgattt gtttcaagat ttaaagccaa 780

aggtgtgggt tcatgactta ggtgtcattg cgtgtgggaa agatgaaatt aaaaattgtt 840

ccacaagggt taagtgtctg gtggtaaagt tgggagaaac tggatggatg cacatcgcat 900

ggctggtggc gagcccatct ctcttctctc gggtgagaga accgggccaa gctgagttgg 960

tttgttcact ttaatgggtc tccgtttccc ctgccacctg tgctgaggac atttcccagc 1020

ctgagctggg ggaggcagca tttgctgaag tgtggagttg tctctgtgga gactcaagtt 1080

acagatctta aggggcctgc ctagaatttt ctcctctggg caggcgaccc aggaaagggt 1140

ttggagtgag gctgtgagca cttacttgat attttacaag tttggatttg gtgttaattt 1200

ttttccttgt ccgttttttc ctgttgacta acggctcatc ttttccttgt ttttgttttt 1260

tttttgttct ttttttccat gtcactaaag 1290

<210> 8
<211> 1290
<212> DNA
<213> Artificial Sequence

<220>
<223> intron 8 delta 6

<400> 8
gtaagtgaac agagtttcca aaattcccaa ctcccagcaa tgctttgtct gattgttcat 60

ttgcagatgt cttagcgtgt taatttaaat gtcaaaggtt ttgaggtgtc cagaaccacc 120

tccagaaagg ggtagggtag aatgccacct gttgcctggt gtgtgctaac ctggagcagg 180

taggggtaag actcaatagt catcttttac caaatgggtt tgccccaggt taataagagg 240

ggtctagtag gccttggact gggccgttgc cacacctggc acttagtgac catcatcatg 300

agaaactgga gagtgcgtgc tggaacacgt ggtgccatct tggctttagg atccttttga 360

tcgttgtgtc caaggcttgt gtgtgtgtga gtgtgtggga gacaactccg aatgtttaat 420

tctggaagag ggatgtaaca ttgccctgag gatggtgaag ttggtataca tttataaagt 480

acggaatggt gtcaatgaat gcaattctat gtatatggac ttaactgaga tgggcaaata 540

gaaactagct ctgggaagga acatgtgcac tacttcaaga aagattggaa gcatgtgtgg 600

ctcatgggaa ataaccaggt cttaaacagc acaaactgaa ttcgtggacc aggaaggtct 660

taaacagcac aaactgaatt catggaaaaa tgacaaattt gagaagtctc ccagtaagct 720

ggaacttttc tggtttggtt aacaaaaggt ttcttgattt gtttcaagat ttaaagccaa 780

aggtgtgggt tcatgactta ggtgtcattg cgtgtgggta caatatttat atatggcgaa 840

ttcagataaa cattggtcaa agatggtctc tggaaaaaca aaatagaggc tgcattacgg 900

aaataagatt tctggtctgt tccctgggac atgcttaaaa aatacaatag ctattatgta 960

tggtttttat tttcatgtgg tttcggggaa acaacacggt tttaaggatg gtttctaaag 1020

atgaaattaa aaattgttcc acaaggttta agtgtctggt ggtaaagttg ggagaaactg 1080

gatggatgca catcgcatgg ctggtggcga gcccactggg caggcgaccc aggaaagggt 1140

ttggagtgag gctgtgagca cttacttgat attttacaag tttggatttg gtgttaattt 1200

ttttccttgt ccgttttttc ctgttgacta acggctcatc ttttccttgt ttttgttttt 1260

tttttgttct ttttttccat gtcactaaag 1290

<210> 9
<211> 4796
<212> DNA
<213> Artificial Sequence

<220>
<223> séquence complète sans 3'UTR

<400> 9
acttaagctt cgacgcagga ggcggggctg ctcagtcctc caggcgtcgg tactcagcgg 60

tgttggaact tcgttgcttg cttgcctgtg cgcgcgtgcg cggacatggc ctcaaacgat 120

tatacccaac aagcaaccca aagctatggg gcctacccca cccagcccgg gcagggctat 180

tcccagcaga gcagtcagcc ctacggacag cagagttaca gtggttatag ccagtccacg 240

gacacttcag gctatggcca gagcagctat tcttcttatg gccagagcca gaacacaggc 300

tatggaactc agtcaactcc ccagggatat ggctcgactg gcggctatgg cagtagccag 360

agctcccaat cgtcttacgg gcagcagtcc tcctaccctg gctatggcca gcagccagct 420

cccagcagca cctcgggaag ttacggtagc agttctcaga gcagcagcta tgggcagccc 480

cagagtggga gctacagcca gcagcctagc tatggtggac agcagcaaag ctatggacag 540

cagcaaagct ataatccccc tcagggctat ggacagcaga accagtacaa cagcagcagt 600

ggtggtggag gtggaggtgg aggtggaggt aactatggcc aagatcaatc ctccatgagt 660

agtggtggtg gcagtggtgg cggttatggc aatcaagacc agagtggtgg aggtggcagc 720

ggtggctatg gacagcagga ccgtggaggc cgcggcaggg gtggcagtgg tggcggcggc 780

ggcggcggcg gtggtggtta caaccgcagc agtggtggct atgaacccag aggtcgtgga 840

ggtggccgtg gaggcagagg tggcatgggg taggtgtctc atgagccagg gagtatcttt 900

ggtggggagt gtggaggatt gcatgaatct ccctgaagcc agtccctagt gcatggttta 960

gtattcttgt tgtctaggga tctgtgaggg ctttgatttg ggggcagtga ctttcttttt 1020

acatccccat tttatttttg tgagaacttg ggagcctgaa ctcccatcca taccactgaa 1080

tagagatttt gagtaatgat acttgtttcc aaaaaaaaag aaaccataca tagatacgta 1140

tggattggag tcattaatat cctaggcaag aaacatggaa gtgaagactt ctttctctgc 1200

aagggaaacc gatgatccca ctcctgggaa atagtaggga aacttggtat gtgtattccc 1260

atgtgtcctc tagggagttg gtaatggtta acctgacttc agcttccagg aattggctac 1320

tcttcccgtt ttctatagtc atttgaatcc acgagcttga tttgcactaa tttgaccgac 1380

attgattttg tgtgtgactt ggtttatggg gccagctgac tgaagtaagc agaccttttg 1440

ggcaaaaata tgctttgaca gtggtctccc acctatttgt tccactgtct gccttcccct 1500

ggttacttaa aattcatcag cttgtccaac tggaccttct ttccttcctg ctgaagttga 1560

tttgaagtaa aaccttagat ttgatgttaa aacagttgtc aaatctgttg gtaaataaga 1620

tttgaaggac cctactctgt ctcccttgaa aaaggggagg aatgtcagtg ttactgtttt 1680

tggaaaaagt agatttttaa accgagtttg gaaatggtaa gtatgcagag gtgggtgggg 1740

gcaatctcaa aaacgtgcaa aaatgaggaa aacaaaaatg aggaaatgtg tgcgtgtgtt 1800

taatgcaaaa ctttaaaaag aaaaacaact gttatgtgac tgttaacttg ctctgcattt 1860

tatgtgccac aggtatgaaa ggtgacattg caaaatactc cgctcttctc gcagtgtaga 1920

aggggtgacc ccgggggttg ggggagatca aaaacagctc agtagttagg acagagctta 1980

gctaagtttg tcttgcttta aggggaagtt gcctttggtt ttgacttttt atggaatggg 2040

gttgggtctg cttgctgctt tcaaagcaaa aaccacaaaa atgtgttcaa ggctacccca 2100

gcctggtgtg aaatgtcttc tgggtaaatt ggggtagggt ttttaaacca actacttggt 2160

tgtcaaccac ttgcgacaag aggaaaaaaa aacatctgct ccatcggaag aacgaccaag 2220

gaaaatgggt tatttttttt ccagaggaaa tagataacgt aaccttttaa agcaaaatct 2280

ttataaactg tgtctgagaa attgcacacg tgtgtgtgac atgctcaaag gtcagacaag 2340

gggtggtcag gaagggatgt attttagtag ccacttgtat ctttttccaa aaacacctac 2400

ccatgtttgg ggaatgttaa acaaaatcaa aaaacaacct tttgtagccg ttggaagctt 2460

catgtccttt cttctaactt gtcttctcca gcggaagtga ccgtggtggc ttcaataaat 2520

ttggtggtaa gtgaacagag tttccaaaat tcccaactcc cagcaatgct ttgtctgatt 2580

gttcatttgc agatgtctta gcgtgttaat ttaaatgtca aaggttttga ggtgtccaga 2640

accacctcca gaaaggggta gggtagaatg ccacctgttg cctggtgtgt gctaacctgg 2700

agcaggtagg ggtaagactc aatagtcatc ttttaccaaa tgggtttgcc ccaggttaat 2760

aagaggggtc tagtaggcct tggactgggc cgttgccaca cctggcactt agtgaccatc 2820

atcatgagaa actggagagt gcgtgctgga acacgtggtg ccatcttggc tttaggatcc 2880

ttttgatcgt tgtgtccaag gcttgtgtgt gtgtgagtgt gtgggagaca actccgaatg 2940

tttaattctg gaagagggat gtaacattgc cctgaggatg gtgaagttgg tatacattta 3000

taaagtacgg aatggtgtca atgaatgcaa ttctatgtat atggacttaa ctgagatggg 3060

caaatagaaa ctagctctgg gaaggaacat gtgcactact tcaagaaaga ttggaagcat 3120

gtgtggctca tgggaaataa ccaggtctta aacagcacaa actgaattcg tggaccagga 3180

aggtcttaaa cagcacaaac tgaattcatg gaaaaatgac aaatttgaga agtctcccag 3240

taagctggaa cttttctggt ttggttaaca aaaggtttct tgatttgttt caagatttaa 3300

agccaaaggt gtgggttcat gacttaggtg tcattgcgtg tgggtacaat atttatatat 3360

ggcgaattca gataaacatt ggtcaaagat ggtctctgga aaaacaaaat agaggctgca 3420

ttacggaaat aagatttctg gtctgttccc tgggacatgc ttaaaaaata caatagctat 3480

tatgtatggt ttttattttc atgtggtttc ggggaaacaa cacggtttta aggatggttt 3540

ctaaagatga aattaaaaat tgttccacaa gggttaagtg tctggtggta aagttgggag 3600

aaactggatg gatgcacatc gcatggctgg tggcgagccc atctctcttc tctcgggtga 3660

gagaaccggg ccaagctgag ttggtttgtt cactttaatg ggtctccgtt tcccctgcca 3720

cctgtgctga ggacatttcc cagcctgagc tgggggaggc agcatttgct gaagtgtgga 3780

gttgtctctg tggagactca agttacagat cttaaggggc ctgcctagaa ttttctcctc 3840

tgggcaggcg acccaggaaa gggtttggag tgaggctgtg agcacttact tgatatttta 3900

caagtttgga tttggtgtta atttttttcc ttgtccgttt tttcctgttg actaacggct 3960

catcttttcc ttgtttttgt tttttttttg ttcttttttt ccatgtcact aaaggccctc 4020

gggaccaagg atcacgtcat gactccgaac aggataattc agacaacaac accatctttg 4080

tgcaaggcct gggtgagaat gttacaattg agtctgtggc tgattacttc aagcagattg 4140

gtattattaa gacaaacaag aaaacgggac agcccatgat taatttgtac acagacaggg 4200

aaactggcaa gctgaaggga gaggcaacgg tctcttttga tgacccacct tcagctaaag 4260

cagctattga ctggtttgat ggtaaagaat tctccggaaa tcctatcaag gtctcatttg 4320

ctactcgccg ggcagacttt aatcggggtg gtggcaatgg tcgtggaggc cgagggcgag 4380

gaggacccat gggccgtgga ggctatggag gtggtggcag tggtggtggt ggccgaggag 4440

gatttcccag tggaggtggt ggcggtggag gacagcagcg agctggtgac tggaagtgtc 4500

ctaatcccac ctgtgagaat atgaacttct cttggaggaa tgaatgcaac cagtgtaagg 4560

cccctaaacc agatggccca ggagggggac caggtggctc tcacatgggg ggtaactacg 4620

gggatgatcg tcgtggtggc agaggaggct atgatcgagg cggctaccgg ggccgcggcg 4680

gggaccgtgg aggcttccga gggggccggg gtggtgggga cagaggtggc tttggccctg 4740

gcaagatgga ttccaggggt gagcacagac aggatcgcag ggagaggccg tattaa 4796

<210> 10
<211> 8229
<212> DNA
<213> Artificial Sequence

<220>
<223> sequence complète

<400> 10
acttaagctt cgacgcagga ggcggggctg ctcagtcctc caggcgtcgg tactcagcgg 60

tgttggaact tcgttgcttg cttgcctgtg cgcgcgtgcg cggacatggc ctcaaacgat 120

tatacccaac aagcaaccca aagctatggg gcctacccca cccagcccgg gcagggctat 180

tcccagcaga gcagtcagcc ctacggacag cagagttaca gtggttatag ccagtccacg 240

gacacttcag gctatggcca gagcagctat tcttcttatg gccagagcca gaacacaggc 300

tatggaactc agtcaactcc ccagggatat ggctcgactg gcggctatgg cagtagccag 360

agctcccaat cgtcttacgg gcagcagtcc tcctaccctg gctatggcca gcagccagct 420

cccagcagca cctcgggaag ttacggtagc agttctcaga gcagcagcta tgggcagccc 480

cagagtggga gctacagcca gcagcctagc tatggtggac agcagcaaag ctatggacag 540

cagcaaagct ataatccccc tcagggctat ggacagcaga accagtacaa cagcagcagt 600

ggtggtggag gtggaggtgg aggtggaggt aactatggcc aagatcaatc ctccatgagt 660

agtggtggtg gcagtggtgg cggttatggc aatcaagacc agagtggtgg aggtggcagc 720

ggtggctatg gacagcagga ccgtggaggc cgcggcaggg gtggcagtgg tggcggcggc 780

ggcggcggcg gtggtggtta caaccgcagc agtggtggct atgaacccag aggtcgtgga 840

ggtggccgtg gaggcagagg tggcatgggg taggtgtctc atgagccagg gagtatcttt 900

ggtggggagt gtggaggatt gcatgaatct ccctgaagcc agtccctagt gcatggttta 960

gtattcttgt tgtctaggga tctgtgaggg ctttgatttg ggggcagtga ctttcttttt 1020

acatccccat tttatttttg tgagaacttg ggagcctgaa ctcccatcca taccactgaa 1080

tagagatttt gagtaatgat acttgtttcc aaaaaaaaag aaaccataca tagatacgta 1140

tggattggag tcattaatat cctaggcaag aaacatggaa gtgaagactt ctttctctgc 1200

aagggaaacc gatgatccca ctcctgggaa atagtaggga aacttggtat gtgtattccc 1260

atgtgtcctc tagggagttg gtaatggtta acctgacttc agcttccagg aattggctac 1320

tcttcccgtt ttctatagtc atttgaatcc acgagcttga tttgcactaa tttgaccgac 1380

attgattttg tgtgtgactt ggtttatggg gccagctgac tgaagtaagc agaccttttg 1440

ggcaaaaata tgctttgaca gtggtctccc acctatttgt tccactgtct gccttcccct 1500

ggttacttaa aattcatcag cttgtccaac tggaccttct ttccttcctg ctgaagttga 1560

tttgaagtaa aaccttagat ttgatgttaa aacagttgtc aaatctgttg gtaaataaga 1620

tttgaaggac cctactctgt ctcccttgaa aaaggggagg aatgtcagtg ttactgtttt 1680

tggaaaaagt agatttttaa accgagtttg gaaatggtaa gtatgcagag gtgggtgggg 1740

gcaatctcaa aaacgtgcaa aaatgaggaa aacaaaaatg aggaaatgtg tgcgtgtgtt 1800

taatgcaaaa ctttaaaaag aaaaacaact gttatgtgac tgttaacttg ctctgcattt 1860

tatgtgccac aggtatgaaa ggtgacattg caaaatactc cgctcttctc gcagtgtaga 1920

aggggtgacc ccgggggttg ggggagatca aaaacagctc agtagttagg acagagctta 1980

gctaagtttg tcttgcttta aggggaagtt gcctttggtt ttgacttttt atggaatggg 2040

gttgggtctg cttgctgctt tcaaagcaaa aaccacaaaa atgtgttcaa ggctacccca 2100

gcctggtgtg aaatgtcttc tgggtaaatt ggggtagggt ttttaaacca actacttggt 2160

tgtcaaccac ttgcgacaag aggaaaaaaa aacatctgct ccatcggaag aacgaccaag 2220

gaaaatgggt tatttttttt ccagaggaaa tagataacgt aaccttttaa agcaaaatct 2280

ttataaactg tgtctgagaa attgcacacg tgtgtgtgac atgctcaaag gtcagacaag 2340

gggtggtcag gaagggatgt attttagtag ccacttgtat ctttttccaa aaacacctac 2400

ccatgtttgg ggaatgttaa acaaaatcaa aaaacaacct tttgtagccg ttggaagctt 2460

catgtccttt cttctaactt gtcttctcca gcggaagtga ccgtggtggc ttcaataaat 2520

ttggtggtaa gtgaacagag tttccaaaat tcccaactcc cagcaatgct ttgtctgatt 2580

gttcatttgc agatgtctta gcgtgttaat ttaaatgtca aaggttttga ggtgtccaga 2640

accacctcca gaaaggggta gggtagaatg ccacctgttg cctggtgtgt gctaacctgg 2700

agcaggtagg ggtaagactc aatagtcatc ttttaccaaa tgggtttgcc ccaggttaat 2760

aagaggggtc tagtaggcct tggactgggc cgttgccaca cctggcactt agtgaccatc 2820

atcatgagaa actggagagt gcgtgctgga acacgtggtg ccatcttggc tttaggatcc 2880

ttttgatcgt tgtgtccaag gcttgtgtgt gtgtgagtgt gtgggagaca actccgaatg 2940

tttaattctg gaagagggat gtaacattgc cctgaggatg gtgaagttgg tatacattta 3000

taaagtacgg aatggtgtca atgaatgcaa ttctatgtat atggacttaa ctgagatggg 3060

caaatagaaa ctagctctgg gaaggaacat gtgcactact tcaagaaaga ttggaagcat 3120

gtgtggctca tgggaaataa ccaggtctta aacagcacaa actgaattcg tggaccagga 3180

aggtcttaaa cagcacaaac tgaattcatg gaaaaatgac aaatttgaga agtctcccag 3240

taagctggaa cttttctggt ttggttaaca aaaggtttct tgatttgttt caagatttaa 3300

agccaaaggt gtgggttcat gacttaggtg tcattgcgtg tgggtacaat atttatatat 3360

ggcgaattca gataaacatt ggtcaaagat ggtctctgga aaaacaaaat agaggctgca 3420

ttacggaaat aagatttctg gtctgttccc tgggacatgc ttaaaaaata caatagctat 3480

tatgtatggt ttttattttc atgtggtttc ggggaaacaa cacggtttta aggatggttt 3540

ctaaagatga aattaaaaat tgttccacaa gggttaagtg tctggtggta aagttgggag 3600

aaactggatg gatgcacatc gcatggctgg tggcgagccc atctctcttc tctcgggtga 3660

gagaaccggg ccaagctgag ttggtttgtt cactttaatg ggtctccgtt tcccctgcca 3720

cctgtgctga ggacatttcc cagcctgagc tgggggaggc agcatttgct gaagtgtgga 3780

gttgtctctg tggagactca agttacagat cttaaggggc ctgcctagaa ttttctcctc 3840

tgggcaggcg acccaggaaa gggtttggag tgaggctgtg agcacttact tgatatttta 3900

caagtttgga tttggtgtta atttttttcc ttgtccgttt tttcctgttg actaacggct 3960

catcttttcc ttgtttttgt tttttttttg ttcttttttt ccatgtcact aaaggccctc 4020

gggaccaagg atcacgtcat gactccgaac aggataattc agacaacaac accatctttg 4080

tgcaaggcct gggtgagaat gttacaattg agtctgtggc tgattacttc aagcagattg 4140

gtattattaa gacaaacaag aaaacgggac agcccatgat taatttgtac acagacaggg 4200

aaactggcaa gctgaaggga gaggcaacgg tctcttttga tgacccacct tcagctaaag 4260

cagctattga ctggtttgat ggtaaagaat tctccggaaa tcctatcaag gtctcatttg 4320

ctactcgccg ggcagacttt aatcggggtg gtggcaatgg tcgtggaggc cgagggcgag 4380

gaggacccat gggccgtgga ggctatggag gtggtggcag tggtggtggt ggccgaggag 4440

gatttcccag tggaggtggt ggcggtggag gacagcagcg agctggtgac tggaagtgtc 4500

ctaatcccac ctgtgagaat atgaacttct cttggaggaa tgaatgcaac cagtgtaagg 4560

cccctaaacc agatggccca ggagggggac caggtggctc tcacatgggg ggtaactacg 4620

gggatgatcg tcgtggtggc agaggaggct atgatcgagg cggctaccgg ggccgcggcg 4680

gggaccgtgg aggcttccga gggggccggg gtggtgggga cagaggtggc tttggccctg 4740

gcaagatgga ttccaggggt gagcacagac aggatcgcag ggagaggccg tattaattag 4800

cctggctccc caggttctgg aacagctttt tgtcctgtac ccagtgttac cctcgttatt 4860

ttgtaacctt ccaattcctg atcacccaag ggtttttttg tgtcggacta tgtaattgta 4920

actatacctc tggttcccat taaaagtgac cattttagtt aaattttgtt cctcttcccc 4980

cttttcactt tcctggaaga tcgatgtccc gatcaggaag gtagagagtt ttcctgttca 5040

gattaccctg cccagcagga actggaatac agtgttcggg gagaaggcca aatgatatcc 5100

ttgagagcag agattaaact tttctgtcat ggggaaagtt ggtgtataaa tgagaaatga 5160

agaacatggg atgtcatgag tgttggccta aatttgccca gctatgggga atttttcctt 5220

taccacattt atttgcatac tggtcttagt ttatttgcag cagtttatcc ctttttaaga 5280

actctttgat cttttggccc ttttaatggt gaggctcaaa caaactacat ttaaatgggg 5340

cagtattcag atttgaccat ggtggagagc gcttagccac tctgggtctt tcacaggaag 5400

gagagtaact gagtgctgca ggagtttgtg gagtggagtc aggatctagg aggtgagtga 5460

ctcccttcct agctgccctg gtgaacagcg cttgggtaga tacctgctat aaggagactg 5520

gtctggctgg gttactttca catcctgcct gtactcagag ggcttgaggt cattgacatt 5580

atgagatttt aggcttgatc cctttttgat tggagggtgg aaggccctcc taagggaatg 5640

ataagtgata agagggggaa ggggttgcag ccaatgagtt aaaaccttag agcagtgctc 5700

ctcagcctct taccatgtgg ttgtaaactt gcacgtacct gccaaccagt tatttagcat 5760

gctttttatt ttagttacac agagcgtaac attaacccaa gagcagaaag gttttattta 5820

cagggttttc gaacttggtt tgtaagacag ctgccatcac aagcatagct tacaaatgtg 5880

ctggggaccc ctaattggga agtgctttcc tctcaaattt ttatttttta tttttagaga 5940

cagagtcttg ctctgtcatc caggctggag tgcagtggcg tgatctcggc tcactgtagc 6000

ctctgcctcc tgagtttaag cgattctcct gcctcaggag aatcccagct tctgagtagc 6060

tgagactaca ggcgtgggcc accatgccca cctagttttt gcatttttag tggaggtgtg 6120

gtttcactgt gttggccagg ctggtctctt aactcctgac ctcaggtgat ccacctgcct 6180

tggcctccca aagtgctgga attacaggca tgagccgctg catctggcca tcctctcaaa 6240

ttttcaagtg ttccacaagt atgttctcta ctgaagagtt gctgcatcct tgaatcttgg 6300

gtgatttgag gcacagaaac tatgacttta ttttttgaga tggagttttg ctcttgttgc 6360

ccaggctgga gtgcaatggc acgtttttcg gcttaccgca actgccgcct cctgggttca 6420

agcgatagct gggattacag gcatgcgcca ccatgcccag ctaatttatt tgtattttta 6480

gtagagacgg ggtttctcca tgttggtcag gctggtctcg aactcccgac ctcaggtgat 6540

gtgcacacct cagcctccca ataaaccatg acttttaaga ggaatagcag gtttacttcc 6600

cctgccagca ttggggtgct ctctaagcaa cagtaggcgg agagtggtct ggcgtattaa 6660

aaacaaagga tcgtcaagtg ggccttccca ggcattgctt tgacttagta catgtagagg 6720

atgtggcagt tctctccgtc cctgccactg ctggtttctt tgttaaatgt ttagttgaaa 6780

tggcctgata cgatatttga gtagttcact gttggtgctt tgcctagcag gattctaatc 6840

ttgctttggt tgtggtcccc tgatgccctc ctgttaggag tggaggaggt cgaagctcct 6900

tgtaagatat gattactggg accattagtg tcaagttcct gtgtccttca aatggcatat 6960

gtgattggcc ttgaccttaa aaggaaatag ggtcccaggt gactgtttag tgggtaggtc 7020

cagtttgggg ggatcttcca ggagaatgga tagagacacc tagcagcaga gagaacattg 7080

gtgcctctca agccaacctc ccacctcagc ctcccaagta gctgggacta caggtgcttc 7140

ctcgctacca cacctgggta attttttttt taattacttt ttttttttta agaaacaggg 7200

tttcactggg ttgcccaggc tggtcttgaa ctggcctcaa gtgacctgcc tgcctcagcc 7260

tcccaaagtg ctgggatcac aggtgtgacc cactgcgttt ggccagaata ctctattctt 7320

actgaatgat tgaaatctgt cttgaagcat taggtgtccc atttttgtga gttggaattg 7380

ggacaggcta agtaggaagt gaggagggtg gggagagctg tgctgtaggt ctgtttgtcc 7440

cttccttgat gtagccttca gttagccctt tcagcttttt tccccatctt gtgccgggcc 7500

ttcctgggtt tcagtacttg gatgtagggc tgcagttatg tcagtggtgg gtagattgac 7560

caggaattaa ggtctagggt ccagcccatg tgagacttga ctcactgatc tacctttagg 7620

catgtcttcc ttccagtctc atccttttta aatttttttt ttttttttga gacggtctca 7680

ctctcaccca ggctggattg cagtggtgtg atctcgacca actgcaacct ctgcctccca 7740

cccgcaagct atctgcccac ctcagcctct ggagtagctg ggacgggact acaggcacct 7800

gccaccatga ctggctaatt tttttttgta ttttttgtgg agatggggtc ttgccatgtt 7860

gctcaggctg gtctggtctc aaaactgctc tgggctcaag tgattgtccc actttggcct 7920

cccagagtgc tgggattaag gtgtgagata ctgtgtccgg ctatgaaaat tttattttta 7980

attaacttgt atatatttat ggggtacaat gtcctatttc tgtacatgta cacattgtgg 8040

aatcaaatca ggctaatata tccatcactt catatcatta gcatgaatga gaacatacaa 8100

agccactctt agaaaatttt gaaatttatg ttatttcagc ccttttatgc tggaggttgc 8160

aaatgttttg tgaataatca gaccaaaaat aaaaacaaaa aatgattgac ttcagtcatt 8220

cagtaagaa 8229

<210> 11
<211> 7932
<212> DNA
<213> Artificial Sequence

<220>
<223> sequence complete delta 1

<400> 11
acttaagctt cgacgcagga ggcggggctg ctcagtcctc caggcgtcgg tactcagcgg 60

tgttggaact tcgttgcttg cttgcctgtg cgcgcgtgcg cggacatggc ctcaaacgat 120

tatacccaac aagcaaccca aagctatggg gcctacccca cccagcccgg gcagggctat 180

tcccagcaga gcagtcagcc ctacggacag cagagttaca gtggttatag ccagtccacg 240

gacacttcag gctatggcca gagcagctat tcttcttatg gccagagcca gaacacaggc 300

tatggaactc agtcaactcc ccagggatat ggctcgactg gcggctatgg cagtagccag 360

agctcccaat cgtcttacgg gcagcagtcc tcctaccctg gctatggcca gcagccagct 420

cccagcagca cctcgggaag ttacggtagc agttctcaga gcagcagcta tgggcagccc 480

cagagtggga gctacagcca gcagcctagc tatggtggac agcagcaaag ctatggacag 540

cagcaaagct ataatccccc tcagggctat ggacagcaga accagtacaa cagcagcagt 600

ggtggtggag gtggaggtgg aggtggaggt aactatggcc aagatcaatc ctccatgagt 660

agtggtggtg gcagtggtgg cggttatggc aatcaagacc agagtggtgg aggtggcagc 720

ggtggctatg gacagcagga ccgtggaggc cgcggcaggg gtggcagtgg tggcggcggc 780

ggcggcggcg gtggtggtta caaccgcagc agtggtggct atgaacccag aggtcgtgga 840

ggtggccgtg gaggcagagg tggcatgggg taggtgtctc atgagccagg gagtatcttt 900

ggtggggagt gtggaggatt gcatgaatct ccctgaagcc agtccctagt gcatggttta 960

gtattcttgt tgtctaggga tctgtgaggg ctttgatttg ggggcagtga ctttcttttt 1020

acatccccat tttatttttg tgagaacttg ggagcctgaa ctcccatcca taccactgaa 1080

tagagatttt gagtaatgat acttgtttcc aaaaaaaaag aaaccataca tagatacgta 1140

tggattggag tcattaatat cctaggcaag aaacatggaa gtgaagactt ctttctctgc 1200

aagggaaacc gatgatccca ctcctgggaa atagtaggga aacttggtat gtgtattccc 1260

atgtgtgaag taaaacctta gatttgatgt taaaacagtt gtcaaatctg ttggtaaata 1320

agatttgaag gaccctactc tgtctccctt gaaaaagggg aggaatgtca gtgttactgt 1380

ttttggaaaa agtagatttt taaaccgagt ttggaaatgg taagtatgca gaggtgggtg 1440

ggggcaatct caaaaacgtg caaaaatgag gaaaacaaaa atgaggaaat gtgtgcgtgt 1500

gtttaatgca aaactttaaa aagaaaaaca actgttatgt gactgttaac ttgctctgca 1560

ttttatgtgc cacaggtatg aaaggtgaca ttgcaaaata ctccgctctt ctcgcagtgt 1620

agaaggggtg accccggggg ttgggggaga tcaaaaacag ctcagtagtt aggacagagc 1680

ttagctaagt ttgtcttgct ttaaggggaa gttgcctttg gttttgactt tttatggaat 1740

ggggttgggt ctgcttgctg ctttcaaagc aaaaaccaca aaaatgtgtt caaggctacc 1800

ccagcctggt gtgaaatgtc ttctgggtaa attggggtag ggtttttaaa ccaactactt 1860

ggttgtcaac cacttgcgac aagaggaaaa aaaaacatct gctccatcgg aagaacgacc 1920

aaggaaaatg ggttattttt tttccagagg aaatagataa cgtaaccttt taaagcaaaa 1980

tctttataaa ctgtgtctga gaaattgcac acgtgtgtgt gacatgctca aaggtcagac 2040

aaggggtggt caggaaggga tgtattttag tagccacttg tatctttttc caaaaacacc 2100

tacccatgtt tggggaatgt taaacaaaat caaaaaacaa ccttttgtag ccgttggaag 2160

cttcatgtcc tttcttctaa cttgtcttct ccagcggaag tgaccgtggt ggcttcaata 2220

aatttggtgg taagtgaaca gagtttccaa aattcccaac tcccagcaat gctttgtctg 2280

attgttcatt tgcagatgtc ttagcgtgtt aatttaaatg tcaaaggttt tgaggtgtcc 2340

agaaccacct ccagaaaggg gtagggtaga atgccacctg ttgcctggtg tgtgctaacc 2400

tggagcaggt aggggtaaga ctcaatagtc atcttttacc aaatgggttt gccccaggtt 2460

aataagaggg gtctagtagg ccttggactg ggccgttgcc acacctggca cttagtgacc 2520

atcatcatga gaaactggag agtgcgtgct ggaacacgtg gtgccatctt ggctttagga 2580

tccttttgat cgttgtgtcc aaggcttgtg tgtgtgtgag tgtgtgggag acaactccga 2640

atgtttaatt ctggaagagg gatgtaacat tgccctgagg atggtgaagt tggtatacat 2700

ttataaagta cggaatggtg tcaatgaatg caattctatg tatatggact taactgagat 2760

gggcaaatag aaactagctc tgggaaggaa catgtgcact acttcaagaa agattggaag 2820

catgtgtggc tcatgggaaa taaccaggtc ttaaacagca caaactgaat tcgtggacca 2880

ggaaggtctt aaacagcaca aactgaattc atggaaaaat gacaaatttg agaagtctcc 2940

cagtaagctg gaacttttct ggtttggtta acaaaaggtt tcttgatttg tttcaagatt 3000

taaagccaaa ggtgtgggtt catgacttag gtgtcattgc gtgtgggtac aatatttata 3060

tatggcgaat tcagataaac attggtcaaa gatggtctct ggaaaaacaa aatagaggct 3120

gcattacgga aataagattt ctggtctgtt ccctgggaca tgcttaaaaa atacaatagc 3180

tattatgtat ggtttttatt ttcatgtggt ttcggggaaa caacacggtt ttaaggatgg 3240

tttctaaaga tgaaattaaa aattgttcca caagggttaa gtgtctggtg gtaaagttgg 3300

gagaaactgg atggatgcac atcgcatggc tggtggcgag cccatctctc ttctctcggg 3360

tgagagaacc gggccaagct gagttggttt gttcacttta atgggtctcc gtttcccctg 3420

ccacctgtgc tgaggacatt tcccagcctg agctggggga ggcagcattt gctgaagtgt 3480

ggagttgtct ctgtggagac tcaagttaca gatcttaagg ggcctgccta gaattttctc 3540

ctctgggcag gcgacccagg aaagggtttg gagtgaggct gtgagcactt acttgatatt 3600

ttacaagttt ggatttggtg ttaatttttt tccttgtccg ttttttcctg ttgactaacg 3660

gctcatcttt tccttgtttt tgtttttttt ttgttctttt tttccatgtc actaaaggcc 3720

ctcgggacca aggatcacgt catgactccg aacaggataa ttcagacaac aacaccatct 3780

ttgtgcaagg cctgggtgag aatgttacaa ttgagtctgt ggctgattac ttcaagcaga 3840

ttggtattat taagacaaac aagaaaacgg gacagcccat gattaatttg tacacagaca 3900

gggaaactgg caagctgaag ggagaggcaa cggtctcttt tgatgaccca ccttcagcta 3960

aagcagctat tgactggttt gatggtaaag aattctccgg aaatcctatc aaggtctcat 4020

ttgctactcg ccgggcagac tttaatcggg gtggtggcaa tggtcgtgga ggccgagggc 4080

gaggaggacc catgggccgt ggaggctatg gaggtggtgg cagtggtggt ggtggccgag 4140

gaggatttcc cagtggaggt ggtggcggtg gaggacagca gcgagctggt gactggaagt 4200

gtcctaatcc cacctgtgag aatatgaact tctcttggag gaatgaatgc aaccagtgta 4260

aggcccctaa accagatggc ccaggagggg gaccaggtgg ctctcacatg gggggtaact 4320

acggggatga tcgtcgtggt ggcagaggag gctatgatcg aggcggctac cggggccgcg 4380

gcggggaccg tggaggcttc cgagggggcc ggggtggtgg ggacagaggt ggctttggcc 4440

ctggcaagat ggattccagg ggtgagcaca gacaggatcg cagggagagg ccgtattaat 4500

tagcctggct ccccaggttc tggaacagct ttttgtcctg tacccagtgt taccctcgtt 4560

attttgtaac cttccaattc ctgatcaccc aagggttttt ttgtgtcgga ctatgtaatt 4620

gtaactatac ctctggttcc cattaaaagt gaccatttta gttaaatttt gttcctcttc 4680

ccccttttca ctttcctgga agatcgatgt cccgatcagg aaggtagaga gttttcctgt 4740

tcagattacc ctgcccagca ggaactggaa tacagtgttc ggggagaagg ccaaatgata 4800

tccttgagag cagagattaa acttttctgt catggggaaa gttggtgtat aaatgagaaa 4860

tgaagaacat gggatgtcat gagtgttggc ctaaatttgc ccagctatgg ggaatttttc 4920

ctttaccaca tttatttgca tactggtctt agtttatttg cagcagttta tcccttttta 4980

agaactcttt gatcttttgg cccttttaat ggtgaggctc aaacaaacta catttaaatg 5040

gggcagtatt cagatttgac catggtggag agcgcttagc cactctgggt ctttcacagg 5100

aaggagagta actgagtgct gcaggagttt gtggagtgga gtcaggatct aggaggtgag 5160

tgactccctt cctagctgcc ctggtgaaca gcgcttgggt agatacctgc tataaggaga 5220

ctggtctggc tgggttactt tcacatcctg cctgtactca gagggcttga ggtcattgac 5280

attatgagat tttaggcttg atcccttttt gattggaggg tggaaggccc tcctaaggga 5340

atgataagtg ataagagggg gaaggggttg cagccaatga gttaaaacct tagagcagtg 5400

ctcctcagcc tcttaccatg tggttgtaaa cttgcacgta cctgccaacc agttatttag 5460

catgcttttt attttagtta cacagagcgt aacattaacc caagagcaga aaggttttat 5520

ttacagggtt ttcgaacttg gtttgtaaga cagctgccat cacaagcata gcttacaaat 5580

gtgctgggga cccctaattg ggaagtgctt tcctctcaaa tttttatttt ttatttttag 5640

agacagagtc ttgctctgtc atccaggctg gagtgcagtg gcgtgatctc ggctcactgt 5700

agcctctgcc tcctgagttt aagcgattct cctgcctcag gagaatccca gcttctgagt 5760

agctgagact acaggcgtgg gccaccatgc ccacctagtt tttgcatttt tagtggaggt 5820

gtggtttcac tgtgttggcc aggctggtct cttaactcct gacctcaggt gatccacctg 5880

ccttggcctc ccaaagtgct ggaattacag gcatgagccg ctgcatctgg ccatcctctc 5940

aaattttcaa gtgttccaca agtatgttct ctactgaaga gttgctgcat ccttgaatct 6000

tgggtgattt gaggcacaga aactatgact ttattttttg agatggagtt ttgctcttgt 6060

tgcccaggct ggagtgcaat ggcacgtttt tcggcttacc gcaactgccg cctcctgggt 6120

tcaagcgata gctgggatta caggcatgcg ccaccatgcc cagctaattt atttgtattt 6180

ttagtagaga cggggtttct ccatgttggt caggctggtc tcgaactccc gacctcaggt 6240

gatgtgcaca cctcagcctc ccaataaacc atgactttta agaggaatag caggtttact 6300

tcccctgcca gcattggggt gctctctaag caacagtagg cggagagtgg tctggcgtat 6360

taaaaacaaa ggatcgtcaa gtgggccttc ccaggcattg ctttgactta gtacatgtag 6420

aggatgtggc agttctctcc gtccctgcca ctgctggttt ctttgttaaa tgtttagttg 6480

aaatggcctg atacgatatt tgagtagttc actgttggtg ctttgcctag caggattcta 6540

atcttgcttt ggttgtggtc ccctgatgcc ctcctgttag gagtggagga ggtcgaagct 6600

ccttgtaaga tatgattact gggaccatta gtgtcaagtt cctgtgtcct tcaaatggca 6660

tatgtgattg gccttgacct taaaaggaaa tagggtccca ggtgactgtt tagtgggtag 6720

gtccagtttg gggggatctt ccaggagaat ggatagagac acctagcagc agagagaaca 6780

ttggtgcctc tcaagccaac ctcccacctc agcctcccaa gtagctggga ctacaggtgc 6840

ttcctcgcta ccacacctgg gtaatttttt ttttaattac tttttttttt ttaagaaaca 6900

gggtttcact gggttgccca ggctggtctt gaactggcct caagtgacct gcctgcctca 6960

gcctcccaaa gtgctgggat cacaggtgtg acccactgcg tttggccaga atactctatt 7020

cttactgaat gattgaaatc tgtcttgaag cattaggtgt cccatttttg tgagttggaa 7080

ttgggacagg ctaagtagga agtgaggagg gtggggagag ctgtgctgta ggtctgtttg 7140

tcccttcctt gatgtagcct tcagttagcc ctttcagctt ttttccccat cttgtgccgg 7200

gccttcctgg gtttcagtac ttggatgtag ggctgcagtt atgtcagtgg tgggtagatt 7260

gaccaggaat taaggtctag ggtccagccc atgtgagact tgactcactg atctaccttt 7320

aggcatgtct tccttccagt ctcatccttt ttaaattttt tttttttttt tgagacggtc 7380

tcactctcac ccaggctgga ttgcagtggt gtgatctcga ccaactgcaa cctctgcctc 7440

ccacccgcaa gctatctgcc cacctcagcc tctggagtag ctgggacggg actacaggca 7500

cctgccacca tgactggcta attttttttt gtattttttg tggagatggg gtcttgccat 7560

gttgctcagg ctggtctggt ctcaaaactg ctctgggctc aagtgattgt cccactttgg 7620

cctcccagag tgctgggatt aaggtgtgag atactgtgtc cggctatgaa aattttattt 7680

ttaattaact tgtatatatt tatggggtac aatgtcctat ttctgtacat gtacacattg 7740

tggaatcaaa tcaggctaat atatccatca cttcatatca ttagcatgaa tgagaacata 7800

caaagccact cttagaaaat tttgaaattt atgttatttc agccctttta tgctggaggt 7860

tgcaaatgtt ttgtgaataa tcagaccaaa aataaaaaca aaaaatgatt gacttcagtc 7920

attcagtaag aa 7932

<210> 12
<211> 8031
<212> DNA
<213> Artificial Sequence

<220>
<223> sequence delta 2

<400> 12
acttaagctt cgacgcagga ggcggggctg ctcagtcctc caggcgtcgg tactcagcgg 60

tgttggaact tcgttgcttg cttgcctgtg cgcgcgtgcg cggacatggc ctcaaacgat 120

tatacccaac aagcaaccca aagctatggg gcctacccca cccagcccgg gcagggctat 180

tcccagcaga gcagtcagcc ctacggacag cagagttaca gtggttatag ccagtccacg 240

gacacttcag gctatggcca gagcagctat tcttcttatg gccagagcca gaacacaggc 300

tatggaactc agtcaactcc ccagggatat ggctcgactg gcggctatgg cagtagccag 360

agctcccaat cgtcttacgg gcagcagtcc tcctaccctg gctatggcca gcagccagct 420

cccagcagca cctcgggaag ttacggtagc agttctcaga gcagcagcta tgggcagccc 480

cagagtggga gctacagcca gcagcctagc tatggtggac agcagcaaag ctatggacag 540

cagcaaagct ataatccccc tcagggctat ggacagcaga accagtacaa cagcagcagt 600

ggtggtggag gtggaggtgg aggtggaggt aactatggcc aagatcaatc ctccatgagt 660

agtggtggtg gcagtggtgg cggttatggc aatcaagacc agagtggtgg aggtggcagc 720

ggtggctatg gacagcagga ccgtggaggc cgcggcaggg gtggcagtgg tggcggcggc 780

ggcggcggcg gtggtggtta caaccgcagc agtggtggct atgaacccag aggtcgtgga 840

ggtggccgtg gaggcagagg tggcatgggg taggtgtctc atgagccagg gagtatcttt 900

ggtggggagt gtggaggatt gcatgaatct ccctgaagcc agtccctagt gcatggttta 960

gtattcttgt tgtctaggga tctgtgaggg ctttgatttg ggggcagtga ctttcttttt 1020

acatccccat tttatttttg tgagaacttg ggagcctgaa ctcccatcca taccactgaa 1080

tagagatttt gagtaatgat acttgtttcc aaaaaaaaag aaaccataca tagatacgta 1140

tggattggag tcattaatat cctaggcaag aaacatggaa gtgaagactt ctttctctgc 1200

aagggaaacc gatgatccca ctcctgggaa atagtaggga aacttggtat gtgtattccc 1260

atgtgtcctc tagggagttg gtaatggtta acctgacttc agcttccagg aattggctac 1320

tcttcccgtt ttctatagtc atttgaatcc acgagcttga tttgcactaa tttgaccgac 1380

attgattttg tgtgtgactt ggtttatggg gccagctgac tgaagtaagc agaccttttg 1440

ggcaaaaata tgctttgaca gtggtctccc acctatttgt tccactgtct gccttcccct 1500

ggttacttaa aattcatcag cttgtccaac tggaccttct ttccttcctg ctgaagttga 1560

tttgaagtaa aaccttagat ttgatgttaa aacagttgtc aaatctgttg gtaaataaga 1620

tttgaaggac cctactctgt ctcccttgaa aaaggggagg aatgtcagtg ttactgtttt 1680

tggaaaaagt agatttttaa accgagtttg gaaatggtaa gtatgcagag gtgggtgggg 1740

gcaatctcaa aaacgtgcaa aaatgaggaa aacaaaaatg aggaaatgtg tgcgtgtgtt 1800

taatgcaaaa ctttaaaaag aaaaacaact gttatgtgac tgttaacttg ctctgcattc 1860

tttcaaagca aaaaccacaa aaatgtgttc aaggctaccc cagcctggtg tgaaatgtct 1920

tctgggtaaa ttggggtagg gtttttaaac caactacttg gttgtcaacc acttgcgaca 1980

agaggaaaaa aaaacatctg ctccatcgga agaacgacca aggaaaatgg gttatttttt 2040

ttccagagga aatagataac gtaacctttt aaagcaaaat ctttataaac tgtgtctgag 2100

aaattgcaca cgtgtgtgtg acatgctcaa aggtcagaca aggggtggtc aggaagggat 2160

gtattttagt agccacttgt atctttttcc aaaaacacct acccatgttt ggggaatgtt 2220

aaacaaaatc aaaaaacaac cttttgtagc cgttggaagc ttcatgtcct ttcttctaac 2280

ttgtcttctc cagcggaagt gaccgtggtg gcttcaataa atttggtggt aagtgaacag 2340

agtttccaaa attcccaact cccagcaatg ctttgtctga ttgttcattt gcagatgtct 2400

tagcgtgtta atttaaatgt caaaggtttt gaggtgtcca gaaccacctc cagaaagggg 2460

tagggtagaa tgccacctgt tgcctggtgt gtgctaacct ggagcaggta ggggtaagac 2520

tcaatagtca tcttttacca aatgggtttg ccccaggtta ataagagggg tctagtaggc 2580

cttggactgg gccgttgcca cacctggcac ttagtgacca tcatcatgag aaactggaga 2640

gtgcgtgctg gaacacgtgg tgccatcttg gctttaggat ccttttgatc gttgtgtcca 2700

aggcttgtgt gtgtgtgagt gtgtgggaga caactccgaa tgtttaattc tggaagaggg 2760

atgtaacatt gccctgagga tggtgaagtt ggtatacatt tataaagtac ggaatggtgt 2820

caatgaatgc aattctatgt atatggactt aactgagatg ggcaaataga aactagctct 2880

gggaaggaac atgtgcacta cttcaagaaa gattggaagc atgtgtggct catgggaaat 2940

aaccaggtct taaacagcac aaactgaatt cgtggaccag gaaggtctta aacagcacaa 3000

actgaattca tggaaaaatg acaaatttga gaagtctccc agtaagctgg aacttttctg 3060

gtttggttaa caaaaggttt cttgatttgt ttcaagattt aaagccaaag gtgtgggttc 3120

atgacttagg tgtcattgcg tgtgggtaca atatttatat atggcgaatt cagataaaca 3180

ttggtcaaag atggtctctg gaaaaacaaa atagaggctg cattacggaa ataagatttc 3240

tggtctgttc cctgggacat gcttaaaaaa tacaatagct attatgtatg gtttttattt 3300

tcatgtggtt tcggggaaac aacacggttt taaggatggt ttctaaagat gaaattaaaa 3360

attgttccac aagggttaag tgtctggtgg taaagttggg agaaactgga tggatgcaca 3420

tcgcatggct ggtggcgagc ccatctctct tctctcgggt gagagaaccg ggccaagctg 3480

agttggtttg ttcactttaa tgggtctccg tttcccctgc cacctgtgct gaggacattt 3540

cccagcctga gctgggggag gcagcatttg ctgaagtgtg gagttgtctc tgtggagact 3600

caagttacag atcttaaggg gcctgcctag aattttctcc tctgggcagg cgacccagga 3660

aagggtttgg agtgaggctg tgagcactta cttgatattt tacaagtttg gatttggtgt 3720

taattttttt ccttgtccgt tttttcctgt tgactaacgg ctcatctttt ccttgttttt 3780

gttttttttt tgttcttttt ttccatgtca ctaaaggccc tcgggaccaa ggatcacgtc 3840

atgactccga acaggataat tcagacaaca acaccatctt tgtgcaaggc ctgggtgaga 3900

atgttacaat tgagtctgtg gctgattact tcaagcagat tggtattatt aagacaaaca 3960

agaaaacggg acagcccatg attaatttgt acacagacag ggaaactggc aagctgaagg 4020

gagaggcaac ggtctctttt gatgacccac cttcagctaa agcagctatt gactggtttg 4080

atggtaaaga attctccgga aatcctatca aggtctcatt tgctactcgc cgggcagact 4140

ttaatcgggg tggtggcaat ggtcgtggag gccgagggcg aggaggaccc atgggccgtg 4200

gaggctatgg aggtggtggc agtggtggtg gtggccgagg aggatttccc agtggaggtg 4260

gtggcggtgg aggacagcag cgagctggtg actggaagtg tcctaatccc acctgtgaga 4320

atatgaactt ctcttggagg aatgaatgca accagtgtaa ggcccctaaa ccagatggcc 4380

caggaggggg accaggtggc tctcacatgg ggggtaacta cggggatgat cgtcgtggtg 4440

gcagaggagg ctatgatcga ggcggctacc ggggccgcgg cggggaccgt ggaggcttcc 4500

gagggggccg gggtggtggg gacagaggtg gctttggccc tggcaagatg gattccaggg 4560

gtgagcacag acaggatcgc agggagaggc cgtattaatt agcctggctc cccaggttct 4620

ggaacagctt tttgtcctgt acccagtgtt accctcgtta ttttgtaacc ttccaattcc 4680

tgatcaccca agggtttttt tgtgtcggac tatgtaattg taactatacc tctggttccc 4740

attaaaagtg accattttag ttaaattttg ttcctcttcc cccttttcac tttcctggaa 4800

gatcgatgtc ccgatcagga aggtagagag ttttcctgtt cagattaccc tgcccagcag 4860

gaactggaat acagtgttcg gggagaaggc caaatgatat ccttgagagc agagattaaa 4920

cttttctgtc atggggaaag ttggtgtata aatgagaaat gaagaacatg ggatgtcatg 4980

agtgttggcc taaatttgcc cagctatggg gaatttttcc tttaccacat ttatttgcat 5040

actggtctta gtttatttgc agcagtttat ccctttttaa gaactctttg atcttttggc 5100

ccttttaatg gtgaggctca aacaaactac atttaaatgg ggcagtattc agatttgacc 5160

atggtggaga gcgcttagcc actctgggtc tttcacagga aggagagtaa ctgagtgctg 5220

caggagtttg tggagtggag tcaggatcta ggaggtgagt gactcccttc ctagctgccc 5280
tggtgaacag cgcttgggta gatacctgct ataaggagac tggtctggct gggttacttt 5340
cacatcctgc ctgtactcag agggcttgag gtcattgaca ttatgagatt ttaggcttga 5400
tccctttttg attggagggt ggaaggccct cctaagggaa tgataagtga taagaggggg 5460
aaggggttgc agccaatgag ttaaaacctt agagcagtgc tcctcagcct cttaccatgt 5520
ggttgtaaac ttgcacgtac ctgccaacca gttatttagc atgcttttta ttttagttac 5580
acagagcgta acattaaccc aagagcagaa aggttttatt tacagggttt tcgaacttgg 5640
tttgtaagac agctgccatc acaagcatag cttacaaatg tgctggggac ccctaattgg 5700
gaagtgcttt cctctcaaat ttttattttt tatttttaga gacagagtct tgctctgtca 5760
tccaggctgg agtgcagtgg cgtgatctcg gctcactgta gcctctgcct cctgagttta 5820
agcgattctc ctgcctcagg agaatcccag cttctgagta gctgagacta caggcgtggg 5880
ccaccatgcc cacctagttt ttgcattttt agtggaggtg tggtttcact gtgttggcca 5940
ggctggtctc ttaactcctg acctcaggtg atccacctgc cttggcctcc caaagtgctg 6000
gaattacagg catgagccgc tgcatctggc catcctctca aattttcaag tgttccacaa 6060
gtatgttctc tactgaagag ttgctgcatc cttgaatctt gggtgatttg aggcacagaa 6120
actatgactt tattttttga gatggagttt tgctcttgtt gcccaggctg gagtgcaatg 6180
gcacgttttt cggcttaccg caactgccgc ctcctgggtt caagcgatag ctgggattac 6240
aggcatgcgc caccatgccc agctaattta tttgtatttt tagtagagac ggggtttctc 6300
catgttggtc aggctggtct cgaactcccg acctcaggtg atgtgcacac ctcagcctcc 6360
caataaacca tgacttttaa gaggaatagc aggtttactt cccctgccag cattggggtg 6420
ctctctaagc aacagtaggc ggagagtggt ctggcgtatt aaaaacaaag gatcgtcaag 6480
tgggccttcc caggcattgc tttgacttag tacatgtaga ggatgtggca gttctctccg 6540
tccctgccac tgctggtttc tttgttaaat gtttagttga aatggcctga tacgatattt 6600
gagtagttca ctgttggtgc tttgcctagc aggattctaa tcttgctttg gttgtggtcc 6660
cctgatgccc tcctgttagg agtggaggag gtcgaagctc cttgtaagat atgattactg 6720
ggaccattag tgtcaagttc ctgtgtcctt caaatggcat atgtgattgg ccttgacctt 6780
aaaaggaaat agggtcccag gtgactgttt agtgggtagg tccagtttgg ggggatcttc 6840
caggagaatg gatagagaca cctagcagca gagagaacat tggtgcctct caagccaacc 6900
tcccacctca gcctcccaag tagctgggac tacaggtgct tcctcgctac cacacctggg 6960
taattttttt tttaattact tttttttttt taagaaacag ggtttcactg ggttgcccag 7020
gctggtcttg aactggcctc aagtgacctg cctgcctcag cctcccaaag tgctgggatc 7080
acaggtgtga cccactgcgt ttggccagaa tactctattc ttactgaatg attgaaatct 7140

gtcttgaagc attaggtgtc ccatttttgt gagttggaat tgggacaggc taagtaggaa 7200

gtgaggaggg tggggagagc tgtgctgtag gtctgtttgt cccttccttg atgtagcctt 7260

cagttagccc tttcagcttt tttccccatc ttgtgccggg ccttcctggg tttcagtact 7320

tggatgtagg gctgcagtta tgtcagtggt gggtagattg accaggaatt aaggtctagg 7380

gtccagccca tgtgagactt gactcactga tctaccttta ggcatgtctt ccttccagtc 7440

tcatcctttt taaatttttt tttttttttt gagacggtct cactctcacc caggctggat 7500

tgcagtggtg tgatctcgac caactgcaac ctctgcctcc cacccgcaag ctatctgccc 7560

acctcagcct ctggagtagc tgggacggga ctacaggcac ctgccaccat gactggctaa 7620

tttttttttg tattttttgt ggagatgggg tcttgccatg ttgctcaggc tggtctggtc 7680

tcaaaactgc tctgggctca agtgattgtc ccactttggc ctcccagagt gctgggatta 7740

aggtgtgaga tactgtgtcc ggctatgaaa attttatttt taattaactt gtatatattt 7800

atggggtaca atgtcctatt tctgtacatg tacacattgt ggaatcaaat caggctaata 7860

tatccatcac ttcatatcat tagcatgaat gagaacatac aaagccactc ttagaaaatt 7920

ttgaaattta tgttatttca gcccttttat gctggaggtt gcaaatgttt tgtgaataat 7980

cagaccaaaa ataaaaacaa aaaatgattg acttcagtca ttcagtaaga a 8031

<210> 13
<211> 8031
<212> DNA
<213> Artificial Sequence

<220>
<223> sequence delta 3

<400> 13
acttaagctt cgacgcagga ggcggggctg ctcagtcctc caggcgtcgg tactcagcgg 60

tgttggaact tcgttgcttg cttgcctgtg cgcgcgtgcg cggacatggc ctcaaacgat 120

tatacccaac aagcaaccca aagctatggg gcctacccca cccagcccgg gcagggctat 180

tcccagcaga gcagtcagcc ctacggacag cagagttaca gtggttatag ccagtccacg 240

gacacttcag gctatggcca gagcagctat tcttcttatg gccagagcca gaacacaggc 300

tatggaactc agtcaactcc ccagggatat ggctcgactg gcggctatgg cagtagccag 360

agctcccaat cgtcttacgg gcagcagtcc tcctaccctg gctatggcca gcagccagct 420

cccagcagca cctcgggaag ttacggtagc agttctcaga gcagcagcta tgggcagccc 480

cagagtggga gctacagcca gcagcctagc tatggtggac agcagcaaag ctatggacag 540

cagcaaagct ataatccccc tcagggctat ggacagcaga accagtacaa cagcagcagt 600

ggtggtggag gtggaggtgg aggtggaggt aactatggcc aagatcaatc ctccatgagt 660

agtggtggtg gcagtggtgg cggttatggc aatcaagacc agagtggtgg aggtggcagc 720

ggtggctatg gacagcagga ccgtggaggc cgcggcaggg gtggcagtgg tggcggcggc 780

ggcggcggcg gtggtggtta caaccgcagc agtggtggct atgaacccag aggtcgtgga 840

ggtggccgtg gaggcagagg tggcatgggg taggtgtctc atgagccagg gagtatcttt 900

ggtggggagt gtggaggatt gcatgaatct ccctgaagcc agtccctagt gcatggttta 960

gtattcttgt tgtctaggga tctgtgaggg ctttgatttg ggggcagtga ctttcttttt 1020

acatccccat tttatttttg tgagaacttg ggagcctgaa ctcccatcca taccactgaa 1080

tagagatttt gagtaatgat acttgtttcc aaaaaaaaag aaaccataca tagatacgta 1140

tggattggag tcattaatat cctaggcaag aaacatggaa gtgaagactt ctttctctgc 1200

aagggaaacc gatgatccca ctcctgggaa atagtaggga aacttggtat gtgtattccc 1260

atgtgtcctc tagggagttg gtaatggtta acctgacttc agcttccagg aattggctac 1320

tcttcccgtt ttctatagtc atttgaatcc acgagcttga tttgcactaa tttgaccgac 1380

attgattttg tgtgtgactt ggtttatggg gccagctgac tgaagtaagc agaccttttg 1440

ggcaaaaata tgctttgaca gtggtctccc acctatttgt tccactgtct gccttcccct 1500

ggttacttaa aattcatcag cttgtccaac tggaccttct ttccttcctg ctgaagttga 1560

tttgaagtaa aaccttagat ttgatgttaa aacagttgtc aaatctgttg gtaaataaga 1620

tttgaaggac cctactctgt ctcccttgaa aaaggggagg aatgtcagtg ttactgtttt 1680

tggaaaaagt agatttttaa accgagtttg gaaatggtaa gtatgcagag gtgggtgggg 1740

gcaatctcaa aaacgtgcaa aaatgaggaa aacaaaaatg aggaaatgtg tgcgtgtgtt 1800

taatgcaaaa ctttaaaaag aaaaacaact gttatgtgac tgttaacttg ctctgcattt 1860

tatgtgccac aggtatgaaa ggtgacattg caaaatactc cgctcttctc gcagtgtaga 1920

aggggtgacc ccgggggttg ggggagatca aaaacagctc agtagttagg acagagctta 1980

gctaagtttg tcttgcttta aggggaagtt gcctttggtt ttgacttttt atggaatggg 2040

gttgggtctg cttgctgctt tcaaagcaaa aaccacaaaa atgtgttcaa ggctacccca 2100

gcctggtgtg aaatgtcttc tgggtaaatt ggggtagggt ttttaaacca actacttggt 2160

tgtcaaccac ttgcgacaag aggaaaaaaa aacatctgct ccatcggaag aacgaccaag 2220

gaaaatgggt tatttttttt ccagaggaaa tagataacgt aaccttttaa agcaaaatct 2280

ttataaactg tgtctgagaa attgcacacg tgtgtgtgac atgctcaaag gtcagacaag 2340

gggtggtcag gaagggatgt attttagtag ccacttgtat ctttttccaa aaacacctac 2400

ccatgtttgg ggaatgttaa acaaaatcaa aaaacaacct tttgtagccg ttggaagctt 2460

catgtccttt cttctaactt gtcttctcca gcggaagtga ccgtggtggc ttcaataaat 2520

ttggtggtaa gtgaacagag tttccaaaat tcccaactcc cagcaatgct ttgtctgatt 2580

gttcatttgc agatgtctta gcgtgttaat ttaaatgtca aaggttttga ggtgtccaga 2640

accacctcca gaaaggggta gggtagaatg ccacctgttg cctggtgtgt gctaacctgg 2700

agcaggtagg ggtaagactc aatagtcatc ttttaccaaa tgggtttgcc tggaagaggg 2760

atgtaacatt gccctgagga tggtgaagtt ggtatacatt tataaagtac ggaatggtgt 2820

caatgaatgc aattctatgt atatggactt aactgagatg ggcaaataga aactagctct 2880

gggaaggaac atgtgcacta cttcaagaaa gattggaagc atgtgtggct catgggaaat 2940

aaccaggtct taaacagcac aaactgaatt cgtggaccag gaaggtctta aacagcacaa 3000

actgaattca tggaaaaatg acaaatttga gaagtctccc agtaagctgg aacttttctg 3060

gtttggttaa caaaaggttt cttgatttgt ttcaagattt aaagccaaag gtgtgggttc 3120

atgacttagg tgtcattgcg tgtgggtaca atatttatat atggcgaatt cagataaaca 3180

ttggtcaaag atggtctctg gaaaaacaaa atagaggctg cattacggaa ataagatttc 3240

tggtctgttc cctgggacat gcttaaaaaa tacaatagct attatgtatg gtttttattt 3300

tcatgtggtt tcggggaaac aacacggttt taaggatggt ttctaaagat gaaattaaaa 3360

attgttccac aagggttaag tgtctggtgg taaagttggg agaaactgga tggatgcaca 3420

tcgcatggct ggtggcgagc ccatctctct tctctcgggt gagagaaccg ggccaagctg 3480

agttggtttg ttcactttaa tgggtctccg tttcccctgc cacctgtgct gaggacattt 3540

cccagcctga gctgggggag gcagcatttg ctgaagtgtg gagttgtctc tgtggagact 3600

caagttacag atcttaaggg gcctgcctag aattttctcc tctgggcagg cgacccagga 3660

aagggtttgg agtgaggctg tgagcactta cttgatattt tacaagtttg gatttggtgt 3720

taattttttt ccttgtccgt tttttcctgt tgactaacgg ctcatctttt ccttgttttt 3780

gttttttttt tgttcttttt ttccatgtca ctaaaggccc tcgggaccaa ggatcacgtc 3840

atgactccga acaggataat tcagacaaca acaccatctt tgtgcaaggc ctgggtgaga 3900

atgttacaat tgagtctgtg gctgattact tcaagcagat tggtattatt aagacaaaca 3960

agaaaacggg acagcccatg attaatttgt acacagacag ggaaactggc aagctgaagg 4020

gagaggcaac ggtctctttt gatgacccac cttcagctaa agcagctatt gactggtttg 4080

atggtaaaga attctccgga aatcctatca aggtctcatt tgctactcgc cgggcagact 4140

ttaatcgggg tggtggcaat ggtcgtggag gccgagggcg aggaggaccc atgggccgtg 4200

gaggctatgg aggtggtggc agtggtggtg gtggccgagg aggatttccc agtggaggtg 4260

gtggcggtgg aggacagcag cgagctggtg actggaagtg tcctaatccc acctgtgaga 4320

atatgaactt ctcttggagg aatgaatgca accagtgtaa ggcccctaaa ccagatggcc 4380

caggaggggg accaggtggc tctcacatgg ggggtaacta cggggatgat cgtcgtggtg 4440

gcagaggagg ctatgatcga ggcggctacc ggggccgcgg cggggaccgt ggaggcttcc 4500
gagggggccg gggtggtggg gacagaggtg gctttggccc tggcaagatg gattccaggg 4560
gtgagcacag acaggatcgc agggagaggc cgtattaatt agcctggctc cccaggttct 4620
ggaacagctt tttgtcctgt acccagtgtt accctcgtta ttttgtaacc ttccaattcc 4680
tgatcaccca agggtttttt tgtgtcggac tatgtaattg taactatacc tctggttccc 4740
attaaaagtg accattttag ttaaattttg ttcctcttcc cccttttcac tttcctggaa 4800
gatcgatgtc ccgatcagga aggtagagag ttttcctgtt cagattaccc tgcccagcag 4860
gaactggaat acagtgttcg gggagaaggc caaatgatat ccttgagagc agagattaaa 4920
cttttctgtc atggggaaag ttggtgtata aatgagaaat gaagaacatg ggatgtcatg 4980
agtgttggcc taaatttgcc cagctatggg gaatttttcc tttaccacat ttatttgcat 5040
actggtctta gtttatttgc agcagtttat ccctttttaa gaactctttg atcttttggc 5100
ccttttaatg gtgaggctca aacaaactac atttaaatgg ggcagtattc agatttgacc 5160
atggtggaga gcgcttagcc actctgggtc tttcacagga aggagagtaa ctgagtgctg 5220
caggagtttg tggagtggag tcaggatcta ggaggtgagt gactcccttc ctagctgccc 5280
tggtgaacag cgcttgggta gatacctgct ataaggagac tggtctggct gggttacttt 5340
cacatcctgc ctgtactcag agggcttgag gtcattgaca ttatgagatt ttaggcttga 5400
tccctttttg attggagggt ggaaggccct cctaagggaa tgataagtga taagaggggg 5460
aaggggttgc agccaatgag ttaaaacctt agagcagtgc tcctcagcct cttaccatgt 5520
ggttgtaaac ttgcacgtac ctgccaacca gttatttagc atgcttttta ttttagttac 5580
acagagcgta acattaaccc aagagcagaa aggttttatt tacagggttt tcgaacttgg 5640
tttgtaagac agctgccatc acaagcatag cttacaaatg tgctggggac ccctaattgg 5700
gaagtgcttt cctctcaaat ttttattttt tatttttaga gacagagtct tgctctgtca 5760
tccaggctgg agtgcagtgg cgtgatctcg gctcactgta gcctctgcct cctgagttta 5820
agcgattctc ctgcctcagg agaatcccag cttctgagta gctgagacta caggcgtggg 5880
ccaccatgcc cacctagttt ttgcattttt agtggaggtg tggtttcact gtgttggcca 5940
ggctggtctc ttaactcctg acctcaggtg atccacctgc cttggcctcc caaagtgctg 6000
gaattacagg catgagccgc tgcatctggc catcctctca aattttcaag tgttccacaa 6060
gtatgttctc tactgaagag ttgctgcatc cttgaatctt gggtgatttg aggcacagaa 6120
actatgactt tattttttga gatggagttt tgctcttgtt gcccaggctg gagtgcaatg 6180
gcacgttttt cggcttaccg caactgccgc ctcctgggtt caagcgatag ctgggattac 6240
aggcatgcgc caccatgccc agctaattta tttgtatttt tagtagagac ggggtttctc 6300
catgttggtc aggctggtct cgaactcccg acctcaggtg atgtgcacac ctcagcctcc 6360

caataaacca tgacttttaa gaggaatagc aggtttactt cccctgccag cattggggtg 6420

ctctctaagc aacagtaggc ggagagtggt ctggcgtatt aaaaacaaag gatcgtcaag 6480

tgggccttcc caggcattgc tttgacttag tacatgtaga ggatgtggca gttctctccg 6540

tccctgccac tgctggtttc tttgttaaat gtttagttga aatggcctga tacgatattt 6600

gagtagttca ctgttggtgc tttgcctagc aggattctaa tcttgctttg gttgtggtcc 6660

cctgatgccc tcctgttagg agtggaggag gtcgaagctc cttgtaagat atgattactg 6720

ggaccattag tgtcaagttc ctgtgtcctt caaatggcat atgtgattgg ccttgacctt 6780

aaaaggaaat agggtcccag gtgactgttt agtgggtagg tccagtttgg ggggatcttc 6840

caggagaatg gatagagaca cctagcagca gagagaacat tggtgcctct caagccaacc 6900

tcccacctca gcctcccaag tagctgggac tacaggtgct tcctcgctac cacacctggg 6960

taattttttt tttaattact tttttttttt taagaaacag ggtttcactg ggttgcccag 7020

gctggtcttg aactggcctc aagtgacctg cctgcctcag cctcccaaag tgctgggatc 7080

acaggtgtga cccactgcgt ttggccagaa tactctattc ttactgaatg attgaaatct 7140

gtcttgaagc attaggtgtc ccatttttgt gagttggaat tgggacaggc taagtaggaa 7200

gtgaggaggg tggggagagc tgtgctgtag gtctgtttgt cccttccttg atgtagcctt 7260

cagttagccc tttcagcttt tttccccatc ttgtgccggg ccttcctggg tttcagtact 7320

tggatgtagg gctgcagtta tgtcagtggt gggtagattg accaggaatt aaggtctagg 7380

gtccagccca tgtgagactt gactcactga tctaccttta ggcatgtctt ccttccagtc 7440

tcatcctttt taaatttttt tttttttttt gagacggtct cactctcacc caggctggat 7500

tgcagtggtg tgatctcgac caactgcaac ctctgcctcc cacccgcaag ctatctgccc 7560

acctcagcct ctggagtagc tgggacggga ctacaggcac ctgccaccat gactggctaa 7620

tttttttttg tattttttgt ggagatgggg tcttgccatg ttgctcaggc tggtctggtc 7680

tcaaaactgc tctgggctca agtgattgtc ccactttggc ctcccagagt gctgggatta 7740

aggtgtgaga tactgtgtcc ggctatgaaa attttatttt taattaactt gtatatattt 7800

atggggtaca atgtcctatt tctgtacatg tacacattgt ggaatcaaat caggctaata 7860

tatccatcac ttcatatcat tagcatgaat gagaacatac aaagccactc ttagaaaatt 7920

ttgaaattta tgttatttca gcccttttat gctggaggtt gcaaatgttt tgtgaataat 7980

cagaccaaaa ataaaaacaa aaaatgattg acttcagtca ttcagtaaga a 8031

<210> 14
<211> 8031
<212> DNA
<213> Artificial Sequence

<220>
<223>  sequence delta 4

<400>  14
acttaagctt cgacgcagga ggcggggctg ctcagtcctc caggcgtcgg tactcagcgg      60

tgttggaact tcgttgcttg cttgcctgtg cgcgcgtgcg cggacatggc ctcaaacgat     120

tatacccaac aagcaaccca aagctatggg gcctacccca cccagcccgg gcagggctat     180

tcccagcaga gcagtcagcc ctacggacag cagagttaca gtggttatag ccagtccacg     240

gacacttcag gctatggcca gagcagctat tcttcttatg gccagagcca gaacacaggc     300

tatggaactc agtcaactcc ccagggatat ggctcgactg gcggctatgg cagtagccag     360

agctcccaat cgtcttacgg gcagcagtcc tcctaccctg gctatggcca gcagccagct     420

cccagcagca cctcgggaag ttacggtagc agttctcaga gcagcagcta tgggcagccc     480

cagagtggga gctacagcca gcagcctagc tatggtggac agcagcaaag ctatggacag     540

cagcaaagct ataatccccc tcagggctat ggacagcaga accagtacaa cagcagcagt     600

ggtggtggag gtggaggtgg aggtggaggt aactatggcc aagatcaatc ctccatgagt     660

agtggtggtg gcagtggtgg cggttatggc aatcaagacc agagtggtgg aggtggcagc     720

ggtggctatg gacagcagga ccgtggaggc cgcggcaggg gtggcagtgg tggcggcggc     780

ggcggcggcg gtggtggtta caaccgcagc agtggtggct atgaacccag aggtcgtgga     840

ggtggccgtg gaggcagagg tggcatgggg taggtgtctc atgagccagg gagtatcttt     900

ggtggggagt gtggaggatt gcatgaatct ccctgaagcc agtccctagt gcatggttta     960

gtattcttgt tgtctaggga tctgtgaggg ctttgatttg ggggcagtga ctttcttttt    1020

acatccccat tttatttttg tgagaacttg ggagcctgaa ctcccatcca taccactgaa    1080

tagagatttt gagtaatgat acttgtttcc aaaaaaaaag aaaccataca tagatacgta    1140

tggattggag tcattaatat cctaggcaag aaacatggaa gtgaagactt ctttctctgc    1200

aagggaaacc gatgatccca ctcctgggaa atagtaggga aacttggtat gtgtattccc    1260

atgtgtcctc tagggagttg gtaatggtta acctgacttc agcttccagg aattggctac    1320

tcttcccgtt ttctatagtc atttgaatcc acgagcttga tttgcactaa tttgaccgac    1380

attgattttg tgtgtgactt ggtttatggg gccagctgac tgaagtaagc agaccttttg    1440

ggcaaaaata tgctttgaca gtggtctccc acctatttgt tccactgtct gccttcccct    1500

ggttacttaa aattcatcag cttgtccaac tggaccttct ttccttcctg ctgaagttga    1560

tttgaagtaa aaccttagat ttgatgttaa aacagttgtc aaatctgttg gtaaataaga    1620

tttgaaggac cctactctgt ctcccttgaa aaaggggagg aatgtcagtg ttactgtttt    1680

tggaaaaagt agatttttaa accgagtttg gaaatggtaa gtatgcagag gtgggtgggg    1740

gcaatctcaa aaacgtgcaa aaatgaggaa aacaaaaatg aggaaatgtg tgcgtgtgtt    1800

taatgcaaaa ctttaaaaag aaaaacaact gttatgtgac tgttaacttg ctctgcattt 1860

tatgtgccac aggtatgaaa ggtgacattg caaaatactc cgctcttctc gcagtgtaga 1920

aggggtgacc ccgggggttg ggggagatca aaaacagctc agtagttagg acagagctta 1980

gctaagtttg tcttgcttta aggggaagtt gcctttggtt ttgacttttt atggaatggg 2040

gttgggtctg cttgctgctt tcaaagcaaa aaccacaaaa atgtgttcaa ggctacccca 2100

gcctggtgtg aaatgtcttc tgggtaaatt ggggtagggt ttttaaacca actacttggt 2160

tgtcaaccac ttgcgacaag aggaaaaaaa aacatctgct ccatcggaag aacgaccaag 2220

gaaaatgggt tatttttttt ccagaggaaa tagataacgt aaccttttaa agcaaaatct 2280

ttataaactg tgtctgagaa attgcacacg tgtgtgtgac atgctcaaag gtcagacaag 2340

gggtggtcag gaagggatgt attttagtag ccacttgtat ctttttccaa aaacacctac 2400

ccatgtttgg ggaatgttaa acaaaatcaa aaaacaacct tttgtagccg ttggaagctt 2460

catgtccttt cttctaactt gtcttctcca gcggaagtga ccgtggtggc ttcaataaat 2520

ttggtggtaa gtgaacagag tttccaaaat tcccaactcc cagcaatgct ttgtctgatt 2580

gttcatttgc agatgtctta gcgtgttaat ttaaatgtca aaggttttga ggtgtccaga 2640

accacctcca gaaaggggta gggtagaatg ccacctgttg cctggtgtgt gctaacctgg 2700

agcaggtagg ggtaagactc aatagtcatc ttttaccaaa tgggtttgcc ccaggttaat 2760

aagaggggtc tagtaggcct tggactgggc cgttgccaca cctggcactt agtgaccatc 2820

atcatgagaa actggagagt gcgtgctgga acacgtggtg ccatcttggc tttaggatcc 2880

ttttgatcgt tgtgtccaag gcttgtgtgt gtgtgagtgt gtgggagaca actccgaatg 2940

tttaattctg gaagagggat gtaacattgc cctgaggatg gtgaagttgg tatacattta 3000

taaagtacgg aatggtgtca atgaatgcaa ttctatgtat atggacttgg aacttttctg 3060

gtttggttaa caaaaggttt cttgatttgt ttcaagattt aaagccaaag gtgtgggttc 3120

atgacttagg tgtcattgcg tgtgggtaca atatttatat atggcgaatt cagataaaca 3180

ttggtcaaag atggtctctg gaaaaacaaa atagaggctg cattacggaa ataagatttc 3240

tggtctgttc cctgggacat gcttaaaaaa tacaatagct attatgtatg gtttttattt 3300

tcatgtggtt tcggggaaac aacacggttt taaggatggt ttctaaagat gaaattaaaa 3360

attgttccac aagggttaag tgtctggtgg taaagttggg agaaactgga tggatgcaca 3420

tcgcatggct ggtggcgagc ccatctctct tctctcgggt gagagaaccg ggccaagctg 3480

agttggtttg ttcactttaa tgggtctccg tttcccctgc cacctgtgct gaggacattt 3540

cccagcctga gctgggggag gcagcatttg ctgaagtgtg gagttgtctc tgtggagact 3600

caagttacag atcttaaggg gcctgcctag aattttctcc tctgggcagg cgacccagga 3660

aagggtttgg agtgaggctg tgagcactta cttgatattt tacaagtttg gatttggtgt 3720

taattttttt ccttgtccgt tttttcctgt tgactaacgg ctcatctttt ccttgttttt 3780

gttttttttt tgttcttttt ttccatgtca ctaaaggccc tcgggaccaa ggatcacgtc 3840

atgactccga acaggataat tcagacaaca acaccatctt tgtgcaaggc ctgggtgaga 3900

atgttacaat tgagtctgtg gctgattact tcaagcagat tggtattatt aagacaaaca 3960

agaaaacggg acagcccatg attaatttgt acacagacag ggaaactggc aagctgaagg 4020

gagaggcaac ggtctctttt gatgacccac cttcagctaa agcagctatt gactggtttg 4080

atggtaaaga attctccgga aatcctatca aggtctcatt tgctactcgc cgggcagact 4140

ttaatcgggg tggtggcaat ggtcgtggag gccgagggcg aggaggaccc atgggccgtg 4200

gaggctatgg aggtggtggc agtggtggtg gtggccgagg aggatttccc agtggaggtg 4260

gtggcggtgg aggacagcag cgagctggtg actggaagtg tcctaatccc acctgtgaga 4320

atatgaactt ctcttggagg aatgaatgca accagtgtaa ggcccctaaa ccagatggcc 4380

caggaggggg accaggtggc tctcacatgg ggggtaacta cggggatgat cgtcgtggtg 4440

gcagaggagg ctatgatcga ggcggctacc ggggccgcgg cggggaccgt ggaggcttcc 4500

gagggggccg gggtggtggg gacagaggtg gctttggccc tggcaagatg gattccaggg 4560

gtgagcacag acaggatcgc agggagaggc cgtattaatt agcctggctc cccaggttct 4620

ggaacagctt tttgtcctgt acccagtgtt accctcgtta ttttgtaacc ttccaattcc 4680

tgatcaccca agggtttttt tgtgtcggac tatgtaattg taactatacc tctggttccc 4740

attaaaagtg accattttag ttaaattttg ttcctcttcc cccttttcac tttcctggaa 4800

gatcgatgtc ccgatcagga aggtagagag ttttcctgtt cagattaccc tgcccagcag 4860

gaactggaat acagtgttcg gggagaaggc caaatgatat ccttgagagc agagattaaa 4920

cttttctgtc atggggaaag ttggtgtata aatgagaaat gaagaacatg ggatgtcatg 4980

agtgttggcc taaatttgcc cagctatggg gaatttttcc tttaccacat ttatttgcat 5040

actggtctta gtttatttgc agcagtttat ccctttttaa gaactctttg atcttttggc 5100

ccttttaatg gtgaggctca aacaaactac atttaaatgg ggcagtattc agatttgacc 5160

atggtggaga gcgcttagcc actctgggtc tttcacagga aggagagtaa ctgagtgctg 5220

caggagtttg tggagtggag tcaggatcta ggaggtgagt gactcccttc ctagctgccc 5280

tggtgaacag cgcttgggta gatacctgct ataaggagac tggtctggct gggttacttt 5340

cacatcctgc ctgtactcag agggcttgag gtcattgaca ttatgagatt ttaggcttga 5400

tccctttttg attggagggt ggaaggccct cctaagggaa tgataagtga taagaggggg 5460

aaggggttgc agccaatgag ttaaaacctt agagcagtgc tcctcagcct cttaccatgt 5520

ggttgtaaac ttgcacgtac ctgccaacca gttatttagc atgcttttta ttttagttac 5580

acagagcgta acattaaccc aagagcagaa aggttttatt tacagggttt tcgaacttgg 5640

tttgtaagac agctgccatc acaagcatag cttacaaatg tgctggggac ccctaattgg 5700

gaagtgcttt cctctcaaat ttttattttt tatttttaga gacagagtct tgctctgtca 5760

tccaggctgg agtgcagtgg cgtgatctcg gctcactgta gcctctgcct cctgagttta 5820

agcgattctc ctgcctcagg agaatcccag cttctgagta gctgagacta caggcgtggg 5880

ccaccatgcc cacctagttt ttgcattttt agtggaggtg tggtttcact gtgttggcca 5940

ggctggtctc ttaactcctg acctcaggtg atccacctgc cttggcctcc caaagtgctg 6000

gaattacagg catgagccgc tgcatctggc catcctctca aattttcaag tgttccacaa 6060

gtatgttctc tactgaagag ttgctgcatc cttgaatctt gggtgatttg aggcacagaa 6120

actatgactt tattttttga gatggagttt tgctcttgtt gcccaggctg gagtgcaatg 6180

gcacgttttt cggcttaccg caactgccgc ctcctgggtt caagcgatag ctgggattac 6240

aggcatgcgc caccatgccc agctaattta tttgtatttt tagtagagac ggggtttctc 6300

catgttggtc aggctggtct cgaactcccg acctcaggtg atgtgcacac ctcagcctcc 6360

caataaacca tgacttttaa gaggaatagc aggtttactt cccctgccag cattggggtg 6420

ctctctaagc aacagtaggc ggagagtggt ctggcgtatt aaaaacaaag gatcgtcaag 6480

tgggccttcc caggcattgc tttgacttag tacatgtaga ggatgtggca gttctctccg 6540

tccctgccac tgctggtttc tttgttaaat gtttagttga aatggcctga tacgatattt 6600

gagtagttca ctgttggtgc tttgcctagc aggattctaa tcttgctttg gttgtggtcc 6660

cctgatgccc tcctgttagg agtggaggag gtcgaagctc cttgtaagat atgattactg 6720

ggaccattag tgtcaagttc ctgtgtcctt caaatggcat atgtgattgg ccttgacctt 6780

aaaaggaaat agggtcccag gtgactgttt agtgggtagg tccagtttgg ggggatcttc 6840

caggagaatg gatagagaca cctagcagca gagagaacat tggtgcctct caagccaacc 6900

tcccacctca gcctcccaag tagctgggac tacaggtgct tcctcgctac cacacctggg 6960

taattttttt tttaattact tttttttttt taagaaacag ggtttcactg ggttgcccag 7020

gctggtcttg aactggcctc aagtgacctg cctgcctcag cctcccaaag tgctgggatc 7080

acaggtgtga cccactgcgt ttggccagaa tactctattc ttactgaatg attgaaatct 7140

gtcttgaagc attaggtgtc ccatttttgt gagttggaat tgggacaggc taagtaggaa 7200

gtgaggaggg tggggagagc tgtgctgtag gtctgtttgt cccttccttg atgtagcctt 7260

cagttagccc tttcagcttt tttccccatc ttgtgccggg ccttcctggg tttcagtact 7320

tggatgtagg gctgcagtta tgtcagtggt gggtagattg accaggaatt aaggtctagg 7380

gtccagccca tgtgagactt gactcactga tctaccttta ggcatgtctt ccttccagtc 7440

tcatcctttt taaatttttt tttttttttt gagacggtct cactctcacc caggctggat 7500

tgcagtggtg tgatctcgac caactgcaac ctctgcctcc cacccgcaag ctatctgccc 7560

acctcagcct ctggagtagc tgggacggga ctacaggcac ctgccaccat gactggctaa 7620

tttttttttg tattttttgt ggagatgggg tcttgccatg ttgctcaggc tggtctggtc 7680

tcaaaactgc tctgggctca agtgattgtc ccactttggc ctcccagagt gctgggatta 7740

aggtgtgaga tactgtgtcc ggctatgaaa attttatttt taattaactt gtatatattt 7800

atggggtaca atgtcctatt tctgtacatg tacacattgt ggaatcaaat caggctaata 7860

tatccatcac ttcatatcat tagcatgaat gagaacatac aaagccactc ttagaaaatt 7920

ttgaaattta tgttatttca gcccttttat gctggaggtt gcaaatgttt tgtgaataat 7980

cagaccaaaa ataaaaacaa aaaatgattg acttcagtca ttcagtaaga a 8031

<210> 15
<211> 8031
<212> DNA
<213> Artificial Sequence

<220>
<223> séquence delta 5

<400> 15
acttaagctt cgacgcagga ggcggggctg ctcagtcctc caggcgtcgg tactcagcgg 60

tgttggaact tcgttgcttg cttgcctgtg cgcgcgtgcg cggacatggc ctcaaacgat 120

tatacccaac aagcaaccca aagctatggg gcctacccca cccagcccgg gcagggctat 180

tcccagcaga gcagtcagcc ctacggacag cagagttaca gtggttatag ccagtccacg 240

gacacttcag gctatggcca gagcagctat tcttcttatg gccagagcca gaacacaggc 300

tatggaactc agtcaactcc ccagggatat ggctcgactg gcggctatgg cagtagccag 360

agctcccaat cgtcttacgg gcagcagtcc tcctaccctg gctatggcca gcagccagct 420

cccagcagca cctcgggaag ttacggtagc agttctcaga gcagcagcta tgggcagccc 480

cagagtggga gctacagcca gcagcctagc tatggtggac agcagcaaag ctatggacag 540

cagcaaagct ataatccccc tcagggctat ggacagcaga accagtacaa cagcagcagt 600

ggtggtggag gtggaggtgg aggtggaggt aactatggcc aagatcaatc ctccatgagt 660

agtggtggtg gcagtggtgg cggttatggc aatcaagacc agagtggtgg aggtggcagc 720

ggtggctatg gacagcagga ccgtggaggc cgcggcaggg gtggcagtgg tggcggcggc 780

ggcggcggcg gtggtggtta caaccgcagc agtggtggct atgaacccag aggtcgtgga 840

ggtggccgtg gaggcagagg tggcatgggg taggtgtctc atgagccagg gagtatcttt 900

ggtggggagt gtggaggatt gcatgaatct ccctgaagcc agtccctagt gcatggttta 960

gtattcttgt tgtctaggga tctgtgaggg ctttgatttg ggggcagtga ctttcttttt 1020

acatccccat tttatttttg tgagaacttg ggagcctgaa ctcccatcca taccactgaa 1080

tagagatttt gagtaatgat acttgtttcc aaaaaaaaag aaaccataca tagatacgta 1140

tggattggag tcattaatat cctaggcaag aaacatggaa gtgaagactt ctttctctgc 1200

aagggaaacc gatgatccca ctcctgggaa atagtaggga aacttggtat gtgtattccc 1260

atgtgtcctc tagggagttg gtaatggtta acctgacttc agcttccagg aattggctac 1320

tcttcccgtt ttctatagtc atttgaatcc acgagcttga tttgcactaa tttgaccgac 1380

attgattttg tgtgtgactt ggtttatggg gccagctgac tgaagtaagc agaccttttg 1440

ggcaaaaata tgctttgaca gtggtctccc acctatttgt tccactgtct gccttcccct 1500

ggttacttaa aattcatcag cttgtccaac tggaccttct ttccttcctg ctgaagttga 1560

tttgaagtaa aaccttagat ttgatgttaa aacagttgtc aaatctgttg gtaaataaga 1620

tttgaaggac cctactctgt ctcccttgaa aaaggggagg aatgtcagtg ttactgtttt 1680

tggaaaaagt agatttttaa accgagtttg gaaatggtaa gtatgcagag gtgggtgggg 1740

gcaatctcaa aaacgtgcaa aaatgaggaa aacaaaaatg aggaaatgtg tgcgtgtgtt 1800

taatgcaaaa ctttaaaaag aaaaacaact gttatgtgac tgttaacttg ctctgcattt 1860

tatgtgccac aggtatgaaa ggtgacattg caaaatactc cgctcttctc gcagtgtaga 1920

aggggtgacc ccgggggttg ggggagatca aaaacagctc agtagttagg acagagctta 1980

gctaagtttg tcttgcttta aggggaagtt gcctttggtt ttgacttttt atggaatggg 2040

gttgggtctg cttgctgctt tcaaagcaaa aaccacaaaa atgtgttcaa ggctacccca 2100

gcctggtgtg aaatgtcttc tgggtaaatt ggggtagggt ttttaaacca actacttggt 2160

tgtcaaccac ttgcgacaag aggaaaaaaa aacatctgct ccatcggaag aacgaccaag 2220

gaaaatgggt tatttttttt ccagaggaaa tagataacgt aaccttttaa agcaaaatct 2280

ttataaactg tgtctgagaa attgcacacg tgtgtgtgac atgctcaaag gtcagacaag 2340

gggtggtcag gaagggatgt attttagtag ccacttgtat ctttttccaa aaacacctac 2400

ccatgtttgg ggaatgttaa acaaaatcaa aaaacaacct tttgtagccg ttggaagctt 2460

catgtccttt cttctaactt gtcttctcca gcggaagtga ccgtggtggc ttcaataaat 2520

ttggtggtaa gtgaacagag tttccaaaat tcccaactcc cagcaatgct ttgtctgatt 2580

gttcatttgc agatgtctta gcgtgttaat ttaaatgtca aaggttttga ggtgtccaga 2640

accacctcca gaaaggggta gggtagaatg ccacctgttg cctggtgtgt gctaacctgg 2700

agcaggtagg ggtaagactc aatagtcatc ttttaccaaa tgggtttgcc ccaggttaat 2760

aagaggggtc tagtaggcct tggactgggc cgttgccaca cctggcactt agtgaccatc 2820

atcatgagaa actggagagt gcgtgctgga acacgtggtg ccatcttggc tttaggatcc 2880

ttttgatcgt tgtgtccaag gcttgtgtgt gtgtgagtgt gtgggagaca actccgaatg 2940

tttaattctg gaagagggat gtaacattgc cctgaggatg gtgaagttgg tatacattta 3000

taaagtacgg aatggtgtca atgaatgcaa ttctatgtat atggacttaa ctgagatggg 3060

caaatagaaa ctagctctgg gaaggaacat gtgcactact tcaagaaaga ttggaagcat 3120

gtgtggctca tgggaaataa ccaggtctta aacagcacaa actgaattcg tggaccagga 3180

aggtcttaaa cagcacaaac tgaattcatg gaaaaatgac aaatttgaga agtctcccag 3240

taagctggaa cttttctggt ttggttaaca aaaggtttct tgatttgttt caagatttaa 3300

agccaaaggt gtgggttcat gacttaggtg tcattgcgtg tgggaaagat gaaattaaaa 3360

attgttccac aagggttaag tgtctggtgg taaagttggg agaaactgga tggatgcaca 3420

tcgcatggct ggtggcgagc ccatctctct tctctcgggt gagagaaccg ggccaagctg 3480

agttggtttg ttcactttaa tgggtctccg tttcccctgc cacctgtgct gaggacattt 3540

cccagcctga gctgggggag gcagcatttg ctgaagtgtg gagttgtctc tgtggagact 3600

caagttacag atcttaaggg gcctgcctag aattttctcc tctgggcagg cgacccagga 3660

aagggtttgg agtgaggctg tgagcactta cttgatattt tacaagtttg gatttggtgt 3720

taattttttt ccttgtccgt tttttcctgt tgactaacgg ctcatctttt ccttgttttt 3780

gttttttttt tgttcttttt ttccatgtca ctaaaggccc tcgggaccaa ggatcacgtc 3840

atgactccga acaggataat tcagacaaca acaccatctt tgtgcaaggc ctgggtgaga 3900

atgttacaat tgagtctgtg gctgattact tcaagcagat tggtattatt aagacaaaca 3960

agaaaacggg acagcccatg attaatttgt acacagacag ggaaactggc aagctgaagg 4020

gagaggcaac ggtctctttt gatgacccac cttcagctaa agcagctatt gactggtttg 4080

atggtaaaga attctccgga aatcctatca aggtctcatt tgctactcgc cgggcagact 4140

ttaatcgggg tggtggcaat ggtcgtggag gccgagggcg aggaggaccc atgggccgtg 4200

gaggctatgg aggtggtggc agtggtggtg gtggccgagg aggatttccc agtggaggtg 4260

gtggcggtgg aggacagcag cgagctggtg actggaagtg tcctaatccc acctgtgaga 4320

atatgaactt ctcttggagg aatgaatgca accagtgtaa ggcccctaaa ccagatggcc 4380

caggaggggg accaggtggc tctcacatgg ggggtaacta cggggatgat cgtcgtggtg 4440

gcagaggagg ctatgatcga ggcggctacc ggggccgcgg cggggaccgt ggaggcttcc 4500

gagggggccg gggtggtggg gacagaggtg gctttggccc tggcaagatg gattccaggg 4560

gtgagcacag acaggatcgc agggagaggc cgtattaatt agcctggctc cccaggttct 4620

ggaacagctt tttgtcctgt acccagtgtt accctcgtta ttttgtaacc ttccaattcc 4680

tgatcaccca agggtttttt tgtgtcggac tatgtaattg taactatacc tctggttccc 4740

attaaaagtg accattttag ttaaattttg ttcctcttcc cccttttcac tttcctggaa 4800

gatcgatgtc ccgatcagga aggtagagag ttttcctgtt cagattaccc tgcccagcag 4860

gaactggaat acagtgttcg gggagaaggc caaatgatat ccttgagagc agagattaaa 4920

cttttctgtc atggggaaag ttggtgtata aatgagaaat gaagaacatg ggatgtcatg 4980

agtgttggcc taaatttgcc cagctatggg gaatttttcc tttaccacat ttatttgcat 5040

actggtctta gtttatttgc agcagtttat ccctttttaa gaactctttg atcttttggc 5100

ccttttaatg gtgaggctca aacaaactac atttaaatgg ggcagtattc agatttgacc 5160

atggtggaga gcgcttagcc actctgggtc tttcacagga aggagagtaa ctgagtgctg 5220

caggagtttg tggagtggag tcaggatcta ggaggtgagt gactcccttc ctagctgccc 5280

tggtgaacag cgcttgggta gatacctgct ataaggagac tggtctggct gggttacttt 5340

cacatcctgc ctgtactcag agggcttgag gtcattgaca ttatgagatt ttaggcttga 5400

tccctttttg attggagggt ggaaggccct cctaagggaa tgataagtga taagaggggg 5460

aaggggttgc agccaatgag ttaaaacctt agagcagtgc tcctcagcct cttaccatgt 5520

ggttgtaaac ttgcacgtac ctgccaacca gttatttagc atgcttttta ttttagttac 5580

acagagcgta acattaaccc aagagcagaa aggttttatt tacagggttt tcgaacttgg 5640

tttgtaagac agctgccatc acaagcatag cttacaaatg tgctggggac ccctaattgg 5700

gaagtgcttt cctctcaaat tttatttttt tatttttaga gacagagtct tgctctgtca 5760

tccaggctgg agtgcagtgg cgtgatctcg gctcactgta gcctctgcct cctgagttta 5820

agcgattctc ctgcctcagg agaatcccag cttctgagta gctgagacta caggcgtggg 5880

ccaccatgcc cacctagttt ttgcattttt agtggaggtg tggtttcact gtgttggcca 5940

ggctggtctc ttaactcctg acctcaggtg atccacctgc cttggcctcc caaagtgctg 6000

gaattacagg catgagccgc tgcatctggc catcctctca aattttcaag tgttccacaa 6060

gtatgttctc tactgaagag ttgctgcatc cttgaatctt gggtgatttg aggcacagaa 6120

actatgactt tattttttga gatggagttt tgctcttgtt gcccaggctg gagtgcaatg 6180

gcacgttttt cggcttaccg caactgccgc ctcctgggtt caagcgatag ctgggattac 6240

aggcatgcgc caccatgccc agctaattta tttgtatttt tagtagagac ggggtttctc 6300

catgttggtc aggctggtct cgaactcccg acctcaggtg atgtgcacac ctcagcctcc 6360

caataaacca tgacttttaa gaggaatagc aggtttactt cccctgccag cattggggtg 6420

ctctctaagc aacagtaggc ggagagtggt ctggcgtatt aaaaacaaag gatcgtcaag 6480

tgggccttcc caggcattgc tttgacttag tacatgtaga ggatgtggca gttctctccg 6540

tccctgccac tgctggtttc tttgttaaat gtttagttga aatggcctga tacgatattt 6600

gagtagttca ctgttggtgc tttgcctagc aggattctaa tcttgctttg gttgtggtcc 6660

cctgatgccc tcctgttagg agtggaggag gtcgaagctc cttgtaagat atgattactg 6720

ggaccattag tgtcaagttc ctgtgtcctt caaatggcat atgtgattgg ccttgacctt 6780

aaaaggaaat agggtcccag gtgactgttt agtgggtagg tccagtttgg ggggatcttc 6840

caggagaatg gatagagaca cctagcagca gagagaacat tggtgcctct caagccaacc 6900

tcccacctca gcctcccaag tagctgggac tacaggtgct tcctcgctac cacacctggg 6960

taattttttt tttaattact tttttttttt taagaaacag ggtttcactg ggttgcccag 7020

gctggtcttg aactggcctc aagtgacctg cctgcctcag cctcccaaag tgctgggatc 7080

acaggtgtga cccactgcgt ttggccagaa tactctattc ttactgaatg attgaaatct 7140

gtcttgaagc attaggtgtc ccatttttgt gagttggaat tgggacaggc taagtaggaa 7200

gtgaggaggg tggggagagc tgtgctgtag gtctgtttgt cccttccttg atgtagcctt 7260

cagttagccc tttcagcttt tttccccatc ttgtgccggg ccttcctggg tttcagtact 7320

tggatgtagg gctgcagtta tgtcagtggt gggtagattg accaggaatt aaggtctagg 7380

gtccagccca tgtgagactt gactcactga tctaccttta ggcatgtctt ccttccagtc 7440

tcatcctttt taaatttttt tttttttttt gagacggtct cactctcacc caggctggat 7500

tgcagtggtg tgatctcgac caactgcaac ctctgcctcc cacccgcaag ctatctgccc 7560

acctcagcct ctggagtagc tgggacggga ctacaggcac ctgccaccat gactggctaa 7620

tttttttttg tattttttgt ggagatgggg tcttgccatg ttgctcaggc tggtctggtc 7680

tcaaaactgc tctgggctca agtgattgtc ccactttggc ctcccagagt gctgggatta 7740

aggtgtgaga tactgtgtcc ggctatgaaa attttatttt taattaactt gtatatattt 7800

atggggtaca atgtcctatt tctgtacatg tacacattgt ggaatcaaat caggctaata 7860

tatccatcac ttcatatcat tagcatgaat gagaacatac aaagccactc ttagaaaatt 7920

ttgaaattta tgttatttca gcccttttat gctggaggtt gcaaatgttt tgtgaataat 7980

cagaccaaaa ataaaaacaa aaaatgattg acttcagtca ttcagtaaga a 8031

<210> 16
<211> 8031
<212> DNA
<213> Artificial Sequence

<220>
<223> sequence delta 6

<400> 16
acttaagctt cgacgcagga ggcggggctg ctcagtcctc caggcgtcgg tactcagcgg 60

tgttggaact tcgttgcttg cttgcctgtg cgcgcgtgcg cggacatggc ctcaaacgat 120

tatacccaac aagcaaccca aagctatggg gcctacccca cccagcccgg gcagggctat 180

tcccagcaga gcagtcagcc ctacggacag cagagttaca gtggttatag ccagtccacg 240

gacacttcag gctatggcca gagcagctat tcttcttatg gccagagcca gaacacaggc 300

tatggaactc agtcaactcc ccagggatat ggctcgactg gcggctatgg cagtagccag 360

agctcccaat cgtcttacgg gcagcagtcc tcctaccctg gctatggcca gcagccagct 420

cccagcagca cctcgggaag ttacggtagc agttctcaga gcagcagcta tgggcagccc 480

cagagtggga gctacagcca gcagcctagc tatggtggac agcagcaaag ctatggacag 540

cagcaaagct ataatccccc tcagggctat ggacagcaga accagtacaa cagcagcagt 600

ggtggtggag gtggaggtgg aggtggaggt aactatggcc aagatcaatc ctccatgagt 660

agtggtggtg gcagtggtgg cggttatggc aatcaagacc agagtggtgg aggtggcagc 720

ggtggctatg gacagcagga ccgtggaggc cgcggcaggg gtggcagtgg tggcggcggc 780

ggcggcggcg gtggtggtta caaccgcagc agtggtggct atgaacccag aggtcgtgga 840

ggtggccgtg gaggcagagg tggcatgggg taggtgtctc atgagccagg gagtatcttt 900

ggtggggagt gtggaggatt gcatgaatct ccctgaagcc agtccctagt gcatggttta 960

gtattcttgt tgtctaggga tctgtgaggg ctttgatttg ggggcagtga ctttcttttt 1020

acatccccat tttatttttg tgagaacttg ggagcctgaa ctcccatcca taccactgaa 1080

tagagatttt gagtaatgat acttgtttcc aaaaaaaaag aaaccataca tagatacgta 1140

tggattggag tcattaatat cctaggcaag aaacatggaa gtgaagactt ctttctctgc 1200

aagggaaacc gatgatccca ctcctgggaa atagtaggga aacttggtat gtgtattccc 1260

atgtgtcctc tagggagttg gtaatggtta acctgacttc agcttccagg aattggctac 1320

tcttcccgtt ttctatagtc atttgaatcc acgagcttga tttgcactaa tttgaccgac 1380

attgattttg tgtgtgactt ggtttatggg gccagctgac tgaagtaagc agaccttttg 1440

ggcaaaaata tgctttgaca gtggtctccc acctatttgt tccactgtct gccttcccct 1500

ggttacttaa aattcatcag cttgtccaac tggaccttct ttccttcctg ctgaagttga 1560

tttgaagtaa aaccttagat ttgatgttaa aacagttgtc aaatctgttg gtaaataaga 1620

tttgaaggac cctactctgt ctcccttgaa aaaggggagg aatgtcagtg ttactgtttt 1680

tggaaaaagt agatttttaa accgagtttg gaaatggtaa gtatgcagag gtgggtgggg 1740

gcaatctcaa aaacgtgcaa aaatgaggaa aacaaaaatg aggaaatgtg tgcgtgtgtt 1800

taatgcaaaa ctttaaaaag aaaaacaact gttatgtgac tgttaacttg ctctgcattt 1860

tatgtgccac aggtatgaaa ggtgacattg caaaatactc cgctcttctc gcagtgtaga 1920

aggggtgacc ccgggggttg ggggagatca aaaacagctc agtagttagg acagagctta 1980

gctaagtttg tcttgcttta aggggaagtt gcctttggtt ttgacttttt atggaatggg 2040

gttgggtctg cttgctgctt tcaaagcaaa aaccacaaaa atgtgttcaa ggctacccca 2100

gcctggtgtg aaatgtcttc tgggtaaatt ggggtagggt ttttaaacca actacttggt 2160

tgtcaaccac ttgcgacaag aggaaaaaaa aacatctgct ccatcggaag aacgaccaag 2220

gaaaatgggt tatttttttt ccagaggaaa tagataacgt aaccttttaa agcaaaatct 2280

ttataaactg tgtctgagaa attgcacacg tgtgtgtgac atgctcaaag gtcagacaag 2340

gggtggtcag gaagggatgt attttagtag ccacttgtat ctttttccaa aaacacctac 2400

ccatgtttgg ggaatgttaa acaaaatcaa aaaacaacct tttgtagccg ttggaagctt 2460

catgtccttt cttctaactt gtcttctcca gcggaagtga ccgtggtggc ttcaataaat 2520

ttggtggtaa gtgaacagag tttccaaaat tcccaactcc cagcaatgct ttgtctgatt 2580

gttcatttgc agatgtctta gcgtgttaat ttaaatgtca aaggttttga ggtgtccaga 2640

accacctcca gaaaggggta gggtagaatg ccacctgttg cctggtgtgt gctaacctgg 2700

agcaggtagg ggtaagactc aatagtcatc ttttaccaaa tgggtttgcc ccaggttaat 2760

aagaggggtc tagtaggcct tggactgggc cgttgccaca cctggcactt agtgaccatc 2820

atcatgagaa actggagagt gcgtgctgga acacgtggtg ccatcttggc tttaggatcc 2880

ttttgatcgt tgtgtccaag gcttgtgtgt gtgtgagtgt gtgggagaca actccgaatg 2940

tttaattctg gaagagggat gtaacattgc cctgaggatg gtgaagttgg tatacattta 3000

taaagtacgg aatggtgtca atgaatgcaa ttctatgtat atggacttaa ctgagatggg 3060

caaatagaaa ctagctctgg gaaggaacat gtgcactact tcaagaaaga ttggaagcat 3120

gtgtggctca tgggaaataa ccaggtctta aacagcacaa actgaattcg tggaccagga 3180

aggtcttaaa cagcacaaac tgaattcatg gaaaaatgac aaatttgaga agtctcccag 3240

taagctggaa cttttctggt ttggttaaca aaaggtttct tgatttgttt caagatttaa 3300

agccaaaggt gtgggttcat gacttaggtg tcattgcgtg tgggtacaat atttatatat 3360

ggcgaattca gataaacatt ggtcaaagat ggtctctgga aaaacaaaat agaggctgca 3420

ttacggaaat aagatttctg gtctgttccc tgggacatgc ttaaaaaata caatagctat 3480

tatgtatggt tttatttttc atgtggtttc ggggaaacaa cacggtttta aggatggttt 3540

ctaaagatga aattaaaaat tgttccacaa gggttaagtg tctggtggta aagttgggag 3600

aaactggatg gatgcacatc gcatggctgg tggcgagccc actgggcagg cgacccagga 3660

aagggtttgg agtgaggctg tgagcactta cttgatattt tacaagtttg gatttggtgt 3720

taattttttt ccttgtccgt tttttcctgt tgactaacgg ctcatctttt ccttgttttt 3780

gttttttttt tgttcttttt ttccatgtca ctaaaggccc tcgggaccaa ggatcacgtc 3840

atgactccga acaggataat tcagacaaca acaccatctt tgtgcaaggc ctgggtgaga 3900

atgttacaat tgagtctgtg gctgattact tcaagcagat tggtattatt aagacaaaca 3960

agaaaacggg acagcccatg attaatttgt acacagacag ggaaactggc aagctgaagg 4020

gagaggcaac ggtctctttt gatgacccac cttcagctaa agcagctatt gactggtttg 4080

atggtaaaga attctccgga aatcctatca aggtctcatt tgctactcgc cgggcagact 4140

ttaatcgggg tggtggcaat ggtcgtggag gccgagggcg aggaggaccc atgggccgtg 4200

gaggctatgg aggtggtggc agtggtggtg gtggccgagg aggatttccc agtggaggtg 4260

gtggcggtgg aggacagcag cgagctggtg actggaagtg tcctaatccc acctgtgaga 4320

atatgaactt ctcttggagg aatgaatgca accagtgtaa ggcccctaaa ccagatggcc 4380

caggaggggg accaggtggc tctcacatgg ggggtaacta cggggatgat cgtcgtggtg 4440

gcagaggagg ctatgatcga ggcggctacc ggggccgcgg cggggaccgt ggaggcttcc 4500

gagggggccg gggtggtggg gacagaggtg gctttggccc tggcaagatg gattccaggg 4560

gtgagcacag acaggatcgc agggagaggc cgtattaatt agcctggctc cccaggttct 4620

ggaacagctt tttgtcctgt acccagtgtt accctcgtta ttttgtaacc ttccaattcc 4680

tgatcaccca agggtttttt tgtgtcggac tatgtaattg taactatacc tctggttccc 4740

attaaaagtg accattttag ttaaattttg ttcctcttcc cccttttcac tttcctggaa 4800

gatcgatgtc ccgatcagga aggtagagag ttttcctgtt cagattaccc tgcccagcag 4860

gaactggaat acagtgttcg gggagaaggc caaatgatat ccttgagagc agagattaaa 4920

cttttctgtc atggggaaag ttggtgtata aatgagaaat gaagaacatg ggatgtcatg 4980

agtgttggcc taaatttgcc cagctatggg gaatttttcc tttaccacat ttatttgcat 5040

actggtctta gtttatttgc agcagtttat ccctttttaa gaactctttg atcttttggc 5100

ccttttaatg gtgaggctca aacaaactac atttaaatgg ggcagtattc agatttgacc 5160

atggtggaga gcgcttagcc actctgggtc tttcacagga aggagagtaa ctgagtgctg 5220

caggagtttg tggagtggag tcaggatcta ggaggtgagt gactcccttc ctagctgccc 5280

tggtgaacag cgcttgggta gatacctgct ataaggagac tggtctggct gggttacttt 5340

cacatcctgc ctgtactcag agggcttgag gtcattgaca ttatgagatt ttaggcttga 5400

tccctttttg attggagggt ggaaggccct cctaagggaa tgataagtga taagaggggg 5460

aaggggttgc agccaatgag ttaaaacctt agagcagtgc tcctcagcct cttaccatgt 5520

ggttgtaaac ttgcacgtac ctgccaacca gttatttagc atgcttttta ttttagttac 5580

acagagcgta acattaaccc aagagcagaa aggttttatt tacagggttt tcgaacttgg 5640

tttgtaagac agctgccatc acaagcatag cttacaaatg tgctggggac ccctaattgg 5700

gaagtgcttt cctctcaaat ttttattttt tatttttaga gacagagtct tgctctgtca 5760

tccaggctgg agtgcagtgg cgtgatctcg gctcactgta gcctctgcct cctgagttta 5820

agcgattctc ctgcctcagg agaatcccag cttctgagta gctgagacta caggcgtggg 5880

ccaccatgcc cacctagttt ttgcattttt agtggaggtg tggtttcact gtgttggcca 5940

ggctggtctc ttaactcctg acctcaggtg atccacctgc cttggcctcc caaagtgctg 6000

gaattacagg catgagccgc tgcatctggc catcctctca aattttcaag tgttccacaa 6060

gtatgttctc tactgaagag ttgctgcatc cttgaatctt gggtgatttg aggcacagaa 6120

actatgactt tattttttga gatggagttt tgctcttgtt gcccaggctg gagtgcaatg 6180

gcacgttttt cggcttaccg caactgccgc ctcctgggtt caagcgatag ctgggattac 6240

aggcatgcgc caccatgccc agctaattta tttgtatttt tagtagagac ggggtttctc 6300

catgttggtc aggctggtct cgaactcccg acctcaggtg atgtgcacac ctcagcctcc 6360

caataaacca tgacttttaa gaggaatagc aggtttactt cccctgccag cattggggtg 6420

ctctctaagc aacagtaggc ggagagtggt ctggcgtatt aaaaacaaag gatcgtcaag 6480

tgggccttcc caggcattgc tttgacttag tacatgtaga ggatgtggca gttctctccg 6540

tccctgccac tgctggtttc tttgttaaat gtttagttga aatggcctga tacgatattt 6600

gagtagttca ctgttggtgc tttgcctagc aggattctaa tcttgctttg gttgtggtcc 6660

cctgatgccc tcctgttagg agtggaggag gtcgaagctc cttgtaagat atgattactg 6720

ggaccattag tgtcaagttc ctgtgtcctt caaatggcat atgtgattgg ccttgacctt 6780

aaaaggaaat agggtcccag gtgactgttt agtgggtagg tccagtttgg ggggatcttc 6840

caggagaatg gatagagaca cctagcagca gagagaacat tggtgcctct caagccaacc 6900

tcccacctca gcctcccaag tagctgggac tacaggtgct tcctcgctac cacacctggg 6960

taattttttt tttaattact tttttttttt taagaaacag ggtttcactg ggttgcccag 7020

gctggtcttg aactggcctc aagtgacctg cctgcctcag cctcccaaag tgctgggatc 7080

acaggtgtga cccactgcgt ttggccagaa tactctattc ttactgaatg attgaaatct 7140

gtcttgaagc attaggtgtc ccatttttgt gagttggaat tgggacaggc taagtaggaa 7200

gtgaggaggg tggggagagc tgtgctgtag gtctgtttgt cccttccttg atgtagcctt 7260

cagttagccc tttcagcttt tttccccatc ttgtgccggg ccttcctggg tttcagtact 7320

tggatgtagg gctgcagtta tgtcagtggt gggtagattg accaggaatt aaggtctagg 7380

gtccagccca tgtgagactt gactcactga tctaccttta ggcatgtctt ccttccagtc 7440

tcatcctttt taaatttttt tttttttttt gagacggtct cactctcacc caggctggat 7500

tgcagtggtg tgatctcgac caactgcaac ctctgcctcc cacccgcaag ctatctgccc 7560

acctcagcct ctggagtagc tgggacggga ctacaggcac ctgccaccat gactggctaa 7620

tttttttttg tattttttgt ggagatgggg tcttgccatg ttgctcaggc tggtctggtc 7680

tcaaaactgc tctgggctca agtgattgtc ccactttggc ctcccagagt gctgggatta 7740

aggtgtgaga tactgtgtcc ggctatgaaa attttatttt taattaactt gtatatattt 7800

atggggtaca atgtcctatt tctgtacatg tacacattgt ggaatcaaat caggctaata 7860

tatccatcac ttcatatcat tagcatgaat gagaacatac aaagccactc ttagaaaatt 7920

ttgaaattta tgttatttca gcccttttat gctggaggtt gcaaatgttt tgtgaataat 7980

cagaccaaaa ataaaaacaa aaaatgattg acttcagtca ttcagtaaga a 8031

<210> 17
<211> 7140
<212> DNA
<213> Artificial Sequence

<220>
<223> sequence delta 1-6

<400> 17
acttaagctt cgacgcagga ggcggggctg ctcagtcctc caggcgtcgg tactcagcgg 60

tgttggaact tcgttgcttg cttgcctgtg cgcgcgtgcg cggacatggc ctcaaacgat 120

tatacccaac aagcaaccca aagctatggg gcctacccca cccagcccgg gcagggctat 180

tcccagcaga gcagtcagcc ctacggacag cagagttaca gtggttatag ccagtccacg 240

gacacttcag gctatggcca gagcagctat tcttcttatg gccagagcca gaacacaggc 300

tatggaactc agtcaactcc ccagggatat ggctcgactg gcggctatgg cagtagccag 360

agctcccaat cgtcttacgg gcagcagtcc tcctaccctg gctatggcca gcagccagct 420

cccagcagca cctcgggaag ttacggtagc agttctcaga gcagcagcta tgggcagccc 480

cagagtggga gctacagcca gcagcctagc tatggtggac agcagcaaag ctatggacag 540

cagcaaagct ataatccccc tcagggctat ggacagcaga accagtacaa cagcagcagt 600

ggtggtggag gtggaggtgg aggtggaggt aactatggcc aagatcaatc ctccatgagt 660

agtggtggtg gcagtggtgg cggttatggc aatcaagacc agagtggtgg aggtggcagc 720

ggtggctatg gacagcagga ccgtggaggc cgcggcaggg gtggcagtgg tggcggcggc 780

ggcggcggcg gtggtggtta caaccgcagc agtggtggct atgaacccag aggtcgtgga 840

ggtggccgtg gaggcagagg tggcatgggg taggtgtctc atgagccagg gagtatcttt 900

ggtggggagt gtggaggatt gcatgaatct ccctgaagcc agtccctagt gcatggttta 960

gtattcttgt tgtctaggga tctgtgaggg ctttgatttg ggggcagtga ctttcttttt 1020

acatccccat tttatttttg tgagaacttg ggagcctgaa ctcccatcca taccactgaa 1080

tagagatttt gagtaatgat acttgtttcc aaaaaaaaag aaaccataca tagatacgta 1140

tggattggag tcattaatat cctaggcaag aaacatggaa gtgaagactt ctttctctgc 1200

aagggaaacc gatgatccca ctcctgggaa atagtaggga aacttggtat gtgtattccc 1260

atgtgtgaag taaaacctta gatttgatgt taaaacagtt gtcaaatctg ttggtaaata 1320

agatttgaag gaccctactc tgtctccctt gaaaaagggg aggaatgtca gtgttactgt 1380

ttttggaaaa agtagatttt taaaccgagt ttggaaatgg taagtatgca gaggtgggtg 1440

ggggcaatct caaaaacgtg caaaaatgag gaaaacaaaa atgaggaaat gtgtgcgtgt 1500

gtttaatgca aaactttaaa aagaaaaaca actgttatgt gactgttaac ttgctctgca 1560

ttctttcaaa gcaaaaacca caaaaatgtg ttcaaggcta ccccagcctg gtgtgaaatg 1620

tcttctgggt aaattggggt agggttttta aaccaactac ttggttgtca accacttgcg 1680

acaagaggaa aaaaaaacat ctgctccatc ggaagaacga ccaaggaaaa tgggttattt 1740

tttttccaga ggaaatagat aacgtaacct tttaaagcaa aatctttata aactgtgtct 1800

gagaaattgc acacgtgtgt gtgacatgct caaaggtcag acaaggggtg gtcaggaagg 1860

gatgtatttt agtagccact tgtatctttt tccaaaaaca cctacccatg tttggggaat 1920

gttaaacaaa atcaaaaaac aaccttttgt agccgttgga agcttcatgt cctttcttct 1980

aacttgtctt ctccagcgga agtgaccgtg gtggcttcaa taaatttggt ggtaagtgaa 2040

cagagtttcc aaaattccca actcccagca atgctttgtc tgattgttca tttgcagatg 2100

tcttagcgtg ttaatttaaa tgtcaaaggt tttgaggtgt ccagaaccac ctccagaaag 2160

gggtagggta gaatgccacc tgttgcctgg tgtgtgctaa cctggagcag gtaggggtaa 2220

gactcaatag tcatctttta ccaaatgggt ttgcctggaa gagggatgta acattgccct 2280

gaggatggtg aagttggtat acatttataa agtacggaat ggtgtcaatg aatgcaattc 2340

tatgtatatg gacttggaac ttttctggtt tggttaacaa aggtttcttt gatttgtttc 2400

aagatttaaa gccaaaggtg tgggttcatg acttaggtgt cattgcgtgt gggtacaata 2460

tttatatatg gcgaattcag ataaacattg gtcaaagatg gtctctggaa aaacaaaata 2520

gaggctgcat tacggaaata agatttctgg tctgttccct gggacatgct taaaaaatac 2580

aatagctatt atgtatggtt tttattttca tgtggtttcg gggaaacaac acggttttaa 2640

ggatggtttc taaagatgaa attaaaaatt gttccacaag ggttaagtgt ctggtggtaa 2700

agttgggaga aactggatgg atgcacatcg catggctggt ggcgagccca ctgggcaggc 2760

gacccaggaa agggtttgga gtgaggctgt gagcacttac ttgatatttt acaagtttgg 2820

atttggtgtt aatttttttc cttgtccgtt ttttcctgtt gactaacggc tcatcttttc 2880

cttgtttttg tttttttttt gttctttttt tccatgtcac taaaggccct cgggaccaag 2940

gatcacgtca tgactccgaa caggataatt cagacaacaa caccatcttt gtgcaaggcc 3000

tgggtgagaa tgttacaatt gagtctgtgg ctgattactt caagcagatt ggtattatta 3060

agacaaacaa gaaaacggga cagcccatga ttaatttgta cacagacagg gaaactggca 3120

agctgaaggg agaggcaacg gtctcttttg atgacccacc ttcagctaaa gcagctattg 3180

actggtttga tggtaaagaa ttctccggaa atcctatcaa ggtctcattt gctactcgcc 3240

gggcagactt taatcggggt ggtggcaatg gtcgtggagg ccgagggcga ggaggaccca 3300

tgggccgtgg aggctatgga ggtggtggca gtggtggtgg tggccgagga ggatttccca 3360

gtggaggtgg tggcggtgga ggacagcagc gagctggtga ctggaagtgt cctaatccca 3420

cctgtgagaa tatgaacttc tcttggagga atgaatgcaa ccagtgtaag gcccctaaac 3480

cagatggccc aggaggggga ccaggtggct ctcacatggg gggtaactac ggggatgatc 3540

gtcgtggtgg cagaggaggc tatgatcgag gcggctaccg gggccgcggc ggggaccgtg 3600

gaggcttccg agggggccgg ggtggtgggg acagaggtgg ctttggccct ggcaagatgg 3660

attccagggg tgagcacaga caggatcgca gggagaggcc gtattaatta gcctggctcc 3720

ccaggttctg gaacagcttt ttgtcctgta cccagtgtta ccctcgttat tttgtaacct 3780

tccaattcct gatcacccaa gggttttttt gtgtcggact atgtaattgt aactatacct 3840

ctggttccca ttaaaagtga ccattttagt taaattttgt tcctcttccc ccttttcact 3900

ttcctggaag atcgatgtcc cgatcaggaa ggtagagagt tttcctgttc agattaccct 3960

gcccagcagg aactggaata cagtgttcgg ggagaaggcc aaatgatatc cttgagagca 4020

gagattaaac ttttctgtca tggggaaagt tggtgtataa atgagaaatg aagaacatgg 4080

gatgtcatga gtgttggcct aaatttgccc agctatgggg aatttttcct ttaccacatt 4140

tatttgcata ctggtcttag tttatttgca gcagtttatc cctttttaag aactctttga 4200

tcttttggcc cttttaatgg tgaggctcaa acaaactaca tttaaatggg gcagtattca 4260

gatttgacca tggtggagag cgcttagcca ctctgggtct ttcacaggaa ggagagtaac 4320

tgagtgctgc aggagtttgt ggagtggagt caggatctag gaggtgagtg actcccttcc 4380

tagctgccct ggtgaacagc gcttgggtag atacctgcta taaggagact ggtctggctg 4440

ggttactttc acatcctgcc tgtactcaga gggcttgagg tcattgacat tatgagattt 4500

taggcttgat ccctttttga ttggagggtg gaaggccctc ctaagggaat gataagtgat 4560

aagaggggga aggggttgca gccaatgagt taaaacctta gagcagtgct cctcagcctc 4620

ttaccatgtg gttgtaaact tgcacgtacc tgccaaccag ttatttagca tgctttttat 4680

tttagttaca cagagcgtaa cattaaccca agagcagaaa ggttttattt acagggtttt 4740

cgaacttggt ttgtaagaca gctgccatca caagcatagc ttacaaatgt gctggggacc 4800

cctaattggg aagtgctttc ctctcaaatt tttatttttt atttttagag acagagtctt 4860

gctctgtcat ccaggctgga gtgcagtggc gtgatctcgg ctcactgtag cctctgcctc 4920

ctgagtttaa gcgattctcc tgcctcagga gaatcccagc ttctgagtag ctgagactac 4980

aggcgtgggc caccatgccc acctagtttt tgcattttta gtggaggtgt ggtttcactg 5040

tgttggccag gctggtctct taactcctga cctcaggtga tccacctgcc ttggcctccc 5100

aaagtgctgg aattacaggc atgagccgct gcatctggcc atcctctcaa attttcaagt 5160

gttccacaag tatgttctct actgaagagt tgctgcatcc ttgaatcttg ggtgatttga 5220

ggcacagaaa ctatgacttt attttttgag atggagtttt gctcttgttg cccaggctgg 5280

agtgcaatgg cacgtttttc ggcttaccgc aactgccgcc tcctgggttc aagcgatagc 5340

tgggattaca ggcatgcgcc accatgccca gctaatttat ttgtattttt agtagagacg 5400

gggtttctcc atgttggtca ggctggtctc gaactcccga cctcaggtga tgtgcacacc 5460

tcagcctccc aataaaccat gacttttaag aggaatagca ggtttacttc ccctgccagc 5520

attggggtgc tctctaagca acagtaggcg gagagtggtc tggcgtatta aaaacaaagg 5580

atcgtcaagt gggccttccc aggcattgct ttgacttagt acatgtagag gatgtggcag 5640

ttctctccgt ccctgccact gctggtttct ttgttaaatg tttagttgaa atggcctgat 5700

acgatatttg agtagttcac tgttggtgct ttgcctagca ggattctaat cttgctttgg 5760

ttgtggtccc ctgatgccct cctgttagga gtggaggagg tcgaagctcc ttgtaagata 5820

tgattactgg gaccattagt gtcaagttcc tgtgtccttc aaatggcata tgtgattggc 5880

cttgacctta aaaggaaata gggtcccagg tgactgttta gtgggtaggt ccagtttggg 5940

gggatcttcc aggagaatgg atagagacac ctagcagcag agagaacatt ggtgcctctc 6000

aagccaacct cccacctcag cctcccaagt agctgggact acaggtgctt cctcgctacc 6060

acacctgggt aatttttttt ttaattactt tttttttttt aagaaacagg gtttcactgg 6120

gttgcccagg ctggtcttga actggcctca agtgacctgc ctgcctcagc ctcccaaagt 6180

gctgggatca caggtgtgac ccactgcgtt tggccagaat actctattct tactgaatga 6240

ttgaaatctg tcttgaagca ttaggtgtcc catttttgtg agttggaatt gggacaggct 6300

aagtaggaag tgaggagggt ggggagagct gtgctgtagg tctgtttgtc ccttccttga 6360

tgtagccttc agttagccct ttcagctttt ttccccatct tgtgccgggc cttcctgggt 6420

ttcagtactt ggatgtaggg ctgcagttat gtcagtggtg ggtagattga ccaggaatta 6480

aggtctaggg tccagcccat gtgagacttg actcactgat ctacctttag gcatgtcttc 6540

cttccagtct catccttttt aaattttttt tttttttttg agacggtctc actctcaccc 6600

aggctggatt gcagtggtgt gatctcgacc aactgcaacc tctgcctccc acccgcaagc 6660

tatctgccca cctcagcctc tggagtagct gggacgggac tacaggcacc tgccaccatg 6720

actggctaat ttttttttgt attttttgtg gagatggggt cttgccatgt tgctcaggct 6780

ggtctggtct caaaactgct ctgggctcaa gtgattgtcc cactttggcc tcccagagtg 6840

ctgggattaa ggtgtgagat actgtgtccg gctatgaaaa ttttattttt aattaacttg 6900

tatatattta tggggtacaa tgtcctattt ctgtacatgt acacattgtg gaatcaaatc 6960

aggctaatat atccatcact tcatatcatt agcatgaatg agaacataca aagccactct 7020

tagaaaattt tgaaatttat gttatttcag cccttttatg ctggaggttg caaatgtttt 7080

gtgaataatc agaccaaaaa taaaaacaaa aaatgattga cttcagtcat tcagtaagaa 7140

<210> 18
<211> 3707
<212> DNA
<213> Artificial Sequence

<220>
<223> sequence delta 1-6 UTR

<400> 18
acttaagctt cgacgcagga ggcggggctg ctcagtcctc caggcgtcgg tactcagcgg 60

tgttggaact tcgttgcttg cttgcctgtg cgcgcgtgcg cggacatggc ctcaaacgat 120

tatacccaac aagcaaccca aagctatggg gcctacccca cccagcccgg gcagggctat 180

tcccagcaga gcagtcagcc ctacggacag cagagttaca gtggttatag ccagtccacg 240

gacacttcag gctatggcca gagcagctat tcttcttatg gccagagcca gaacacaggc 300

tatggaactc agtcaactcc ccagggatat ggctcgactg gcggctatgg cagtagccag 360

agctcccaat cgtcttacgg gcagcagtcc tcctaccctg gctatggcca gcagccagct 420

cccagcagca cctcgggaag ttacggtagc agttctcaga gcagcagcta tgggcagccc 480

cagagtggga gctacagcca gcagcctagc tatggtggac agcagcaaag ctatggacag 540

cagcaaagct ataatccccc tcagggctat ggacagcaga accagtacaa cagcagcagt 600

ggtggtggag gtggaggtgg aggtggaggt aactatggcc aagatcaatc ctccatgagt 660

agtggtggtg gcagtggtgg cggttatggc aatcaagacc agagtggtgg aggtggcagc 720

ggtggctatg gacagcagga ccgtggaggc cgcggcaggg gtggcagtgg tggcggcggc 780

ggcggcggcg gtggtggtta caaccgcagc agtggtggct atgaacccag aggtcgtgga 840

ggtggccgtg gaggcagagg tggcatgggg taggtgtctc atgagccagg gagtatcttt 900

ggtggggagt gtggaggatt gcatgaatct ccctgaagcc agtccctagt gcatggttta 960

gtattcttgt tgtctaggga tctgtgaggg ctttgatttg ggggcagtga ctttcttttt 1020

acatccccat tttatttttg tgagaacttg ggagcctgaa ctcccatcca taccactgaa 1080

tagagatttt gagtaatgat acttgtttcc aaaaaaaaag aaaccataca tagatacgta 1140

tggattggag tcattaatat cctaggcaag aaacatggaa gtgaagactt ctttctctgc 1200

aagggaaacc gatgatccca ctcctgggaa atagtaggga aacttggtat gtgtattccc 1260

atgtgtgaag taaaacctta gatttgatgt taaaacagtt gtcaaatctg ttggtaaata 1320

agatttgaag gaccctactc tgtctccctt gaaaaagggg aggaatgtca gtgttactgt 1380

ttttggaaaa agtagatttt taaaccgagt ttggaaatgg taagtatgca gaggtgggtg 1440

ggggcaatct caaaaacgtg caaaaatgag gaaaacaaaa atgaggaaat gtgtgcgtgt 1500

gtttaatgca aaactttaaa aagaaaaaca actgttatgt gactgttaac ttgctctgca 1560

ttctttcaaa gcaaaaacca caaaaatgtg ttcaaggcta ccccagcctg gtgtgaaatg 1620

tcttctgggt aaattggggt agggttttta aaccaactac ttggttgtca accacttgcg 1680

acaagaggaa aaaaaaacat ctgctccatc ggaagaacga ccaaggaaaa tgggttattt 1740

tttttccaga ggaaatagat aacgtaacct tttaaagcaa aatctttata aactgtgtct 1800

gagaaattgc acacgtgtgt gtgacatgct caaaggtcag acaaggggtg gtcaggaagg 1860

gatgtatttt agtagccact tgtatctttt tccaaaaaca cctacccatg tttggggaat 1920

gttaaacaaa atcaaaaaac aaccttttgt agccgttgga agcttcatgt cctttcttct 1980

aacttgtctt ctccagcgga agtgaccgtg gtggcttcaa taaatttggt ggtaagtgaa 2040

cagagtttcc aaaattccca actcccagca atgctttgtc tgattgttca tttgcagatg 2100

tcttagcgtg ttaatttaaa tgtcaaaggt tttgaggtgt ccagaaccac ctccagaaag 2160

gggtagggta gaatgccacc tgttgcctgg tgtgtgctaa cctggagcag gtaggggtaa 2220

gactcaatag tcatctttta ccaaatgggt ttgcctggaa gagggatgta acattgccct 2280

gaggatggtg aagttggtat acatttataa agtacggaat ggtgtcaatg aatgcaattc 2340

tatgtatatg gacttggaac ttttctggtt tggttaacaa aaggtttctt gatttgtttc 2400

aagatttaaa gccaaaggtg tgggttcatg acttaggtgt cattgcgtgt gggtacaata 2460

tttatatatg gcgaattcag ataaacattg gtcaaagatg gtctctggaa aaacaaaata 2520

gaggctgcat tacggaaata agatttctgg tctgttccct gggacatgct taaaaaatac 2580

aatagctatt atgtatggtt tttattttca tgtggtttcg gggaaacaac acggttttaa 2640

ggatggtttc taaagatgaa attaaaaatt gttccacaag ggttaagtgt ctggtggtaa 2700

agttgggaga aactggatgg atgcacatcg catggctggt ggcgagccca ctgggcaggc 2760

gacccaggaa agggtttgga gtgaggctgt gagcacttac ttgatatttt acaagtttgg 2820

atttggtgtt aatttttttc cttgtccgtt ttttcctgtt gactaacggc tcatcttttc 2880

cttgtttttg tttttttttt gttctttttt tccatgtcac taaaggccct cgggaccaag 2940

gatcacgtca tgactccgaa caggataatt cagacaacaa caccatcttt gtgcaaggcc 3000

tgggtgagaa tgttacaatt gagtctgtgg ctgattactt caagcagatt ggtattatta 3060

agacaaacaa gaaaacggga cagcccatga ttaatttgta cacagacagg gaaactggca 3120

agctgaaggg agaggcaacg gtctcttttg atgacccacc ttcagctaaa gcagctattg 3180

actggtttga tggtaaagaa ttctccggaa atcctatcaa ggtctcattt gctactcgcc 3240

gggcagactt taatcggggt ggtggcaatg gtcgtggagg ccgaggcgga ggaggaccca 3300

tgggccgtgg aggctatgga ggtggtggca gtggtggtgg tggccgagga ggatttccca 3360

gtggaggtgg tggcggtgga ggacagcagc gagctggtga ctggaagtgt cctaatccca 3420

cctgtgagaa tatgaacttc tcttggagga atgaatgcaa ccagtgtaag gcccctaaac 3480

cagatggccc aggaggggga ccaggtggct ctcacatggg gggtaactac ggggatgatc 3540

gtcgtggtgg cagaggaggc tatgatcgag gcggctaccg gggccgcggc ggggaccgtg 3600

gaggcttccg agggggccgg ggtggtgggg acagaggtgg ctttggccct ggcaagatgg 3660

attccagggg tgagcacaga caggatcgca gggagaggcc gtattaa 3707

<210> 19
<211> 1127
<212> DNA
<213> Artificial Sequence

<220>
<223> intron 7 delta 1 et 2

<400> 19
gtaggtgtct catgagccag ggagtatctt tggtggggag tgtggaggat tgcatgaatc 60

tccctgaagc cagtccctag tgcatggttt agtattcttg ttgtctaggg atctgtgagg 120

gctttgattt gggggcagtg actttctttt tacatcccca ttttattttt gtgagaactt 180

gggagcctga actcccatcc ataccactga atagagattt tgagtaatga tacttgtttc 240

caaaaaaaaa gaaaccatac atagatacgt atggattgga gtcattaata tcctaggcaa 300

gaaacatgga agtgaagact tctttctctg caagggaaac cgatgatccc actcctggga 360

aatagtaggg aaacttggta tgtgtattcc catgtgtgaa gtaaaacctt agatttgatg 420

ttaaaacagt tgtcaaatct gttggtaaat aagatttgaa ggaccctact ctgtctccct 480

tgaaaaaggg gaggaatgtc agtgttactg tttttggaaa aagtagattt ttaaaccgag 540

tttggaaatg gtaagtatgc agaggtgggt gggggcaatc tcaaaaacgt gcaaaaatga 600

ggaaaacaaa aatgaggaaa tgtgtgcgtg tgtttaatgc aaaactttaa aaagaaaaac 660

aactgttatg tgactgttaa cttgctctgc attctttcaa agcaaaaacc acaaaaatgt 720

gttcaaggct accccagcct ggtgtgaaat gtcttctggg taaattgggg tagggttttt 780

aaaccaacta cttggttgtc aaccacttgc gacaagagga aaaaaaaaca tctgctccat 840

cggaagaacg accaaggaaa atgggttatt ttttttccag aggaaataga taacgtaacc 900

ttttaaagca aaatctttat aaactgtgtc tgagaaattg cacacgtgtg tgtgacatgc 960

tcaaaggtca gacaaggggt ggtcaggaag ggatgtattt tagtagccac ttgtatcttt 1020

ttccaaaaac acctacccat gtttggggaa tgttaaacaa aatcaaaaaa caaccttttg 1080

tagccgttgg aagcttcatg tcctttcttc taacttgtct tctccag 1127

<210> 20

<211>  894
<212>  DNA
<213>  Artificial Sequence

<220>
<223>  intron 8 delta 3 à 6

<400>  20
gtaagtgaac agagtttcca aaattcccaa ctcccagcaa tgctttgtct gattgttcat      60

ttgcagatgt cttagcgtgt taatttaaat gtcaaaggtt ttgaggtgtc cagaaccacc     120

tccagaaagg ggtagggtag aatgccacct gttgcctggt gtgtgctaac ctggagcagg     180

taggggtaag actcaatagt catcttttac caaatgggtt tgcctggaag agggatgtaa     240

cattgccctg aggatggtga agttggtata catttataaa gtacggaatg gtgtcaatga     300

atgcaattct atgtatatgg acttggaact tttctggttt ggttaacaaa aggtttcttg     360

atttgtttca agatttaaag ccaaaggtgt gggttcatga cttaggtgtc attgcgtgtg     420

ggtacaatat ttatatatgg cgaattcaga taaacattgg tcaaagatgg tctctggaaa     480

aacaaaatag aggctgcatt acggaaataa gatttctggt ctgttccctg ggacatgctt     540

aaaaaataca atagctatta tgtatggttt ttattttcat gtggtttcgg ggaaacaaca     600

cggttttaag gatggtttct aaagatgaaa ttaaaaattg ttccacaagg gttaagtgtc     660

tggtggtaaa gttgggagaa actggatgga tgcacatcgc atggctggtg gcgagcccac     720

tgggcaggcg acccaggaaa gggtttggag tgaggctgtg agcacttact tgatatttta     780

caagtttgga tttggtgtta atttttttcc ttgtccgttt tttcctgttg actaacggct     840

catcttttcc ttgtttttgt tttttttttg ttcttttttt ccatgtcact aaag           894


<210>  21
<211>  22
<212>  DNA
<213>  Artificial Sequence

<220>
<223>  primer hTLS FUS-For

<400>  21
gaattcgtgg accaggaagg tc                                               22


<210>  22
<211>  23
<212>  DNA
<213>  Artificial Sequence

<220>
<223>  primer hTLS FUS-Rev

<400>  22
cacgtgtgaa ctcaccggag tca                                              23

<210>  23
<211>  26
<212>  DNA
<213>  Artificial Sequence

<220>
<223>  primer FUS-For

<400>  23
gatttgaagt gggtagatag tgcagg                                            26


<210>  24
<211>  27
<212>  DNA
<213>  Artificial Sequence

<220>
<223>  primer FUS - Rev

<400>  24
cctttccaca ctttagttta gtcacag                                           27


<210>  25
<211>  19
<212>  DNA
<213>  Artificial Sequence

<220>
<223>  primer actin Fwd

<400>  25
ccaccagttc gccatggat                                                    19


<210>  26
<211>  19
<212>  DNA
<213>  Artificial Sequence

<220>
<223>  primer actin rev

<400>  26
ggctttgcac atgccggag                                                    19


<210>  27
<211>  22
<212>  DNA
<213>  Artificial Sequence

<220>
<223>  primer TBP fwd

<400>  27
ccaatgactc ctatgacccc ta                                                22


<210>  28
<211>  21
<212>  DNA

<213>  Artificial Sequence

<220>
<223>  primer TBP rev

<400>  28
cagccaagat tcacggtaga t                                                21


<210>  29
<211>  18
<212>  DNA
<213>  Artificial Sequence

<220>
<223>  primer Pol2 fwd

<400>  29
gctgggagac atagacca                                                    18


<210>  30
<211>  20
<212>  DNA
<213>  Artificial Sequence

<220>
<223>  primer Pol2 rev

<400>  30
ttactcccct gcatggtctc                                                  20


<210>  31
<211>  20
<212>  DNA
<213>  Artificial Sequence

<220>
<223>  primer hFUS fwd

<400>  31
gccagaacac aggctatgga                                                  20


<210>  32
<211>  20
<212>  DNA
<213>  Artificial Sequence

<220>
<223>  primer hFUS rev

<400>  32
cgattgggag ctctggctac                                                  20


<210>  33
<211>  23
<212>  DNA
<213>  Artificial Sequence

<220>

<223>  primer T-Fus fwd

<400>  33
ttatggacag acccaaaaac aca                                              23


<210>  34
<211>  20
<212>  DNA
<213>  Artificial Sequence

<220>
<223>  primer T-Fus rev

<400>  34
tgctgcccat aagaagattg                                                  20


<210>  35
<211>  24
<212>  DNA
<213>  Artificial Sequence

<220>
<223>  primer Fus delta NLS fwd

<400>  35
gcaagatgga ctcctagtgt taat                                             24


<210>  36
<211>  22
<212>  DNA
<213>  Artificial Sequence

<220>
<223>  primer FUS delat NLS rev

<400>  36
acctctacaa atgtggtatg gc                                               22


<210>  37
<211>  17
<212>  DNA
<213>  Artificial Sequence

<220>
<223>  primer Fus exon 6-8 fwd

<400>  37
cggcatgggg tcctcgg                                                     17


<210>  38
<211>  20
<212>  DNA
<213>  Artificial Sequence

<220>
<223>  primer Fus exon 6-8 rev

<400>  38

cctaggcctt gcacgaagat                                                    20

<210>  39
<211>  9
<212>  PRT
<213>  Artificial Sequence

<220>
<223>  HA tag

<400>  39

Tyr Pro Tyr Asp Val Pro Asp Tyr Ala
1               5

<210>  40
<211>  10
<212>  PRT
<213>  Artificial Sequence

<220>
<223>  myc tag

<400>  40

Glu Gln Lys Leu Ile Ser Glu Glu Asp Leu
1               5                   10

<210>  41
<211>  8
<212>  PRT
<213>  Artificial Sequence

<220>
<223>  Flag tag

<400>  41

Asp Tyr Lys Asp Asp Asp Asp Lys
1               5

## Description

### FIELD

The invention relates to a nucleic acid encoding human FUS protein comprising a sequence having at least 69% sequence identity with the sequence SEQ ID NO 1 of intron 6 and/or a sequence having at least 60% sequence identity with the sequence SEQ ID NO 2 of intron 7, said sequences being located on either side of exon 7, to a recombinant vector comprising said nucleic acid encoding human FUS protein, and their use as medicament in the treatment and/or prevention of amyotrophic lateral sclerosis (ALS). The invention also relates to a host cell comprising said nucleic acid and/or vector. The invention further relates to a pharmaceutical composition comprising said nucleic acid and/or vector for the prevention and/or treatment of amyotrophic lateral sclerosis (ALS).
The present invention finds application in the therapeutic, veterinary and diagnostic medical technical fields.
In the description below, references in square brackets ([ ]) refer to the list of references at the end of the text.

### BACKGROUND

Amyotrophic lateral sclerosis (ALS), also cited as motor neuron disease (MND) or Lou Gehrig's disease, involves degeneration of motor neurons in the brainstem, spinal cord and motor cortex, with onset around 60 years of age. The consequence of motor neuron loss includes muscle weakness and muscle atrophy. Depending on site of onset, the primary symptoms affect upper limbs, lower limbs or muscles of the face and neck. In other words, people who suffer from Amyotrophic lateral sclerosis (ALS) are losing progressively their capacity eliciting efficient movements, and this culminates to loss of respiratory capacity causing death.

The cause and the biological mechanisms involved in amyotrophic lateral sclerosis (ALS) are not known. However, it appears that this disease involves both genetic and environmental contributions. In particular, a subset of ALS cases are dominantly inherited, demonstrating a genetic contribution, in these so-called familial ALS cases (fALS). fALS patients develop usually motor symptoms earlier, and life expectancy is shorter than non familial (so-called sporadic) patients. Most of the affected people develop their motor symptoms at about 50 - 60 years of age, yet there are cases with onset below 40 years of age. In Europe, the disease affects about two to three new people per 100,000 per year.

Once affected, the mean survival time is about 2 to 6 years. To date, there is no treatment and/or any mean for treating the etiological causes of amyotrophic lateral sclerosis (ALS). Only two treatments are currently approved by the regulatory agencies. Riluzole has a modest effect in prolonging lifespan, yet does not ameliorate function, while edaravone has shown moderate protective activity in a small subset of patients. ALS patients also undergo a number of treatments to manage the progression of symptoms, that can be considered as comfort care. A reason for the failure to develop new efficient drugs might rely on the fact that ALS is rather a compendium of multiple diseases with completely different causes and mechanisms converging to similar clinical picture in end stage. It is thus highly needed to develop new therapeutic strategies, for example which target etiological mechanisms.

There is therefore a real need for a compound and/or method overcoming the shortcomings, disadvantages and obstacles of prior art, particularly for a compound and or method for treating and/or preventing the amyotrophic lateral sclerosis (ALS).

### Description

The present invention allows to overcome the drawback and inconvenient of the prior art by providing a nucleic acid encoding human FUS protein comprising a sequence having at least 69% sequence identity with the sequence SEQ ID NO 1 of intron 6 and/or a sequence having at least 60% sequence identity with the sequence SEQ ID NO 2 of intron 7.

The inventors have surprisingly and unexpectedly demonstrated that a nucleic acid according to the invention are useful to treat people suffering from ALS. In particular, the inventors have surprisingly demonstrated that the nucleic acid of the present invention allows to treat and cure amyotrophic lateral sclerosis (ALS), in particular amyotrophic lateral sclerosis (ALS), from patients affected by severe juvenile forms of ALS related to mutations in the *FUS* gene.

In particular, the inventors have surprisingly demonstrated that the invention is able to simultaneously decrease the mutant protein and replete in normal, physiologically active wild type protein while avoiding excess toxic production of the wild type protein.

In particular, it is known that mutations in *FUS* gene lead to cytoplasmic accumulation of the physiologically nuclear mutated FUS protein. Cytoplasmic accumulation of FUS is a widespread phenomenon in most ALS cases (Tyzack 2019) and in a large subset of cases affected with fronto-temporal dementia (Urwin 2010), although not associated with germline mutations. In cases with germline mutations, it has been demonstrated that the toxicity of the mutation comes from both accumulation of cytoplasmic FUS, and simultaneous loss of nuclear FUS. Indeed, the toxicity of cytoplasmic FUS has been repeatedly demonstrated (eg Scekic-Zahirovic EMBO J 2016), and loss of nuclear FUS leads to a-number of transcriptional abnormalities which are overall toxic for neurons and synapses.

The inventors have demonstrated that an efficient treatment for ALS mediated by FUS mutations should not only correct loss of nuclear function, but also prevent increased cytoplasmic accumulation. A simple overexpression of FUS protein, through cDNA driven overexpression systems, could in principle rescue loss of function, but would not be expected to avoid accumulation of toxic cytoplasmic FUS from mutant alleles, and, most importantly has been shown to be highly toxic to neurons (Mitchell JC, 2013 "Overexpression of human wild-type FUS causes progressive motor neuron degeneration in an age- and dose-dependent fashion" Acta Neuropathologica volume 125, pages273-288(2013 DOI: 10.1007/s00401-012-1043-z [12]).

The inventors have surprisingly demonstrated that the nucleic acid encoding human FUS gene, preferably the full length nucleic acid encoding human FUS gene, is able to synergistically prevent the cytoplasmic accumulation of mutated FUS, restore FUS in the nucleus, without any toxicity. In particular, the present invention, does not induce any toxicity contrary to the classically used overexpression systems.

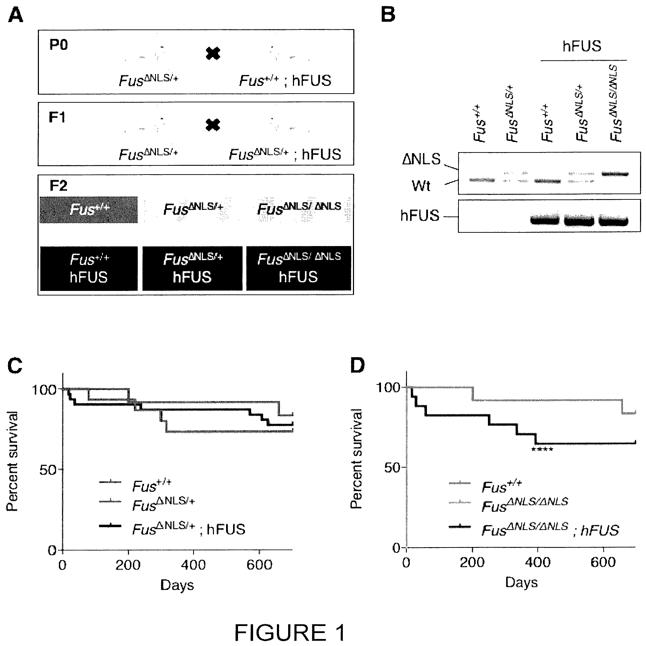

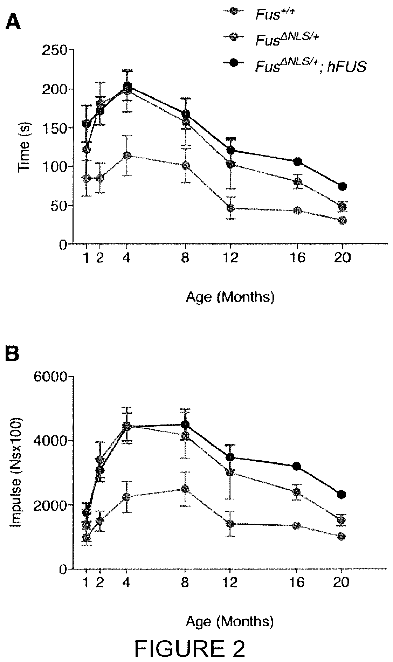

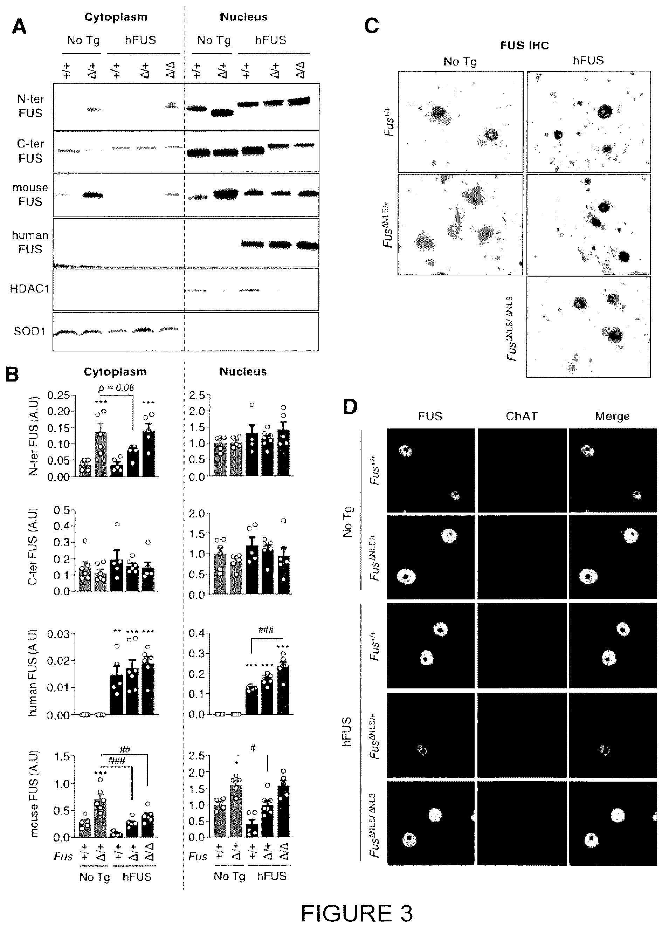

The inventors have surprisingly demonstrated that providing the nucleic acid is sufficient to fully rescue the perinatal lethality of mice carrying homologous Fus mutation (Figure 1), and rescue the motor defect observed in heterozygous Fus mutant mice (Figure 2). Surprisingly and advantageously, the rescue was associated with a complete reversal of cytoplasmic FUS accumulation, as judged from biochemical fractionation of nuclear and cytoplasmic fractions, immunohistochemistry and immunofluorescence (Figure 3). It is particularly striking and surprising that no toxicity was observed in treated transgenic mice until two years of age as wild type FUS protein overexpression driven by cDNA is by itself highly toxic (Mitchell JC, 2013 "Overexpression of human wild-type FUS causes progressive motor neuron degeneration in an age- and dose-dependent fashion" Acta Neuropathologica volume 125, pages273-288(2013 DOI: 10.1007/s00401-012-1043-z [12]). The inventors demonstrated that no toxicity is due to the fact the provided nucleic acid include genomic constructs, allowing for autoregulation of the *FUS* gene and avoiding the deleterious effects of uncontrolled FUS levels.

In the present invention, the nucleic acid encoding human FUS protein according to the invention may comprise a nucleic acid sequence having at least 69% 70%, 75%, 80%, 85%, 90%, 91%, 92%, 93%, 94%, 95%, 96%, 97%, 98%, 99%, 99.5% or 100% sequence identity with the sequence SEQ ID NO 1 of intron 6. It may be, for example, a nucleic acid of sequence SEQ ID NO 3, SEQ ID NO 4 or SEQ ID NO. 19.

In the present invention, when the nucleic acid encoding human FUS protein according to the invention comprises a nucleic acid sequence having at least 69% 70%, 75%, 80%, 85%, 90%, 91%, 92%, 93%, 94%, 95%, 96%, 97%, 98%, 99%, 99.5% or 100% sequence identity with the sequence SEQ ID NO 1 of intron 6, said sequence having an identity with the sequence SEQ ID NO 1 of intron 6 may be located before exon 7.

In the present invention, the nucleic acid encoding human FUS protein according to the invention may comprise a nucleic acid sequence having at least 60%, 65%, 70%, 75%, 80%, 85%, 90%, 91%, 92%, 93%, 94%, 95%, 96%, 97%, 98%, 99%, 99.5% or 100% sequence identity with the sequence SEQ ID NO 2. It may be, for example, a nucleic acid of sequence SEQ ID NO 5, SEQ ID NO 6, SEQ ID NO 7, SEQ ID NO 8 or SEQ ID NO 20.

In the present invention, when the nucleic acid encoding human FUS protein according to the invention comprises a nucleic acid sequence having at least 60%, 65%, 70%, 75%, 80%, 85%, 90%, 91%, 92%, 93%, 94%, 95%, 96%, 97%, 98%, 99%, 99.5% or 100% sequence identity with the sequence SEQ ID NO 2, said sequence having an identity with the sequence SEQ ID NO 2 may be located after exon 7.

In the present invention a nucleic acid encoding human FUS protein according to the invention may comprise a sequence having at least 69% sequence identity with the sequence SEQ ID NO 1 of intron 6 and a sequence having at least 60% sequence identity with the sequence SEQ ID NO 2 of intron 7, said sequences being located on either side of exon 7.

In the present invention a nucleic acid encoding human FUS protein according to the invention may comprise a sequence having at least 69%, 70%, 75%, 80%, 85%, 90%, 91%, 92%, 93%, 94%, 95%, 96%, 97%, 98%, 99%, 99.5% or 100% sequence identity with the sequence SEQ ID NO 1 of intron 6 and a sequence having least 60%, 65%, 70%, 75%, 80%, 85%, 90%, 91%, 92%, 93%, 94%, 95%, 96%, 97%, 98%, 99%, 99.5% or 100% sequence identity with the sequence SEQ ID NO 2 of intron 7, said sequences being located on either side of exon 7. It may be, for example, a nucleic acid encoding human FUS protein of sequence SEQ ID NO 9, SEQ ID NO 10, SEQ ID NO 11, SEQ ID NO 12, SEQ ID NO 13, SEQ ID NO 14, SEQ ID NO15, SEQ ID NO 16, SEQ ID NO 17, SEQ ID NO 18.

In the present invention, the localization of the nucleic acid sequence, for example sequence SEQ ID NO 1 and/or 2, in the nucleic acid encoding human FUS protein may refers to the position of the sequence from the 5' to 3' direction.

In the present invention, gene encoding human FUS means the non mutated and/or wild-type human gene of FUS. I sequence SEQ ID NO 10 located on chromosome 16 (Gene ID: 2521). The term "wild-type", as used herein, refers to a gene or gene product that has the characteristics of that gene or gene product when isolated from a naturally-occurring source. A wild-type gene or gene product (e.g., a polypeptide) is that which is most frequently observed in a population and is thus arbitrarily designed the "normal" or "wild-type" form of the gene.

In the present invention, mutated gene encoding human FUS means any form of the gene encoding human FUS protein carrying a primary sequence with any variations as compared to the wild type form. It may be any mutation that could lead to a mutated human FUS protein, for example a human protein FUS having mutations in the C terminal region of the protein, for example associated with a disease.

Unless the context requires otherwise, throughout the present specification and claims, the word "comprise" and variations thereof, such as, "comprises" and "comprising" are to be construed in an open, inclusive sense, that is as "including, but not limited to".

By "consisting of" is meant including, and limited to, whatever follows the phrase "consisting of." Thus, the phrase "consisting of" indicates that the listed elements are required or mandatory, and that no other elements may be present.

By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

In the present, the term "isolated" and its grammatical equivalents as used herein refer to the removal of a nucleic acid from its natural environment.

In the present, the term "purified" and its grammatical equivalents as used herein refer to a molecule or composition, whether removed from nature (including genomic DNA and mRNA) or synthesized (including cDNA) and/or amplified under laboratory conditions, that has been increased in purity, wherein "purity" is a relative term, not "absolute purity." It is to be understood, however, that nucleic acids and proteins can be formulated with diluents or adjuvants and still for practical purposes be isolated. For example, nucleic acids may be mixed with an acceptable carrier or diluent when used for introduction into cells.

In the present, the term "substantially purified" and its grammatical equivalents as used herein refer to a nucleic acid sequence, polypeptide, protein or other compound which is essentially free, i.e., is more than about 50% free of, more than about 70% free of, more than about 90% free of, the polynucleotides, proteins, polypeptides and other molecules that the nucleic acid, polypeptide, protein or other compound is naturally associated with.

In the present, "polynucleotide(s)", "oligonucleotide(s)", "nucleic acid(s)", "nucleotide(s)", "polynucleic acid(s)", or any grammatical equivalent as used herein refers to a polymeric form of nucleotides or nucleic acids of any length, either ribonucleotides or deoxyribonucleotides. This term refers only to the primary structure of the molecule. Thus, this term includes double and single stranded DNA, triplex DNA, as well as double and single stranded RNA. It also includes modified, for example, by methylation and/or by capping, and unmodified forms of the polynucleotide. The term is also meant to include molecules that include non-naturally occurring or synthetic nucleotides as well as nucleotide analogs. The nucleic acid sequences and vectors disclosed or contemplated herein can be introduced into a cell by, for example, transfection, transformation, or transduction.

In the present "transfection," "transformation," or "transduction" refer to the introduction of one or more exogenous polynucleotides into a host cell by using physical or chemical methods. It may be any transfection and/or transformation and/or transduction adapted method known to one skilled in the art. It may be for example, calcium phosphate DNA co-precipitation (see, e.g., Murray E. J. (ed.), Methods in Molecular Biology, Vol. 7, Gene Transfer and Expression Protocols, Humana Press (1991) [1]); DEAE- dextran; electroporation; cationic liposome-mediated transfection; tungsten particle-facilitated microparticle bombardment (Johnston, Nature, 346: 776-777 (1990) [2]); and strontium phosphate DNA co-precipitation (Brash et al., Mol. Cell Biol., 7: 2031-2034 (1987)). Phage, viral, or non- viral vectors can be introduced into host cells, after growth of infectious particles in suitable packaging cells, many of which are commercially available. In some embodiments, lipofection, nucleofection, or temporary membrane disruption (e.g., electroporation or deformation) can be used to introduce one or more exogenous polynucleotides into the host cell.

In the present "polypeptide", "peptide" and their grammatical equivalents as used herein refer to a polymer of amino acid residues.

In the present a "functional protein" is a protein which is biologically active and which, optionally, comprises glycosylation or other modifications typical for the protein in a given cellular environment.

Polypeptides and proteins disclosed herein (including functional portions and functional variants thereof) can comprise synthetic amino acids in place of one or more naturally-occurring amino acids. It may be any synthetic amino acids known to one skilled in the art, it may be for example aminocyclohexane carboxylic acid, norleucine, a-amino n-decanoic acid, homoserine, S-acetylaminomethyl-cysteine, trans-3- and trans-4-hydroxyproline, 4- aminophenylalanine, 4-nitrophenylalanine, 4-chlorophenylalanine, 4-carboxyphenylalanine, β- phenylserine β-hydroxyphenylalanine, phenylglycine, a-naphthylalanine, cyclohexylalanine, cyclohexylglycine, indoline-2-carboxylic acid, 1,2,3,4-tetrahydroisoquinoline-3-carboxylic acid, aminomalonic acid, aminomalonic acid monoamide, N'-benzyl-N'-methyl-lysine, N',N'- dibenzyl-lysine, 6-hydroxylysine, ornithine, a-aminocyclopentane carboxylic acid, a-aminocyclohexane carboxylic acid, a-aminocycloheptane carboxylic acid, a-(2-amino-2- norbornane)-carboxylic acid, α,γ-diaminobutyric acid, α,β-diaminopropionic acid, homophenylalanine, and a-tert-butylglycine.

In the present polypeptides and proteins may comprise any post-translational modifications known to one skilled in the art. It may comprise, for example one or more amino acids with post- translational modification. The post- translational modification of one or more amino acids may be for example phosphorylation, acylation including acetylation and formylation, glycosylation (including N-linked and O-linked), amidation, hydroxylation, alkylation including methylation and ethylation, ubiquitylation, addition of pyrrolidone carboxylic acid, formation of disulfide bridges, sulfation, myristoylation, palmitoylation, isoprenylation, farnesylation, geranylation, glypiation, lipoylation and iodination.

In the present, nucleic acids and/or nucleic acid sequences may be considered as "homologous" when they are derived, naturally or artificially, from a nucleic acid sequence.

In the present, proteins and/or protein sequences are "homologous" when their encoding DNAs are derived, naturally or artificially, from a nucleic acid sequence.. For example, protein as described herein may be modified by any available adapted mutagenesis method known to one skilled in the art. For example when expressed, the mutagenized nucleic acid encodes a polypeptide that is homologous to the protein encoded by the original nucleic acid. Homology is generally inferred from sequence identity between two or more nucleic acids or proteins (or sequences thereof). The precise percentage of identity between sequences that is useful in establishing homology varies with the nucleic acid and protein at issue, it may be for example a percentage of sequence identity of at least 25%, for example at least 30%, 40%, 50%, 60%, 70%, 80%, 90%, 95% or 99%.

The percentage of sequence identity may be determined by any method known to one skilled in the art. It may be determined for example by use of BLASTP and BLASTN, for example using default parameters.

In the present homologous molecules may be also termed homologs.

In the present, the terms "identical" and its grammatical equivalents as used herein and/or "sequence identity" in the context of two nucleic acid. sequences or of two amino acid sequences of polypeptides refers to the residues in a sequence which are the same when aligned for maximum correspondence over a sequence length of at least 20 contiguous nucleic acid or amino acids. It may be for example two sequences which are the same when aligned for maximum correspondence over a sequence length of at least about 50 at least about 200 contiguous nucleic acid or amino acids. The identity of the sequence may be determined by comparing a sequence to a reference sequence of the same number of contiguous positions after the two sequences are aligned optimally.

The determination of the identity and/or the comparison of a sequence to a reference sequence may be carried out with any methods and/or process adapted known to one skilled in the art. It may be for example a method of alignment of sequences for comparison, for example a method of alignment of sequences using the local homology algorithm of Smith and Waterman, Adv. Appl. Math., 2:482 (1981) [4]; using the alignment algorithm of Needleman and Wunsch, J. Mol. Biol., 48:443 (1970) [5]; using a search for similarity method of Pearson and Lipman, Proc. Nat. Acad. Sci U.S.A., 85:2444 (1988) [6]; using a computerized implementations of these algorithms, for example CLUSTAL in the PC/Gene program by Intelligences, Mountain View Calif, GAP, BESTFIT, BLAST, FASTA, and TFASTA in the Wisconsin Genetics Software Package, Genetics Computer Group (GCG), 575 Science Dr., Madison, Wis., U.S.A.) Alignment is also often performed by inspection and manual alignment.

In the present, the nucleic acid may be produced and/or obtained by any method adapted known from one skilled in the art. For example, the nucleic acid may be synthesized using the BigDye™ Terminator v3.1 Cycle Sequencing Kit (thermofisher, 4337454).

Another object of the present invention is a recombinant vector comprising a nucleic acid encoding human FUS protein comprising a sequence having at least 69% sequence identity with the sequence SEQ ID NO 1 of iritron 6 and/or a sequence having at least 60% sequence identity with the sequence SEQ ID NO 2 of intron 7.

According to the invention, the vector may be any vector known from skilled in the art adapted to the expression of a nucleic acid. It may be for example any vector selected from the vectors listed in the catalogue http://www.promega.com/vectors/mammalian_express_vectors.htm [10] or http://www.qiagen.com/pendantview/qiagenes.aspx?gaw=PROTQIAgenes 0807&gkw=mammalian+expression [11], or else http://www.scbt.com/chap _exp_vectors.php?type=pCruzTM%20Expression%20Vectors [12]. It may, for example, be the expression vector described in document WO 83/004261 [13]. The vector may be, for example Adeno-associated Virus (AAV) vectors, a plasmid, a Yeast Artificial Chromosomes (YAC) or a Bacterial Artificial Chromosome (BAC).

The vector may be any adapted adeno-associated virus (AAV) vector known to one skilled in the art and/or commercially available adapted. It may be, for example any adapted AAV disclosed in Naso et al 2017, "Adeno-Associated Virus (AAV) as a Vector for Gene Therapy" , BioDrugs. 2017 Aug;31(4):317-334. doi: 10.1007/s40259-017-0234-5 and/or in Zincarelli 2008 "Analysis of AAV Serotypes 1-9 Mediated Gene Expression and Tropism in Mice After Systemic Injection" Mol Ther. 2008 Jun;16(6):1073-80. doi: 10.1038/mt.2008.76. Epub 2008 Apr 15. It may be, for example an adeno-associated virus (AAV) vector selected from the group comprising AAV1, AAV2, AAV3, AAV4, AAV5, AAV6, AAV7, AAV8, AAV9 or AAV10 (AAVrh.10). preferably AAV9.

The vector may be any adapted plasmid known to one skilled in the art and/or commercially available. It may be for example a plasmid selected from the group comprising pMX, pUC19, pHP45-CmR, pcDNA3.1(+), pcDNA3.3-TOPO, pcDNA3.4-TOPO, pFastBac1, pET100/D-TOPO, pET151/D-TOPO, pRSET A, pYes2.1V5-His TOPO, pDONR221, pGEX-3X, preferably pcDNA3.1(+).

The vector may be any adapted Yeast Artificial Chromosome known to one skilled in the art. It may be for example a Yeast Artificial Chromosomes selected from the group comprising pYAC-RC, pYAC3

The vector may be any adapted Bacterial Artificial Chromosome known to one skilled in the art. It may be for example a Bacterial Artificial Chromosome selected from the group comprising pUvBBAC, pCC1BAC, pBAC 108L

The vector may comprise a polynucleotide sequence, for example an expression cassette, comprising the following in 5' to 3'order:
a promoter sequence;
a nucleic acid sequence encoding a human human FUS of the invention; and
a polyadenylation (polyA) sequence.

The promoter may be any promoter know to one skilled in the art. It may be for example promoters of housekeeping gene, promoters of viral gene, tissue specific promoter, promoters targeting neurons, promoters targeting muscle.

It may be for example promoters of housekeeping genes selected from the group comprising
It may be for example promoters of viral genes selected from the group comprising CMV promoter.

It may be for example a tissue specific promoter, for example a neural tissue and/or neural cell specific promoter. It may be for example a promoter selected from the group comprising NSE, Camk2a, Thy1, Fezf2, Crym promoter. It may be for example a tissue specific promoter, for example a muscle tissue and/or muscle cell specific promoter. It may be for example a promoter selected from the group comprising myoD, myf5, MyoG, Desmin promoters.

The vector may further comprise a nucleic acid sequence encoding for tag protein. It may be for example any nucleic acid sequence encoding for tag protein know to one skilled in the art..For example, it may be any tag disclosed in Parkinson J1, Blaxter M. "Expressed sequence tags: an overview."Methods Mol Biol. 2009;533:1-12. doi: 10.1007/978-1-60327-136-3_1.

The vector may further comprise additional sequence that may selectively label transgenic protein, for example aka tags, for example sequences allowing the production of the HA (sequence: YPYDVPDYA SEQ ID NO 39), myc (EQKLISEEDL SEQ ID NO 40) or FLAG (DYKDDDDK SEQ ID NO 41) tags
The vector may be selected according to the selected host cell. One skilled in the art, taking into consideration its technical knowledge, will adapt the vector in light of the host cell.

The host cell may be any host suitable for the expression of the nucleic acids or the vectors of the invention. It may, for example, be mammalian cells, *E*. *coli, Pischia pastoris, Saccharomyces cerevisiae,* or insect cells, for example an insect cell-baculovirus system, for example SF9 insect cells used in a baculovirus expression system.

Another object of the present invention is a nucleic acid and/or a recombinant vector and/or host cell for use as a medicament in the treatment and/or prevention of amyotrophic lateral sclerosis (ALS).

The nucleic acid for use as a medicament in the treatment and/or prevention of amyotrophic lateral sclerosis (ALS) may be any nucleic acid of the invention as defined above.

The vector for use as a medicament in the treatment and/or prevention of amyotrophic lateral sclerosis (ALS) may be any vector of the invention as defined above.

The host cell for use as a medicament in the treatment and/or prevention of amyotrophic lateral sclerosis (ALS) may be any host cell of the invention as defined above.

In the present, the terms "treatment," "treat," "treated" or "treating" refer to prophylaxis and/or therapy, particularly wherein the object is to prevent or slow down (lessen) an undesired physiological change or disorder, such as the development and/or progression of muscular disorders and /or inactivation and/or destruction of motoneurons. Beneficial or desired clinical results include, but are not limited to, alleviation of symptoms, diminishment of the extent of disease, stabilized (i.e., not worsening) state of disease, delay or slowing of disease progression, amelioration or palliation of the disease state, and remission (whether partial or total), whether detectable or undetectable. "Treatment" can also mean prolonging survival and/or increased quality of life as compared to expected survival and/or quality of life if not receiving treatment. Advantageously, treatment may include any of the following: decrease of alteration of motoneurons, for example inactivation and/or degradation of motoneurons, improvement of muscle strength, improvement of muscle size, decrease of alteration of walking, speaking and/or breathing capacities, decrease of fasciculations and/or cramps and/or improvement of walking, speaking, heating and/or breathing capacities, weight loss, decrease of upper motor neurones symptoms such as spasticity, hyperreflexia, emotional labidity.

A "subject" includes a mammal, e.g., a human, including a mammal in need of treatment for a disease or disorder, such as a mammal having been diagnosed with having a disease or disorder or determined to be at risk of developing a disease or disorder.

Another object of the present invention is a pharmaceutical composition comprising a nucleic acid and/or a vector and/or a host cell of the present invention.

The nucleic acid in the pharmaceutical composition may be any nucleic acid of the invention as defined above.

The vector in the pharmaceutical composition may be any vector of the invention as defined above.

The host cell in the pharmaceutical composition may be any host cell of the invention as defined above.

The pharmaceutical composition may be in any form that can be administered to a human or an animal.

Administration may be carried out directly, i.e. pure or substantially pure, or after mixing of the nucleic acid and/or a vector and/or a host cell of the present invention with a pharmaceutically acceptable carrier and/or medium. According to the present invention, the pharmaceutical composition may be a syrup or an injectable solution. According to the present invention, the pharmaceutical composition may be a pharmaceutical composition for oral administration selected from the group comprising a liquid formulation, an oral effervescent dosage form, an oral powder, a multiparticule system, an orodispersible dosage form. For example, when the pharmaceutical composition is for oral administration, it may be in the form of a liquid formulation selected from the group comprising a solution, a syrup, a suspension, an emulsion and oral drops. When the pharmaceutical composition is in the form of an oral effervescent dosage form, it may be in a form selected from the group comprising tablets, granules, and powders. When the pharmaceutical composition is the form of an oral powder or a multiparticulate system, it may be in a form selected from the group comprising beads, granules, mini tablets and micro granules. When the pharmaceutical composition is the form of an orodispersible dosage form, it may be in a form selected from the group comprising orodispersible tablets, lyophilized wafers, thin films, a chewable tablet, a tablet and a capsule, a medical chewing gum. According to the present invention, the pharmaceutical composition may be for buccal and sublingual routes, for example selected from the group comprising buccal or sublingual tablets, muco adhesive preparation, lozenges, oro-mucosal drops and sprays. According to the present invention, the pharmaceutical composition may be for topical-transdermal administration, for example selected from the group comprising ointments, cream, gel, lotion, patch and foam.

According to the present invention, the pharmaceutical composition may be for nasal administration, for example selected from the group comprising nasal drops, nasal spray, nasal powder. According to the present invention, the pharmaceutical composition may be for rectal administration, for example suppository or hard gelatin capsule. According to the present invention, the pharmaceutical composition may be for parenteral administration, for example subcutaneous, intramuscular, intravenous administration. The skilled person in the art understands clearly that the term "form" as used herein refers to the pharmaceutical formulation for its practical use.

The pharmaceutically acceptable carrier may be any know pharmaceutically carrier used for the administration of oligonucleotide/vector and or host cell to a human or to an animal, depending on the subject. For example, pharmaceutically acceptable carrier, diluent or excipient includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent or emulsifier. For example, this carrier may be selected from the group as described in Krützfeldt J, Rajewsky N, Braich R, Rajeev KG, Tuschl T, Manoharan M, Stoffel M. Nature. 2005 Dec 1;438(7068):685-9. Epub 2005 Oct 30 [7].

The form of the pharmaceutical composition may be selected with regards to the human or animal to be treated.

In another aspect, the present invention provides a method of treating a subject suffering from amyotrophic lateral sclerosis (ALS), in particular juvenile amyotrophic lateral sclerosis (ALS). This method may comprises the step of administering to said subject a nucleic acid and/or vector and/or host cell of the invention.

Nucleic acid and/or vector and/or host cell of the invention, as well as usable formulations are as defined above. The administration can be made by using any pharmaceutical way known by the skilled person and useful to administrate nucleic acid and/or vector and/or host cell. Examples of administrable forms of medicament are provided above.

Other features and advantages will become further apparent to those skilled in the art upon reading the examples below, given by way of nonlimiting illustration, with reference to the appended figures.

### Brief description of the drawing

Figure 1A is a scheme of the breeding strategy, in the scheme Fus +/+ means mice comprising wild type copy of the Fus gene, Fus ΔNLS/+ means mice comprising a copy of the Fus gene without the nuclear localization sequence (ΔNLS) and a wt copy of the Fus gene, hFUS means mice expressing a copy of the human wild type FUS gene. Figure 1B is a photography of a Western blot of Fus +/+ mice, Fus ΔNLS/+mice, Fus ΔNLS/ΔNLS mice expressing or not a copy of the human wild type FUS gene (hFUS). In this photography, ΔNLS shows the migration line of the ΔNLS protein, wt shows the migration line of the wild type mouse Fus protein and hFUS the migration line of the wild type human Fus protein. Figure 1C is a diagram representing the Kaplan Meier survival curve of the different mice genotypes: Fus +/+ mice, Fus ΔNLS/+and Fus ΔNLS/+; hFUS, the ordinate represent the percentage survival and the abscissa the time in days. Figure 1D is a diagram representing the Kaplan Meier survival curve of the different mice genotypes: Fus +/+ mice, Fus ΔNLS/ ΔNLS and Fus ΔNLS/ ΔNLS; hFUS, the ordinate represent the percentage survival and the abscissa the time in days.
Figure 2 represents diagram comprising curves representing the age-dependent changes in the mean hanging time (s) (Figure 2 A) and holding impulse (N s) (Figure 2 B) in the four-limb wire inverted grid test with Fus +/+, and Fus ΔNLS/+mice with or without hFUS transgene.
Figure 3 represents the analysis of cellular localization of FUS protein. Figure 3A represents photography of immunoblot analysis of FUS protein subcellular localization in cortex of Fus +/+ (+/+) and Fus ΔNLS/+ (Δ/+) mice with (hFUS) or without hFUS (No Tg) transgene and of Fus ΔNLS/ΔNLS (Δ/Δ) mice with hFUS transgene at 1 month of age. Representative results using different antibodies targeting the N-terminal part (N-ter) of FUS (N-ter FUS), the C-terminal (C-ter) NLS FUS (C-Ter FUS), mouse FUS or human FUS. SOD1 and HDAC1 are used as loading controls for cytoplasmic and nuclear protein extracts fractions, respectively. Figure 3B represents histograms representing the quantification of N-ter FUS, C-ter FUS, mouse FUS and human FUS protein levels in cytoplasmic and nuclear fractions in Fus +/+ (+/+) and Fus ΔNLS/+(Δ/+) mice with (hFUS) or without hFUS (No Tg) transgene and of Fus ΔNLS/ΔNLS (Δ/Δ) mice with hFUS transgene, in the figure * means p < 0.05, *** means p < 0.001 vs Fus+/+ mice, # means p<0.05 and ### means p<0.001 vs indicated mice genotype by ANOVA followed by Tukey. Figure 3 C represents photographies of US immunohistochemistry in the spinal cord ventral horn at 22 months of age of in Fus +/+ (+/+) and Fus ΔNLS/+(Δ/+) mice with (hFUS) or without hFUS (No Tg) transgene and of Fus ΔNLS/ΔNLS (Δ/Δ) mice with hFUS transgene. Figure 3D represents photographies of double immunostaining of the motoneuronal marker ChAT and FUS (N-terminal part) in the spinal cord ventral horn of mice at 22 months of age using anti-FUS and anti- ChAT antibodies.

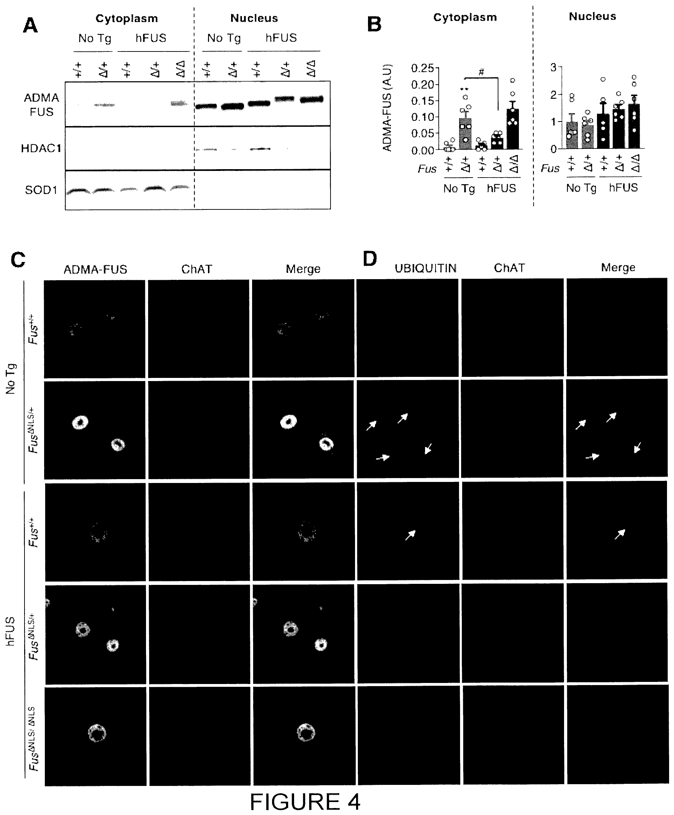

Figure 4 represents the analysis of accumulation of cytoplasmic asymmetrically demethylated (ADMA) FUS and the quantification of ADMA-FUS protein levels in cytoplasmic and nuclear fractions. Figure 4A represents photographies of immunoblot analysis of asymmetrically arginine dimethylated FUS on cytoplasmic and nuclear fractions of cortex of Fus +/+ (+/+) and Fus ΔNLS/+(Δ/+) mice with or without hFUS transgene and of Fus ΔNLS/ΔNLS mice with hFUS transgene at 1 month of age using an antibody recognizing asymmetrically arginine dimethylated FUS (ADMA-FUS), anti-HDAC1 antibody is used as a loading control for nuclear fractions and anti-SOD1 antibody is used as a loading control for nuclear fractions for cytoplasmic fractions. Figure 4B represents histograms corresponding to the Quantification of ADMA-FUS protein levels in cytoplasmic and nuclear fractions of Fus +/+ (+/+) and Fus ΔNLS/+(Δ/+) mice with or without hFUS transgene and of Fus ΔNLS/ΔNLS mice with hFUS transgene. In the figure ** means p < 0.01 vs Fus+/+ , # means p<0.05 vs indicated genotype by ANOVA analysis followed by Tukey. Figure 4C represents represents photographies of immunostaining of with motoneuronal marker ChAT (red) and/or ADMA-FUS (C, green) in the spinal cord ventral horn of Fus +/+ (+/+) and Fus ΔNLS/+(Δ/+) mice with or without hFUS transgene and of Fus ΔNLS/ΔNLS mice with hFUS transgene. Figure 4C represents represents photographies of immunostaining of with motoneuronal marker ChAT (red) and/or ubiquitin (D, →) in the spinal cord ventral horn of Fus +/+ (+/+) and Fus ΔNLS/+ (Δ/+) mice with or without hFUS transgene and of Fus ΔNLS/ΔNLS mice with hFUS transgene.

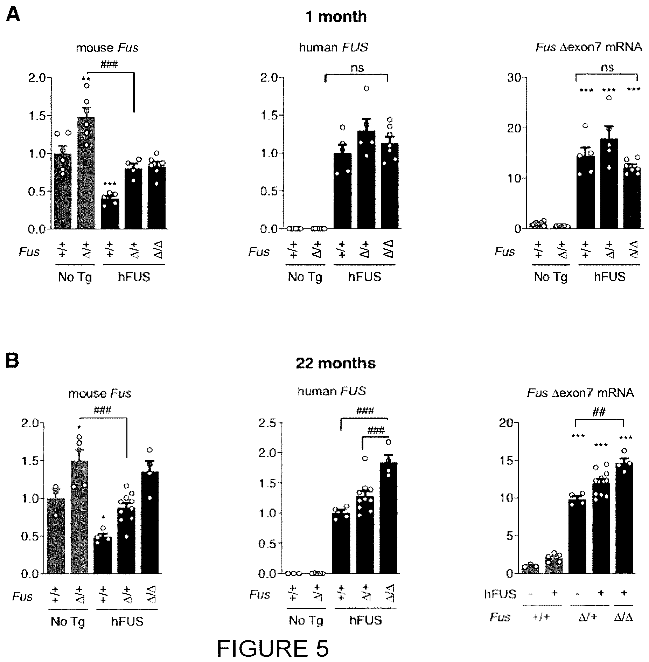

Figure 5 represents histograms representing gene expression in spinal cord determined with RT-qPCR. Figure 5A are histograms representing the results of expression of endogenous mouse Fus mRNA (left), human FUS transgene (middle) and endogenous Fus mRNA deleted of exon 7 (right) in spinal cord at 1 month of age. Figure 5B are histograms representing the results of expression of endogenous mouse Fus mRNA (left), human FUS transgene (middle) and endogenous Fus mRNA deleted of exon 7 (right) in spinal cord at 22 month of age.

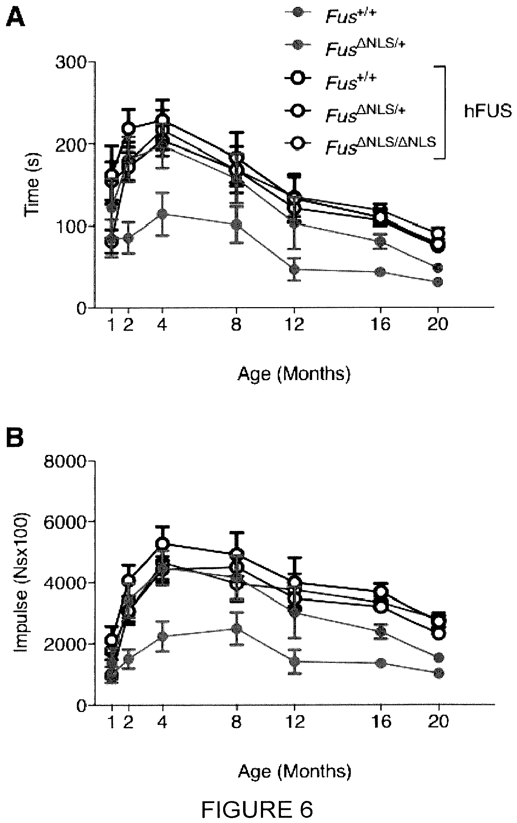

Figure 6 represents diagram comprising curves representing the age-dependent changes in the mean hanging time (s) (Figure 6 A) and holding impulse (N s) (Figure 6 B) in the four-limb wire inverted grid test with Fus +/+, Fus ΔNLS/+mice with or without hFUS transgene and of Fus ΔNLS/ΔNLS mice with hFUS transgene.

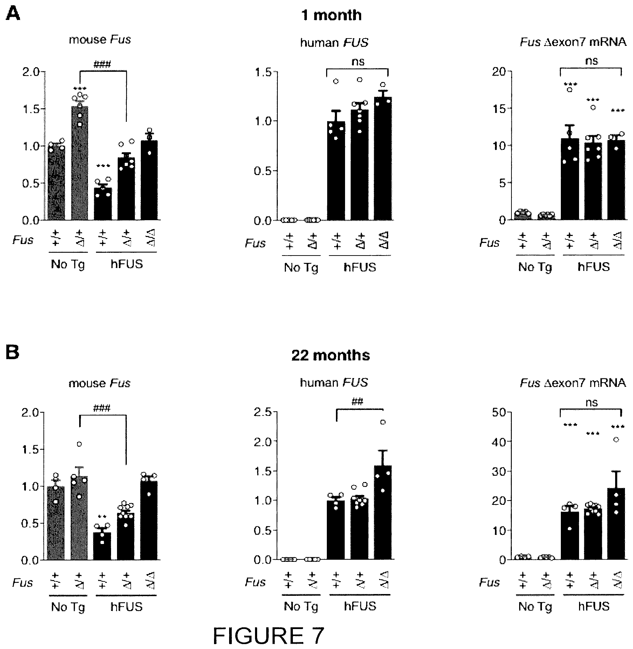

Figure 7 represents histograms representing gene expression in frontal cortex determined with RT-qPCR. Figure 7A are histograms representing the results of expression of endogenous mouse Fus mRNA (left), human FUS transgene (middle) and endogenous Fus mRNA deleted of exon 7 (right) in spinal cord at 1 month of age. Figure 7B are histograms representing the results of expression of endogenous mouse Fus mRNA (left), human FUS transgene (middle) and endogenous Fus mRNA deleted of exon 7 (right) in spinal cord at 22 month of age.

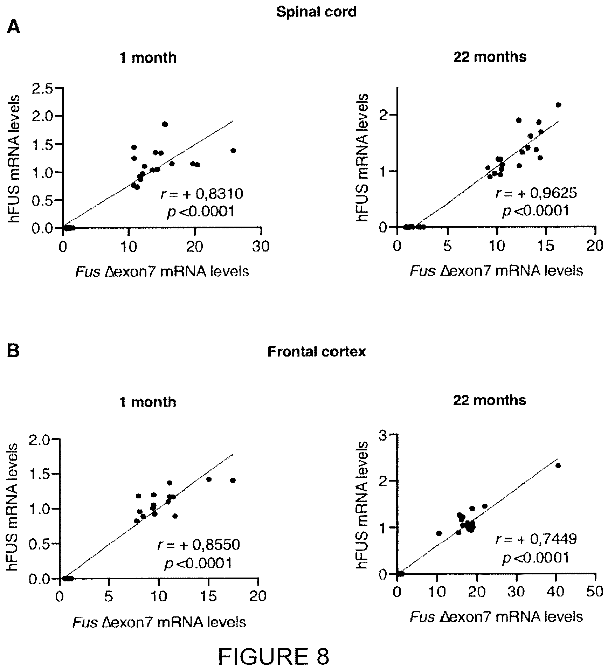

Figure 8 represents diagrams corresponding to the level of expression of human FUS transgene (ordinate) compared to the level of endogenous Fus mRNA deleted of exon 7 in Fus ΔNLS/+mice with hFUS mice 1 month or 22 months aged in spinal cord (Figure 8A) or Frontal cortex (Figure 8B).

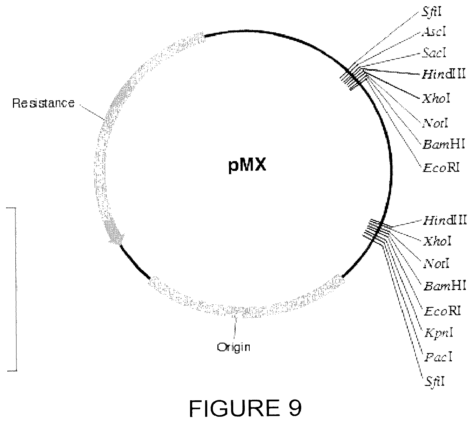

Figure 9 is a schematic representation of plasmid vector pMX

### Equivalents

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance that can be adapted to the practice of this invention in its various embodiments and the equivalents thereof.

### EXEMPLIFICATION

The present invention and its applications can be understood further by the examples that illustrate some of the embodiments by which the inventive product and medical use may be reduced to practice. It will be appreciated, however, that these examples do not limit the invention. Variations of the invention, now known or further developed, are considered to fall within the scope of the present invention as described herein and as hereinafter claimed.

### Examples

### Example 1 : Nucleic acid and vector comprising said nucleic acid

The nucleic acid and recombinant vector are prepared and obtained as disclosed in GeneArt Gene Synthesis products and services form thermofisher scientific.

The nucleic acid used were SEQ ID NO 1 to 20. The nucleic acid were prepared and included if necessary the sequences for the restriction site Notl (GCGGCCGC) and Xba1 (TCTAGA) for litigation with the plasmid. The prepared nucleic acid is 5 µg lyophilized plasmid DNA

The vector used is a plamid vector pMX which is represented in Figure 9 and described in Retrovirus-mediated gene transfer and expression cloning: powerful tools in functional genomics. Exp Hematol. 2003 Nov;31(11):1007-14. Kitamura T, Koshino Y, Shibata F, Oki T, Nakajima H, Nosaka T, Kumagai H. Retroviral vector designed for expression cloning and efficient gene transfer. The pMX vector harbors 5 long terminal repeat (LTR) and the extended packaging signal derived from MFG followed by a multi-cloning site (MSC) suitable for cDNA library construction and 3 LTR of MMLV.

10ng of DNA were incubated 30min on ice with 50µl of competent cells (Invitrogen, 18265-017). An heat shock was performed 20 seconds at 42°C in water bath. Then tubes were places 2min on ice then incubate with medium at 37°C for 1 hour at 225 rpm. Thereafter an agar culture with antibiotic was perfomed overnight at 37°C. A single colony was inoculated in liquid culture with antibiotic overnight at 37°C, 225rpm then plasmid midi kit was performed using manufacturer's recommendation (Qiagen, 12643).

### Example 2 : in vivo treatment of a mouse model of FUS-ALS with a nucleic acid encoding human FUS protein intron 6 and/or a sequence intron 7

### Materials and methods

### Mouse models and genotyping

Mouse experiments were approved by local ethical committee from Strasbourg University (CREMEAS) under reference number 2016111716439395.

The vector used is a BAC comprising the nucleic acid sequence coding for the human FUS of SEQ ID NO 10. Transgenic mice were generated as described in Scekic-Zahirovic et al.2016 [10] and in Scekic-Zahirovic 2017 [11], were bred in Charles River animal facility and housed in the Faculty of medicine from Strasbourg University with 12/12 hours of light/dark cycle (light on at 7:00 am) under constant conditions (21±1°C; 60% humidity) and with unrestricted access to food and water.

Mice were weaned and genotyped at 21 days by PCR from tail biopsy. The following primer sequences were used to genotype mice :
hTLS FUS-For: GAATTCGTGGACCAGGAAGGTC (SEQ ID NO 21)
hTLS FUS - Rev : CACGTGTGAACTCACCGGAGTCA (SEQ ID NO 22)
FUS-For: GATTTGAAGTGGGTAGATAGTGCAGG (SEQ ID NO 23)
FUS - Rev : CCT-TTC-CAC-ACT-TTA-GTT-TAG-TCA-CAG (SEQ ID NO 24)

Heterozygous Fus knock-in mice, lacking the PY-NLS, were crossed with mice expressing human wild type FUS from a complete, autoregulatory competent, human gene to obtain following genotypes: Fus+/+, FusΔNLS/+, FusΔNLS/ΔNLS, Fus+/+: hFUS, FusΔNLS/+: hFUS, FusΔNLS/ΔNLS: hFUS. The genetic background of all mice used in this study is C57BI6/J.

### Mouse behavior

### Survival

Survival was studied during the first hours after birth and dead new born mice were genotype. Other mice were genotyped at 21 days and followed weakly until death or killed using ketamine-xylazine when they reach the following endpoints: auto-mutilation, weight loss greater than 10% of the initial weight and when they could not turn around again within 10 seconds after being laid on their side.

### Inverted grid

Motor performance were performed weakly as described previously (Jelena Acta Neuropathol 2017 [8]). The wire grid hanging time (or "hang time") was defined as the amount of time that it takes the mouse to fall down from the inverted grid and was measured visually with a stop watch. The procedure was repeated 3 times during 5min. The holding impulse corresponds to hanging time normalized with mouse weight and gravitational force.

### Histological techniques

Mice were anesthetized with intraperitoneal injection of 100 mg/kg ketamine chlorhydrate and 5mg/kg xylazine then perfused with PFA 4%. After dissection, spinal cord was included in agar 4% and serial cuts of 40µm thick were made with vibratome.

### Peroxydase immunohistochemistry

For peroxidase immunohistochemistry, endogenous peroxidases were inactivated 10min with H₂O₂ 3% then slides were washed with PBS 1x and incubated overnight with rabbit anti-FUS antibody (ProteinTech, 11570-1-AP, 1:100) at room temperature. After rinsing in PBS, anti-rabbit biotinylated (Jackson, 711-067-003, 1/500) was incubated 2h at room temperature. The staining was revealed using the ABC kit (Vectastain ABC kit, PK-6100, Vector Laboratories Inc.) during 1h. After 3 washes with Phosphate Buffer Salin (PBS), slides were rinse in water and mounted in DPX (Sigma, 06522).

### Immunofluorescence

Sections were incubated in blocking solution (8% Goat serum, 0.3% Bovine Serum Albumin, 0.3% Triton, PBS-0.02% Thimérosal) at room temperature, i.e. 25°C, then incubate overnight at 4°C in primary antibody : rabbit anti-FUS antibody (ProteinTech, 11570-1-AP, 1:100), goat anti-ChAT (Milliport, AB144P, 1/50), rat anti-ADMA (Home Made, Germany, 1/100) or rabbit anti-Ubi (Abcam, ab179434, 1/100). After 3 rinses in PBS, sections were incubate 2h at room temperature i.e 25°C with Hoechst (Sigma, B2261, 1/50.000) and secondary antibody: Alexa-488-conjugated anti-rabbit secondary antibody (Jackson, 711-547-003, 1/500) or Alexa-594-conjugated anti-goat secondary antibody (Molecular Probes, A 11058, 1/500). Finally sections were subsequently washed with PBS (3 x 10 min) and mounted in DPX (Sigma, 06522).

### Electron microscopy

Mice were anesthetized with intraperitoneal injection of 100 mg/kg ketamine chlorhydrate and 5mg/kg xylazine and transcardiacally perfused with glutaraldehyde (2.5% in 0.1M cacodylate buffer at pH 7.4). Brains were dissected and immersed in the same fixative, i.e. glutaraldehyde, overnight, i.e. 12 hours. After 3 rinses in Cacodylate buffer (EMS, 11650), muscles were post fixed in 0.5% osmium and 0.8% potassium ferrocyanide in Cacodylate buffer 1h at room temperature. Finally, tissues were dehydrated in graded ethanol series (25%, 50%, 70%, 95%, 100%), and embedded in Embed 812 (EMS, 13940). The ultrathin sections (50 nm) were cut with an ultramicrotome (Leica, EM UC7), counterstained with uranyl acetate (1% (w/v) in 50% ethanol) and observed with a Hitachi 7500 transmission electron microscope (Hitachi High Technologies Corporation, Tokyo, Japan) equipped with an AMT Hamamatsu digital camera (Hamamatsu Photonics, Hamamatsu City, Japan).

### Tissue fractionation and western blotting

Tissus were washed in Phosphate Buffer Saline (PBS) 1x and lysed in Syn-PER Synaptic Protein Extraction (Thermo Scientific, 87793) according to the manufacturer's instructions. Protein extract were dosed by BCA Assay (Interchim, UP95424A, UP95425A). Thereafter proteins were denatured and SDS page were performed with 10 (for cytoplasmic proteins) and 30µg of protein (for nuclear proteins) on criterion TGX stain free gel 4-20% (Biorad, 5678094). Proteins were blotted on nitrocellulose membrane using semi-dry Transblot Turbo system (BioRad, France) and blocked with 10% non-fat milk during 1h. Primary antibodies (Rabbit anti-hFUS (Home Made, #14080, 1/2000), Rabbit anti-mFUS (Home Made, #14082, 1/4000), Rat anti-FUS ADMA (Home made, Germany, 1/500), Rabbit anti-FUS 293 (Bethyl, A-300-293A, 1/2000), Rabbit anti-FUS 294 (Bethyl, A300-294A, 1/2000), Sheep anti-SOD1 (Calbiochem, 574597, 1/1000), Rabbit anti-HDAC1 (Bethyl, A300-713A, 1/1000) were incubated overnight, i.e 12 hours, at 4°C in 3% non-fat milk. Washing were proceeded with washing buffer (Tris pH 7.4 1 M, NaCl 5M, Tween 20 100 %) and secondary antibody (anti-rabbit HRP (PARIS, BI2407,1/5000), anti-sheep HRP (Jackson, 713-035-147, 1/5000) were incubated 1h30 at room temperature i.e 25°C. After successive washes, i.e. 3 washes, with PBS1x proteins were visualized with chemiluminescence using ECL Lumina Forte (Millipore, France) and chemiluminescence detector (Bio-Rad, France). Total proteins were detected with stain free gel capacity (Biorad, 5678094) and used to normalized.

Antibodies used were the followings :
- Rabbit anti-hFUS (Home Made, #14080, 1/2000),
- Rabbit anti-mFUS (Home Made, #14082, 1/4000)
- Rat anti-FUS ADMA (Home made, Germany, 1/500)
- Rabbit anti-FUS 293 (Bethyl, A-300-293A, 1/2000)
- Rabbit anti-FUS 294 (Bethyl, A300-294A, 1/2000)
- Sheep anti-SOD1 (Calbiochem, 574597, 1/1000)
- Rabbit anti-HDAC1 (Bethyl, A300-713A, 1/1000)
- Anti-rabbit HRP (PARIS, BI2407,1/5000)
- Anti-sheep HRP (Jackson, 713-035-147, 1/5000)

### RNA extraction and RT-qPCR

Total RNA was extracted from spinal cord and frontal cortex using Tissue Lyser (Qiagen) in 100µl of TRIzol® reagent (Life Technologies).

1 µg of RNA was reverse transcribed with iScript™ reverse transcription using manufacturer conditions (Biorad, 1708841). Quantitative polymerase chain reaction was performed using Sso Advanced Universal SYBR Green Supermix (Bio-Rad) and quantified with Bio-Rad software using manufacturer conditions. Gene expression was normalized by calculating a normalization factor using actin, TBP and pol2 genes according to GeNorm software (Vandesompe et al 2002)
Primer sequences were as follows:

| | |
|---|---|
| Actin : | F-CCACCAGTTCGCCATGGAT (SEQ ID NO 25), |
| | R-GGCTTTGCACATGCCGGAG (SEQ ID NO 26) |
| TBP: | F-CCAATGACTCCTATGACCCCTA(SEQ ID NO 27), |
| | R-CAGCCAAGATTCACGGTAGAT (SEQ ID NO 28) |
| Pol2: | F-GCTGGGAGACATAGACCA (SEQ ID NO 29), |
| | R-TTACTCCCCTGCATGGTCTC (SEQ ID NO 30) |
| hFUS: | F-GCCAGAACACAGGCTATGGA (SEQ ID NO 31), |
| | R-CGATTGGGAGCTCTGGCTAC (SEQ ID NO 32) |
| T-FUS: | F-TTATGGACAGACCCAAAAACACA (SEQ ID NO 33) |
| | R-TGCTGCCCATAAGAAGATTG (SEQ ID NO 34) |
| FUS ΔNLS: | F-GCAAGATGGACTCCTAGTGTTAAT (SEQ ID NO 35), |
| | R-ACCTCTACAAATGTGGTATGGC (SEQ ID NO 36) |
| Fus_exon6-8: | F-CGGCATGGGGTCCTCGG (SEQ ID NO 37), |
| | R-CCTAGGCCTTGCACGAAGAT (SEQ ID NO 38) |

### Statistics

All results from analysis are presented as mean ± standard error of the mean (SEM) and differences were considered significant when p < 0.05. Significance is presented as follows: * p<0.05, ** p<0.01, and *** p<0.001. For comparison of two groups, two-tailed unpaired Student's t - test was used in combination with F-test to confirm that the variances between groups were not significantly different. Comparison for more than two groups was performed using one-way ANOVA and Tukey or Bonferroni's multiple comparison post hoc test. Data were analyzed by using the GrapPad Prism version 6.0.

### Results

### Human wild type FUS transgene rescues perinatal letality in homozygous FusΔNLS mice

Human wild type FUS transgenic mice (hFUS mice) expressing wild type FUS from a BAC have been recently generated and characterized (Lopez-Erauskin J, Tadokoro T, Baughn MW, et al. ALS/FTD-Linked Mutation in FUS Suppresses Intra-axonal Protein Synthesis and Drives Disease Without Nuclear Loss-of-Function of FUS. Neuron 2018; 100(4): 816-30 e7 [9]). These mice express slightly increased FUS levels, mostly of human origin, and do not show ALS-related phenotypes contrary to the human mutant FUS transgenic lines generated and characterized in parallel (Lopez-Erauskin J, Tadokoro T, Baughn MW, et al. ALS/FTD-Linked Mutation in FUS Suppresses Intra-axonal Protein Synthesis and Drives Disease Without Nuclear Loss-of-Function of FUS. Neuron 2018; 100(4): 816-30 e7 [9]). hFUS mice were crossed with FusΔNLS mice (Scekic-Zahirovic J, Sendscheid O, El Oussini H, et al. Toxic gain of function from mutant FUS protein is crucial to trigger cell autonomous motor neuron loss. EMBO J 2016; 35(10): 1077-97 [10]), that we previously showed to develop mild, late onset motor neuron disease as heterozygous (Scekic-Zahirovic J, Oussini HE, Mersmann S, et al. Motor neuron intrinsic and extrinsic mechanisms contribute to the pathogenesis of FUS-associated amyotrophic lateral sclerosis. Acta Neuropathol 2017; 133(6): 887-906 [11]) while homozygous FusΔNLS mice die in the perinatal period (Scekic-Zahirovic J, Sendscheid O, El Oussini H, et al. Toxic gain of function from mutant FUS protein is crucial to trigger cell autonomous motor neuron loss. EMBO J 2016; 35(10): 1077-97 [10]). After two breeding steps, genotypes of interest, including heterozygous and homozygous FusΔNLS mice with or without the hFUS transgene were obtained (Figure 1A). Of note, all mice were in C57BI/6 genetic background, and only mice from the F2 generation were analyzed here, thereby avoiding most of the possible confounding effects of genetic background heterogeneity. Mice were genotyped at 1 month of age or at death if occurring before 1 month of age. As demonstrate in figure 1, D FusΔNLS/ΔNLS mice without hFUS died within the first hours after birth and we did not observe mice with this genotype at 1 month of age (Figure 1C). Contrastingly, the addition of the human wild type FUS transgene allowed the survival of most homozygous FusΔNLS mice until adulthood (Figure 1B, D). A slight but non statically significant increased mortality of all FusΔNLS groups as compared to wild type animals was observed (Figure 1D).

### Human wild type FUS transgene rescues late onset muscle weakness in heterozygous FusΔNLS mice

The mice used were Fus +/+, Fus ΔNLS/+, and Fus ΔNLS/+ expressing H FUS mice. The number of mice used were N =10-28 per group. As shown on figure 2, FusΔNLS/+ mice develop mild, late onset, muscle weakness, that can be easily followed using inverted grid test. Using this test, the deficit in FusΔNLS/+ mice occurred was observed after 2 months of age and was stable with age (Figure 2A-B). Contrastingly, FusΔNLS/+ mice with a human wild type FUS transgene were undistinguishable from wild type littermates in this test, suggesting that the wild type FUS transgene was sufficient to rescue the neuromuscular phenotype (Figure 2A-B).

### Human wild type FUS transgene rescues cytoplasmic mislocalization of the FUS protein in FusΔNLS mice

The effect of the expression of human wild type FUS transgene on the subcellular localization of FUS in FusΔNLS mice was studied. The results obtained are represented on figure 3. As demonstrated on figures 3A and 3B, the cytoplasmic fractions of cerebral cortex of FusΔNLS/+ mice displayed higher levels of FUS than corresponding wild type littermate fractions as assessed using western blotting. This was not observed when an antibody targeting the NLS sequence, absent from the FUSΔNLS protein, was used, demonstrating that this increase is related to the mislocalization of the mutant protein. Importantly, mouse FUS, as identified using a mouse specific FUS antibody, was increased in cytoplasmic fractions of FusΔNLS/+ mice, and this was normalized by the human wild type FUS transgene (Figure 3A-B). Importantly, nuclear FUS levels were unchanged in all genotypes, irrespective of the presence of the FusΔNLS mutation or of the human wild type FUS transgene. Human FUS levels were increased in FusΔNLS/ΔNLS mice carrying a human FUS transgene, likely compensating for the loss of nuclear FUS of mouse origin. To further confirm this rescuing effect of the transgene, immunohistochemistry on spinal cord sections of 22 months old mice was performed. As shown in Figure 3C, FusΔNLS/+ neurons showed lacked nuclear enrichment in FUS staining, and this was fully prevented by the human wild type FUS transgene, whether in FusΔNLS/+ mice or in FusΔNLS/ΔNLS mice. Double immunofluorescence using FUS and ChAT antibodies to unambiguously identify motor neurons further confirmed that the human wild type FUS transgene rescued FUS mislocalization in motor neurons as demonstrated and shown on Figure 3D.

This results clearly demonstrate that products, for example nucleic acid and/or vectors, of the present invention allow in the same time and synergistically to compensate the genetic mutation by providing wild-type protein FUS, to down regulate the expression and the production of mutated protein FUS, and to regulate the expression and production of wild-type protein FUS.

### Human wild type FUS transgene rescues ALS-related pathology in heterozygous FusΔNLS mice

To further document the protective effect of the human wild type FUS transgene, pathological hallmarks developed by Fus +/+ (+/+) and Fus ΔNLS/+(Δ/+) mice with (hFUS) or without hFUS (No Tg) transgene and of Fus ΔNLS/ΔNLS (Δ/Δ) mice with hFUS transgene mice was analysed. The number of mice used were N =4-8 per group. Accumulation of cytoplasmic asymmetrically demethylated (ADMA) FUS is typical of FUS-ALS, and has been previously observed in FusΔNLS/+ mice. Indeed, the large increase in ADMA-FUS in FusΔNLS/+ cytoplasmic fractions was largely prevented in FusΔNLS/+ mice with a wild type human FUS transgene (Figure 4A-B). This was however not the case in FusΔNLS/ΔNLS mice carrying a hFUS transgene that retained high levels of cytoplasmic methylated FUS. Consistently, while ADMA-FUS immunoreactivity is diffuse in motor neurons of FusΔNLS/+ mice, the human wild type FUS transgene led to more localized, perinuclear immunoreactivity in FusΔNLS/+ mice (Figure 4C). Furthermore, the punctate ubiquitin pathology observed in FusΔNLS/+ motor neurons was also prevented by the human wild type FUS transgene (Figure 4D). Thus, the human wild type FUS transgene was able to rescue typical hallmarks of ALS-FUS pathology in FusΔNLS/+ mice.

This results clearly demonstrate that products, for example nucleic acid and/or vectors, of the present invention allow synergistically to eliminate the cytoplasmic accumulation of mutated FUS, to produce wild-type protein FUS and also to regulate the production of the wild-type protein FUS. In addition this results clearly demonstrate that products, for example nucleic acid and/or vectors, of the present invention allow to decrease the expression of the mRNA of mutated FUS protein and to restore and correct the function of FUS protein.

### Human wild type FUS transgene reverts overexpression of endogenous Fus and activates exon 7 skipping

To further document the protective and/or therapeutic effect of the human wild type FUS transgene on the regulation of expression of endothelial FUS gene in Fus +/+ (+/+) and Fus ΔNLS/+(Δ/+) mice with (hFUS) or without hFUS (No Tg) transgene and of Fus ΔNLS/ΔNLS (Δ/Δ) mice with hFUS transgene mice was analysed. Single human wild type FUS transgenic mice had lower expression of the endogenous Fus gene15 while FusΔNLS/+ mice displayed a slight but significant increase in Fus mRNA levels at both 1 and 22 months of age in spinal cord (Figure 5A-B) and frontal cortex (Figure 7). This overexpression of the endogenous murine gene was fully corrected by the human wild type FUS transgene. Furthermore, the expression of the human FUS transgene was accompanied by strongly increased levels of the aberrantly spliced mRNA of Fus devoid of exon 7, substrate to mRNA decay (Figure 5A-B). Indeed, there was a strong correlation between hFUS expression and deletion of exon 7 in endogenous mouse Fus mRNA (Supplementary Figure 3). Thus, the addition of the human wild type FUS transgene leads to decreased expression of the toxic protein through the activation of the autoregulatory loop, and subsequent alleviation of all the downstream consequences of the expression of cytoplasmically mislocalized FUS.

This example clearly demonstrate that products, for example nucleic acid and/or vectors, of the present invention allow to treat and cure amyotrophic lateral sclerosis (ALS), and in particular juvenile amyotrophic lateral sclerosis (ALS). In particular, this example clearly demonstrates that the present invention allow in the same time to decreased expression of the toxic protein, i.e mutated FUS, to produce a functional and homologous protein of human FUS protein, to activate of the autoregulatory loop for mutated FUS, to activate the autoregulatory loop for the expression of the nucleic acid of the invention allowing a wildtype expression of said nucleic acid and to eliminate the cytoplasmic and/or nucleus accumulation/ mislocalization of the mutated FUS. Accordingly this example clearly demonstrates that surprisingly and unexpectedly, the present invention allow in the same time to "cancel" the biological default and to restore the wild-type biological mechanism

### List of References

1. Murray E. J. (ed.), Methods in Molecular Biology, Vol. 7, Gene Transfer and Expression Protocols, Humana Press (1991)
2. Johnston, Nature, 346: 776-777 (1990)
3. Brash et al., Mol. Cell Biol., 7: 2031-2034 (1987)
4. Smith and Waterman, Adv. Appl. Math., 2:482 (1981)
5. Needleman and Wunsch, J. Mol. Biol., 48:443 (1970)
6. Pearson and Lipman, Proc. Nat. Acad. Sci U.S.A., 85:2444 (1988)
7. Krützfeldt J, Rajewsky N, Braich R, Rajeev KG, Tuschl T, Manoharan M, Stoffel M. Nature. 2005 Dec 1;438(7068):685-9. Epub 2005 Oct 30
8. Jelena Acta Neuropathol 2017
9. Lopez-Erauskin J, Tadokoro T, Baughn MW, et al. ALS/FTD-Linked Mutation in FUS Suppresses Intra-axonal Protein Synthesis and Drives Disease Without Nuclear Loss-of-Function of FUS. Neuron 2018; 100(4): 816-30 e7
10. Scekic-Zahirovic J, Sendscheid O, El Oussini H, et al. Toxic gain of function from mutant FUS protein is crucial to trigger cell autonomous motor neuron loss. EMBO J 2016; 35(10): 1077-97
11. Scekic-Zahirovic J, Oussini HE, Mersmann S, et al. Motor neuron intrinsic and extrinsic mechanisms contribute to the pathogenesis of FUS-associated amyotrophic lateral sclerosis. Acta Neuropathol 2017; 133(6): 887-906
12. Mitchell JC, 2013 "Overexpression of human wild-type FUS causes progressive motor neuron degeneration in an age- and dose-dependent fashion" Acta Neuropathologica volume 125, pages273-288(2013 DOI: 10.1007/s00401-012-1043-z

## Claims

1. A nucleic acid encoding human FUS protein comprising a sequence having at least 69% sequence identity with the sequence SEQ ID NO 1 of intron 6 and/or a sequence having at least 60% sequence identity with the sequence SEQ ID NO 2 of intron 7, said sequences being located on either side of exon 7.

2. A nucleic acid according to the claim, wherein said sequence having at least 69% sequence identity with the sequence SEQ ID NO. 1 is selected from the group consisting of the sequence SEQ ID NO. 3, SEQ ID NO. 4 or SEQ ID NO. 19.

3. A nucleic acid according to the claim, wherein said sequence having at least 60% sequence identity with the sequence SEQ ID NO 2 is selected from the group consisting of the sequence SEQ ID NO 5, SEQ ID NO 6, SEQ ID NO 7, SEQ ID NO 8 or SEQ ID NO 20.

4. A nucleic acid according to any of claims 1 to 3, said sequence is a sequence selected from the group consisting of the sequence SEQ ID NO 9, SEQ ID NO 10, SEQ ID NO 11, SEQ ID NO 12, SEQ ID NO 13, SEQ ID NO 14, SEQ ID NO15, SEQ ID NO 16, SEQ ID NO 17, SEQ ID NO 18.

5. A recombinant vector comprising a nucleic acid according to one of claims 1 to 4.

6. Vector according to claim 5 **characterized in that** it is an adenovirus, a plasmid, a YAC (Yeast Artificial Chromosomes) or a BAC (Bacterial Artificial Chromosome).

7. Vector according to one of claims 5 and 6, **characterized in that** it is adenovirus.

8. A nucleic acid according to any of Claims 1 and 4 for use as a medicament in the treatment and/or prevention of amyotrophic lateral sclerosis (ALS).

9. A recombinant vector according to any of claims 5 to 7 for use as a medicament in the treatment and/or prevention of amyotrophic lateral sclerosis (ALS).

10. A pharmaceutical composition for the prevention and/or treatment of amyotrophic lateral sclerosis (ALS), comprising a therapeutically effective amount of nucleic acid according to any of claims 1 to 4 and/or a carrier according to any of claims 5 to 7.

11. A host cell comprising a nucleic acid according to any of claims 1 to 4 and/or a vector according to any of claims 5 to 7.
